(19) Europäisches Patentamt
European Patent Office
Office européen des brevets

(11) EP 1 687 305 B1

(12) **EUROPEAN PATENT SPECIFICATION**

(45) Date of publication and mention of the grant of the patent:
**09.07.2008 Bulletin 2008/28**

(21) Application number: **04798017.2**

(22) Date of filing: **19.11.2004**

(51) Int Cl.:
***C07D 471/04*** *(2006.01)* ***A61K 31/435*** *(2006.01)*
***A61P 35/00*** *(2006.01)*

(86) International application number:
**PCT/EP2004/013178**

(87) International publication number:
**WO 2005/054237 (16.06.2005 Gazette 2005/24)**

(54) **1H-IMIDAZOQUINOLINE DERIVATIVES AS PROTEIN KINASE INHIBITORS**

1H-IMIDAZOCHINOLINDERIVATE ALS PROTEINKINASEINHIBITOREN

DERIVES D'1H-IMIDAZOQUINOLINE EN TANT QU'INHIBITEURS DE LA PROTEINE KINASE

(84) Designated Contracting States:
**AT BE BG CH CY CZ DE DK EE ES FI FR GB GR HU IE IS IT LI LU MC NL PL PT RO SE SI SK TR**

(30) Priority: **21.11.2003 US 524229 P**

(43) Date of publication of application:
**09.08.2006 Bulletin 2006/32**

(73) Proprietors:
- **Novartis AG**
  **4056 Basel (CH)**
  Designated Contracting States:
  **BE BG CH CY CZ DE DK EE ES FI FR GB GR HU IE IS IT LI LU MC NL PL PT RO SE SI SK TR**
- **Novartis Pharma GmbH**
  **1235 Vienna (AT)**
  Designated Contracting States:
  **AT**

(72) Inventors:
- **CAPRARO, Hans-Georg**
  **CH-4310 Rheinfelden (CH)**
- **CARAVATTI, Giorgio**
  **CH-4002 Basel (CH)**
- **FURET, Pascal**
  **F-68800 Thann (FR)**
- **GARCIA-ECHEVERRIA, Carlos**
  **CH-4052 Basel (CH)**
- **IMBACH, Patricia**
  **CH-4303 Kaiseraugst (CH)**
- **STAUFFER, Frédéric**
  **CH-4153 Reinach (CH)**

(74) Representative: **Crawley, Patrick Edward**
**Novartis AG**
**Corporate Intellectual Property**
**4002 Basel (CH)**

(56) References cited:
**EP-A- 0 389 302**
**EP-A- 1 104 764**
**EP-A- 1 256 582**
**WO-A-02/46188**
**US-A- 5 605 899**
**US-B1- 6 331 539**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 687 305 B1

Printed by Jouve, 75001 PARIS (FR)

## Description

### Background of the Invention

The invention relates to novel imidazoquinolines and salts thereof, the use of imidazoquinolines and salts thereof for the manufacture of pharmaceutical preparations for the treatment of said diseases; imidazoquinolines for use in the treatment of protein kinase dependent diseases; pharmaceutical preparations comprising an imidazoquinoline, especially for the treatment of a protein kinase dependent disease; and a process for the preparation of the imidazoquinolines.

### Summary of the Invention

Recently, the concept of treating proliferative diseases by using drugs designed specifically against abnormally active protein kinases has been definitely proven in the treatment of chronic myeloic leukemia (CML) where a first product has now been approved for successful treatment. Clinical studies showed that the drug (*N*-{5-[4-(4-methyl-piperazino-methyl)-benzoylamido]-2-methylphenyl}-4-(3-pyridyl)-2-pyrimidine-amine, especially in the form of the methane sulfonate (monomesylate) salt called STI571, which is sold, e.g., under the tradename Gleevec®, has impressive activity against chronic phase CML. Typical for CML is a characteristic t(9;22) translocation that juxtaposes the 5' end of the bcr gene with the 3' end of the abl gene, resulting in a unique 210 kDa fusion protein $p210^{bcr/abl}$ with constitutive activity. The result is a $p210^{bcr/abl}$-induced transformation ultimately leading to CML. ST1571 is a reversible inhibitor that occupies the ATP binding pocket of $p210^{bcr/abl}$ and stabilizes the kinase in an inactive conformation. This inhibitory action appears to be the basis for its action against CML.

Over-expression or constitutive-expression (activity) of protein kinases appears to be a general principle for transformations that finally lead to proliferative growth of cells and thus cancer, psoriasis or other proliferative diseases. Protein kinase B (PKB), also known as Akt, is a member of a conserved family of kinases that includes PKBα, PKBβ and PKBγ in humans. This serine/threonine kinase mediates the physiological effects of several peptide growth factors, including platelet-derived growth factor, insulin and insulin-like growth factor-1. PKB contains a pleckstrin homology (PH) domain in its amino-terminal domain, a kinase domain in the middle and a regulatory domain in the carboxy-terminal region. The binding of phosphoinositides to the PH domain of PKB recruits PKB to the plasma membrane where it is phosphorylated on threonine-308 (Thr308) and on serine-473 (Ser473). Activation of the PKB pathways results in cellular proliferative, as well as antiapoptotic tumor cell responses. PKBα is amplified in 20% of gastric adenocarcinoma and PKBα is amplified in 15% of ovarian cancers, 12% of pancreatic cancers and 3% of breast carcinomas. PKBα expression and activity is elevated in estrogen receptor negative breast cancer cells and in androgen-independent prostate cancer.

The tumor suppressor protein PTEN is a lipid phosphatase that converts PIP3 into PIP2, and therefore down-regulates all pathways that are PIP3 dependent. See Cantley et al., Proc Nat. Acad Sci USA, Vol. 96, pp. 4240-4245 (1999); Vazquez et al., Biochimica et Biophysica Acta, Vol. 1470, pp. M21-M35 (2000); and Simpson et al., Exper Cell Res, Vol. 264, pp. 29-41 (2001). The PTEN tumor suppressor gene is frequently found mutated or deleted in gliobastoma [see Li et al., Science, Vol. 275, pp. 1943-1947 (1997); and Steck et al., Nat Genetics, Vol. 15, pp. 356-362 (1997)], endometrial carcinoma [see Nagase et al., Br J Cancer, Vol. 74, pp. 1979-1983 (1996)]; Peiffer et al., Cancer Res, Vol. 55, pp. 1922-1926 (1995), prostate adenocarcinoma [see Gray et al., Cancer Res, Vol. 55, No. 21, pp. 4800-4803 (1995); and Ittmann, Cancer Res, Vol. 56, pp. 2143-2147 (1996)], breast adenocarcinoma [see Perren et al., Am J Pathol, Vol. 155, No. 4, pp. 1253-1260 (1999)] and melanoma. See Robertson et al., Cancer Res, Vol. 59, No. 15, pp. 3596-3601 (1999). PTEN mutations have also been found in cancers of the bladder, lung and lymphatic systems. See Cairns et al., Oncogene, Vol. 16, pp. 3215-3218 (1998); Gronbaek et al., Blood, , Vol. 91, pp. 4388-4390 (1998); and Kim et al., Oncogene, Vol. 16, pp. 89-93 (1998). Tumor cell lines with an inactive PTEN allele exhibit elevated PKB kinase activity. See Haas-Kogan et al., Curr Biol, Vol. 8, pp. 1195-1198 (1998); Whang et al., Proc Natl Acad Sci USA, Vol. 95, pp. 5246-5250 (1998); and Wu et al., Proc Nail Acad Sci USA, Vol. 95, pp. 15587-15591 (1998). Consequently, mutations in PTEN that inactivate its lipid phosphatase function allow unregulated PKB activity, a major player in the PI-3K pathway, leading to uncontrolled cell proliferation and increased survival.

Germline mutations of PTEN are also associated to Cowden syndrome, Lhermitte-Dudos disease and Bannayan-Zonana syndrome. See Maehama and Dixon, Trends Cell Biol, Vol. 9, pp. 125-128 (1990). Therefore, compounds that down-regulate the kinase activity of PKB may prove to be of clinical interest for single and combined anticancer treatment modalities.

S6K1, also known as p70 S6 kinase is a serine/threonine kinase that phosphorylates the ribosomal protein S6 (rpS6) in response to mitogen stimulation. Phosphorylation of rpS6 leads of increased expression of the mRNA family members characterized by an oligopyrimidine tract at their 5' transcriptional start sites (5'TOP) and results in upregulation of protein synthesis because the 5'TOP family mRNAs encode components of the translational apparatus including ribosomal proteins and translational elongation factors. S6K1-deficient mice are protected against diet-induced weight

gain and insulin resistance due to the increase in energy expenditure and fatty acid metabolism, suggesting that S6K1 is a potential target of metabolic diseases, e.g., type II diabetes, obesity, hyperlipidemia and atherosclerosis.

The compounds of the present invention also exhibit powerful inhibition of the tyrosine kinase activity of anaplastic lymphoma kinase (ALK) and the fusion protein of NPM-ALK. This protein tyrosine kinase results from a gene fusion of nucleophosmin (NPM) and the anaplastic lymphoma kinase (ALK), rendering the protein tyrosine kinase activity of ALK ligand-independent. NPM-ALK plays a key role in signal transmission in a number of hematopoetic and other human cells leading to hematological and neoplastic diseases, for example in anaplastic large-cell lymphoma (ALCL) and non-Hodgkin's lymphomas (NHL), specifically in ALK+ NHL or Alkomas, in inflammatory myofibroblastic tumors (IMT) and neuroblastomas. See Duyster et al., Oncogene, Vol. 20, pp. 5623-5637 (2001). In addition to NPM-ALK, other gene fusions have been identified in human hematological and neoplastic diseases; mainly TPM3-ALK (a fusion of nonmuscle tropomyosin with ALK).

RET encodes a transmembrane receptor of the protein tyrosine kinase family. RET is the receptor of growth factors belonging to the glial cell line-derived neurotrophic factor (GDNF) family. This family is comprised of the GDNF, neurturin (NTN), persephin (PSP), and artemin, which all have trophic influences on a variety of neuronal populations. These ligands interact with multimeric receptors composed of high-affinity glycosyl-phosphatidylinositol (GPI)-linked receptors and the RET kinase. Ligand-dependent RET activation can promote neuronal cell survival and differentiation. Insufficient PTK signaling may be responsible for developmental diseases. Gain of function of the RET receptor PTK is associated with human cancer. WO 02/46188 A2 describes imidazoquinoline compounds as immunomodulators, and US 5,605,899 e.g. as antiviral and antitumor agents and US 6,331,539 as useful in the treatment of a variety of conditions including viral and neoplastic diseases. However, these documents describe substituents and substitutions which do not anticipate nor suggest other substituents such as those in the present disclosure.

What is desirable from the point of view of possible treatments of proliferative diseases is to have a plethora of compound classes each tailored to specific protein kinases or protein kinase classes, thus allowing to come to specific treatments. Therefore, a strong need exists to find new classes of compounds allowing for such specific inhibitory effects.

## Summary of the Invention

The class of imidazoquinoline compounds described herein, especially novel compounds falling under this class, has surprisingly been found to have pharmaceutically advantageous properties, *inter alia,* allowing for the inhibition of specific types or classes or groups of protein kinases, especially PKB, ALK, RET, S6K1 or any combinations of two or more of these. The class of imidazoquinoline compounds described herein further inhibit mutants of said kinases. In addition to this established activity, the imidazoquinolines have the advantage that their backbone in addition allows for a plethora of substitution patterns that offer a broad possibility to achieve a fine tuning for specific interaction with the ATP-binding site of the kinases, thus opening a new perspective and providing kinase inhibitors of various degrees of specificity.

## Detailed Description of the Invention

The invention in particular relates to imidazoquinolines, namely compounds of the formula (I)

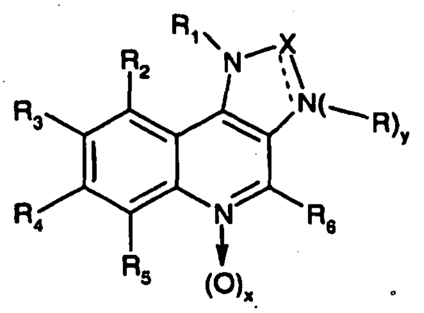

(I)

$R_1$ is substituted or unsubstituted aryl or heteroaryl, especially phenyl, which is substituted with up to 4 substituents, preferably up to 3 substituents, where the substituents are the same or different and are independently selected from halo, e.g., F or Cl; lower alkyl which may be unsubstituted or substituted with halo, especially methyl, ethyl, propyl or trifluoromethyl; cyano; cyano-lower alkyl, e.g., cyanomethyl, cyanoethyl or cyanopropyl; lower alkoxy; amino; amino-lower alkyl; amino-lower alkoxy; amino-lower alkyl sulfanyl; or thiol-lower alkyl, wherein the amino

group can be mono- or di-substituted, e.g., $-(C_1-C_7)_mNR_8R_9$ or $-O-(C_1-C_7)_mNR_8R_9$,
wherein
m is 0 or 1; and
$R_8$ and $R_9$ can be the same or different and are independently H; lower alkyl, e.g., methyl, ethyl or propyl; lower cycloalkyl, e.g., cyclopropyl, or
$R_8$ and $R_9$ , together with the N atom, form a 3- to 8-membered heterocyclic ring containing 1-4 nitrogen, oxygen or sulfur atoms, e.g., azetidinyl, pyrrolidinyl, piperidino, morpholinyl, imidazolinyl, imidazolinyl-ethyl, piperazinyl or lower alkyl-piperazinyl; amino-carbonyl-lower alkyl, e.g., $R_8R_9$-N-C(O)-$CH_2$-, wherein $R_8$ and $R_9$ are as defined above; heterocyclyl; heterocyclyl-lower alkyl; heterocyclyl-lower alkoxy; or heterocyclyl-lower alkanesulfanyl, wherein the heterocyclyl is a 3- to 8-membered heterocyclic ring containing 1-4 nitrogen, oxygen or sulfur atoms, e.g., imidazolyl, imidazolinyl, pyrrolidinyl, morpholinyl, azetidinyl, pyridyl, piperidino, piperidyl, piperazinyl or lower alkyl-piperazinyl; substituted or unsubstituted amide; amide-lower alkyl, e.g., $-CH_2-CH(NH_2)-C(O)-NH_2$), wherein alkyl may be linear or cyclic, e.g., cyclopropylene; and the alkyl in any of the substituents above may optionally be substituted with $-NR_8R_9$, wherein $R_8$ and $R_9$ are as defined above;

X is C=O or C=S, with the proviso that then the dashed line bonding X to N is absent, so that X is bound to the adjacent N via a single bond and with the proviso that then y is 1 and R is hydrogen or an organic moiety that can be bound to nitrogen, or

X is ($CR_7$), wherein $R_7$ is hydrogen or an organic moiety, such as $C_1$-$C_7$lower alkyl; amino; or amino alkyl, wherein the alkyl may be unsubstituted or substituted with halo, e.g., methyl, ethyl, propyl or trifluoromethyl; lower alkoxy, e.g., methoxy; or cycloalkyl, e.g., cyclopropyl; with the proviso that then the dashed line bonding X to N is a bond, so that X is bound to the adjacent N via a double bond; and with the proviso that then y is zero, or y is 1 and then -R is $\rightarrow$O;

$R_2$ is hydrogen;

$R_3$ is unsubstituted or substituted $C_5$-$C_{14}$heterocyclyl, e.g., thienyl, benzo[1,3]dioxolo, indolyl, benzofuranyl or pyridiyl; unsubstituted or substituted $C_5$-$C_{14}$aryl, e.g., phenyl or phenyl substituted with up to 4 substituents, preferably up to 3 substituents, which are the same or different and are selected from halo, e.g., Cl or F; hydroxy; $C_1$-$C_4$lower alkoxy, e.g., methoxy; lower alkyl, e.g., methyl; or $-(C_1-C_4)_mNR_8R_9$,
wherein
m is 0 or 1; and
$R_8$ and $R_9$ , are as defined above, e.g., piperazinyl, methylpiperazinyl, morpholinyl or pyrrolidinyl;

$R_4$ is hydrogen or halo, e.g., fluoro or chloro;

$R_5$ is hydrogen; and

$R_6$ is hydrogen, amino, amino alkyl or alkylamido, e.g., methylamido $-NHC(O)-CH_3$), with the proviso that $R_3$ cannot be unsubstituted phenyl unless $R_1$ is phenyl substituted with an heterocyclic ring;

wherein the prefix 'lower' denotes a radical having 1 carbon atom up to and including a maximum of 7 carbon atoms or a pharmaceutically acceptable salts thereof and use of compounds of formula (I) in the manufacture of pharmaceutical preparations for the treatment of protein kinase dependent diseases.

The present invention is also directed to pharmaceutical preparations comprising an imidazoquinoline compound of the formula (I), especially for the treatment of a protein kinase dependent disease.

The general terms used hereinbefore and hereinafter preferably have within the context of this disclosure the following meanings, unless otherwise indicated:

The prefix "lower" denotes a radical having 1 carbon atom up to and including a maximum of 7 carbon atoms, especially 1 carbon atom up to and including a maximum of 4 carbon atoms, the radicals in question being either linear or branched with single- or multiple-branching.

Lower alkyl, e.g., is methyl, ethyl, *n*-propyl, *sec*-propyl, n-butyl, isobutyl, *sec*-butyl, *tert*-butyl, *n*-pentyl, *n*-hexyl or *n*-heptyl.

Halo or halogen is preferably fluoro, chloro, bromo or iodo, most preferably fluoro, chloro or bromo.

Alkyl preferably has up to 20 carbon atoms, more preferably up to 12 carbon atoms and is linear or branched one or more times; preferred is lower alkyl, especially $C_1$-$C_4$alkyl. Alkyl is linear or cyclic and unsubstituted or substituted by one or more substituents independently selected from those mentioned below under ''substituted". Unsubstituted alkyl, preferably lower alkyl, or hydroxyalkyl, especially hydroxy-lower alky, e.g., 2-hydroxyethyl; or cycloalkyl, e.g., cyclopropyl.

Aryl-lower alkyl is preferably lower alkyl that is substituted (preferably terminally or in 1-position) by unsubstituted or substituted aryl as defined below, especially phenyl-lower alkyl, such as benzyl or phenylethyl, especially 1-phenylethyl.

Heterocyclyl-lower alkyl is preferably lower alkyl that Is substituted (preferably terminally) by unsubstituted or substituted heterocyclyl as defined below.

Cycloalkyl-lower alkyl is preferably lower alkyl that is substituted (preferably terminally) by unsubstituted or

substituted cycloalkyl as defined below.

Alkenyl is preferably a moiety with one or more double bonds and preferably has 2-20 carbon atoms, more preferably up to 12 carbon atoms; it is linear or branched one or more times (as far as possible in view of the number of carbon atoms). Preferred is $C_2$-$C_7$alkenyl, especially $C_3$-$C_4$alkenyl, such as allyl or crotyl. Alkenyl can be unsubstituted or substituted by one or more, more especially up to three of the substituents mentioned below under "substituted". Substituents, such as amino or hydroxy (with free dissociable hydrogen) preferably are not bound to carbon atoms that participate at a double bond, and also other substituents that are not sufficiently stable are preferably excluded. Unsubstituted alkenyl, in particular, $C_2$-$C_7$alkenyl is preferred.

Alkynyl is preferably a moiety with one or more triple bonds and preferably has 2-20 carbon atoms, more preferably up to 12 carbon atoms; it is linear of branched one or more times (as far as possible in view of the number of carbon atoms). Preferred is $C_2$-$C_7$alkynyl, especially $C_3$-$C_4$alkynyl, such as ethynyl or propyn-2-yl. Alkynyl can be unsubstituted or substituted, by one or more, more especially up to three of the substituents mentioned below under "substituted". Substituents, such as amino or hydroxy (with free dissociable hydrogen) preferably are not bound to carbon atoms that participate at a triple bond, and also other substituents that are not sufficiently stable are preferably excluded. Unsubstituted alkynyl, in particular, $C_2$-$C_7$alkynyl is preferred.

Aryl, preferably, has a ring system of not more than 20 carbon atoms, especially not more than 16 carbon atoms, is preferably mono-, bi- or tric-cyclic, and is unsubstituted or substituted as defined below under "substituted". For example, aryl is selected from phenyl, naphthyl, indenyl, azulenyl and anthryl.

In arylcarbonylamino, aryl is preferably aryl as defined in the last paragraph, especially benzoylamino.

Heteroaryl is preferably a heteroaryl radical selected from the group consisting of oxiranyl, azirinyl, 1,2-oxathiolanyl, imidazolyl, thienyl, furyl, tetrahydrofuryl, indolyl, azetidinyl, pyranyl, thiopyranyl, thianthrenyl, isobenzofuranyl, benzofuranyl, chromenyl, 2*H*-pyrrolyl, pyrrolyl, pyrrolinyl, pyrrolidinyl, imidazolyl, imidazolidinyl, benzimidazolyl, pyrazolyl, pyrazinyl, pyrazolidinyl, pyranyol, thiazolyl, isothiazolyl, dithiazolyl, oxazolyl, isoxazolyl, pyridyl, pyrazinyl, pyrimidinyl, piperidyl, piperazinyl, pyridazinyl, morpholinyl, thiomorpholinyl, indolizinyl, isoindolyl, 3*H*-indolyl, indolyl, benzimidazolyl, cumaryl, indazolyl, triazolyl, tetrazolyl, purinyl, 4*H*-quinolizinyl, isoquinolyl, quinolyl, tetrahydroquinolyl, tetrahydroisoquinolyl, decahydroquinolyl, octahydroisoquinolyl, benzofuranyl, dibenzofuranyl, benzothiophenyl, dibenzothiophenyl, phthalazinyl, naphthyridinyl, quinoxalyl, quinazolinyl, quinazolinyl, cinnolinyl, pteridinyl, carbazolyl, β-carbolinyl, phenanthridinyl, acridinyl, perimidinyl, phenanthrolinyl, furazanyl, phenazinyl, phenothiazinyl, phenoxazinyl, chromenyl, isochromanyl and chromanyl; each of these radicals being unsubstituted or substituted by one to two radicals as defined below. Unsubstituted or substituted heterocyclyl or lower alkyl heterocyclyl, e.g., benzo[1,3]-dioxlo, indolyl, benzofuranyl, thienyl, pyridyl, imidazolinyl, lower alkyl imidazolinyl, morpholinyl, piperazinyl, lower alkyl piperazinyl, piperidino, piperidyl, pyrrolidinyl and azetidinyl, are preferred.

Cycloalkyl is preferably $C_3$-$C_{10}$cycloalkyl, especially cyclopropyl, dimethylcyclopropyl, cyclobutyl, cyclopentyl, cyclohexyl or cycloheptyl; cycloalkyl being unsubstituted or substituted by one or more substituents, especially 1-3 substituents independently selected from the group consisting of the substituents defined below under "substituted".

Cycloalkenyl is preferably $C_5$-$C_{10}$cycloalkenyl, especially cyclopentenyl, cyclohexenyl or cycloheptenyl; cycloalkenyl being unsubstituted or substituted by one or more substituents, especially 1-3 substituents, independently selected from the group consisting of the substituents defined below under "substituted".

Preferably, only up to five, more preferably up to three of $R_2$, $R_3$, $R_4$, $R_5$, $R_6$ and $R_7$ as defined herein are/is other than hydrogen.

A very preferred group of compounds of formula (I) are those wherein $R_3$ is one of the organic moieties other than hydrogen, especially those mentioned as being preferred above.

"Substituted", wherever used for a moiety and if not defined otherwise in the claims , means that one or more hydrogen atoms in the respective moiety, especially up to 5 hydrogen atoms, more especially up to three of the hydrogen atoms are replaced independently of each other by the corresponding number of substituents, which preferably are independently selected from the group consisting of lower alkyl, e.g., methyl, ethyl or propyl; halo, e.g., F, Cl, Br or I; halo-lower alkyl, e.g., trifluoromethyl; hydroxy; carboxy; lower alkoxy, e.g., methoxy; phenyl-lower alkoxy; lower alkanoyloxy; lower alkanoyl; hydroxy-lower alkyl, e.g., hydroxymethyl or 2-hydroxyethyl; amino; mono- or di-substituted amino; cyclic amino; amino-lower alkyl, e.g., aminomethyl, 2-aminoethyl or 3-aminopropyl; *N*-lower alkylamino; *N,N*-di-lower alkylamino; *N*-phenyl-lower alkylamino; *N,N-bis*(phenyl-lower alkyl)-amino; amino lower alkoxy; lower alkanoylamino; benzoylamino; carbamoyl-lower alkoxy; *N*-lower alkylcarbamoyl-lower alkoxy or *N,N*-di-lower alkylcarbamoyl-lower alkoxy; amidino; *N*-hydroxy-amidino; guanidino; amino-lower alkyl, e.g., aminomethyl or 2-aminoethyl; amidino-lower alkyl, e.g., 2-amidinoethyl; *N*-hydroxyamidino-lower alkyl, e.g., *N*-hydroxy-amidino-methyl or -2-ethyl; carboxy; lower alkoxycarbonyl; phenyl-, naphthyl- or fluorenyl-lower alkoxycarbonyl, e.g., benzyloxycarbonyl; lower alkanoyl; sulfo; lower alkanesulfonyl, e.g., methanesulfonyl ($CH_3$-$S(O)_2$-); sulfonamide ($NH_2$-$S(O)_2$-); dioxolo; phosphono (-P(=O)$(OH)_2$); hydroxy-lower alkoxy phosphoryl or di-lower alkoxyphosphoryl; carbamoyl; mono- or di-lower alkylcarbamoyl; sulfamoyl; sulfamide; mono- or di-lower alkylaminosulfonyl; cyano-lower alkyl, e.g., cyanomethyl; and cyano; $C_5$-$C_{16}$aryl, e.g., phenyl or naphthyl, where $C_5$-$C_{16}$aryl is substituted with any of the substituents defined above, and especially is phenyl

which is unsubstituted or substituted with up to 4 substituents, preferably up to 2 substituents, wherein the substituents are the same or different and are independently selected from halo, e.g., Cl or F; cyano; cyano lower alkyl, e.g., cyanomethyl, cyanoethyl and cyanopropyl; lower alkyl; lower alkoxy; amino-lower alkyl; amino-lower alkoxy; amino-lower alkyl sulfanyl; or thiol-lower alkyl, wherein the amino group can be mono- or di-substituted, e.g., $-(C_1-C_7)_mNR_8R_9$; or $-O-(C_1-C_7)_mNR_8R_9$,
wherein

m is 0 or 1; and

$R_8$ and $R_9$ can be the same or different and are independently H; lower alkyl, e.g., methyl, ethyl or propyl; lower cycloalkyl, e.g., cyclopropyl, or

$R_8$ and $R_9$ , together with the N atom, form a 3- to 8-membered heterocyclic ring containing 1-4 nitrogen, oxygen or sulfur atoms, e.g., azetidinyl, pyrrolidinyl, piperidino, morpholinyl, imidazolinyl, piperazinyl or lower alkyl-piperazinyl.

"Substituted" also includes amino-carbonyl-lower alkyl, e.g., $R_8R_9$-N-C(O)-$CH_2$-, wherein $R_8$ and $R_9$ are as defined above; heterocyclyl; heterocyclyl-lower alkyl; heterocyclyl-lower alkoxy or heterocyclyl-lower alkanesulfanyl, wherein the heterocyclyl is a substituted or unsubstituted 3- to 8-membered heterocyclic ring containing 1-4 nitrogen, oxygen or sulfur atoms, e.g., imidazolyl, imidazolinyl, pyrrolidinyl, morpholinyl, azetidinyl, pyridyl, piperidino, piperidyl, piperazinyl or lower alkyl-piperazinyl; $C_3$-$C_{10}$cycloalkyl, e.g., cyclopropyl or cyclohexyl; hydroxy$C_3$-$C_8$cycloalkyl, e.g., hydroxy-cyclohexyl; heteroaryl with 4 or 6 ring atoms and 1-4 ring heteroatoms selected from O, N and S, especially furyl; 1,4 oxazinyl; or pyridyl; or -$NR_8R_9$, wherein $R_8$ and $R_9$ can be the same or different and are independently H; lower alkyl, e.g., methyl, ethyl or propyl; lower cycloalkyl, e.g., cyclopropyl; or the $R_8$ and $R_9$ can, with the N atom, form a 3- to 8-membered heterocyclic ring containing 1-4 nitrogen, oxygen or sulfur atoms, e.g., azetidinyl, pyrrolidinyl, piperidino, morpholinyl, imidazolinyl, piperazinyl or lower alkyl-piperazinyl. It goes without saying that substituents are only at positions where they are chemically possible, the person skilled in the art being able to decide (either experimentally or theoretically) without inappropriate effort which substitutions are possible and which are not. For example, amino or hydroxy groups with free hydrogen may be unstable if bound to carbon atoms with unsaturated, e.g., olefinic, bonds.

Salts are preferably the pharmaceutically acceptable salts of compounds of formula (I) if they are carrying salt-forming groups.

Salt-forming groups in a compound of formula (I) are groups or radicals having basic or acidic properties. Compounds having at least one basic group or at least one basic radical, e.g., amino; a secondary amino group not forming a peptide bond or a pyridyl radical, may form acid addition salts, e.g., with inorganic acids, such as hydrochloric acid, sulfuric acid or a phosphoric acid; or with suitable organic carboxylic or sulfonic acids, e.g., aliphatic mono- or di-carboxylic acids, such as trifluoroacetic acid, acetic acid, propionic acid, glycolic acid, succinic acid, maleic acid, fumaric acid, hydroxymaleic acid, malic acid, tartaric acid, citric acid or oxalic acid; or amino acids, such as arginine or lysine; aromatic carboxylic acids, such as benzoic acid; 2-phenoxy-benzoic acid; 2-acetoxy-benzoic acid; salicylic acid; 4-aminosalicylic acid; aromatic-aliphatic carboxylic acids, such as mandelic acid or cinnamic acid; heteroaromatic carboxylic acids, such as nicotinic acid or isonicotinic acid; aliphatic sulfonic acids, such as methane-, ethane- or 2-hydroxyethanesulfonic acid; or aromatic sulfonic acids, e.g., benzene-, p-toluene- or naphthalene-2-sulfonic acid. When several basic groups are present mono- or poly-acid addition salts may be formed.

Compounds of formula (I) having acidic groups, a carboxy group or a phenolic hydroxy group, may form metal or ammonium salts, such as alkali metal or alkaline earth metal salts, e.g., sodium, potassium, magnesium or calcium salts; or ammonium salts with ammonia or suitable organic amines, such as tertiary monoamines, e.g., triethylamine or tri(2-hydroxyethyl)-amine, or heterocyclic bases, e.g., *N*-ethyl-piperidine or *N*,*N'*-dimethylpiperazine. Mixtures of salts are possible.

Compounds of formula (I) having both acidic and basic groups can form internal salts.

For the purposes of isolation or purification, as well as in the case of compounds that are used further as intermediates, it is also possible to use pharmaceutically-unacceptable salts, e.g., the picrates. Only pharmaceutically-acceptable, non-toxic salts may be used for therapeutic purposes, however, and those salts are therefore preferred.

Owing to the close relationship between the novel compounds in free form and in the form of their salts, including those salts that can be used as intermediates, e.g., in the purification of the novel compounds or for the identification thereof, any reference hereinbefore and hereinafter to the free compounds shall be understood as including the corresponding salts, where appropriate and expedient.

Where the plural form is used for compounds, salts, pharmaceutical preparations, diseases and the like, this is intended to mean also a single compound, salt or the like.

Any asymmetric carbon atom may be present in the (*R*)-, (*S*)- or (*R,S*)-configuration, preferably in the (*R*)- or (*S*)-configuration. Substituents at a double bond or a ring may be present in *cis*- (=*Z*-) or *trans* (=*E*-) form. The compounds may thus be present as mixtures of isomers or preferably as pure isomers, preferably as enantiomer-pure diastereomers

or pure enantiomers.

The terms "treatment" or "therapy" refer to the prophylactic or preferably therapeutic including, but not limited to, palliative, curing, symptom-alleviating, symptom-reducing, kinase-regulating and/or kinase-inhibiting, treatment of said diseases, especially of the diseases mentioned below.

Where subsequently or above the term "use" is mentioned (as verb or noun) (relating to the use of a compound of the formula (I) or a pharmaceutically acceptable salt thereof), this includes any one or more of the following embodiments of the invention, respectively: the use for the manufacture of pharmaceutical compositions for use in the treatment of a protein kinase dependent disease, and one or more compounds of the formula (I) for use in the treatment of a protein kinase dependent disease, as appropriate and expedient and if not stated otherwise. In particular, diseases to be treated and are thus preferred for "use" of a compound of formula (I) are selected from protein kinase dependent ("dependent" meaning also "supported", not only "solely dependent") diseases mentioned herein, especially proliferative diseases mentioned herein, more especially any one or more of these or other diseases that depend on one or more of PKB, ALK, S6K1 and RET or any combinations of two or more of these, or a mutant of any one or more of these, and a compound of the formula (I) can therefore be used in the treatment of a kinase dependent disease, especially a disease depending on one or more of the kinases mentioned above and below, where (especially in the case of aberrantly highly-expressed, constitutively activated and/or mutated kinases) said kinase-dependent disease is dependent on the activity of one or more of the said kinases or the pathways they are involved.

The compounds of formula (I) have valuable pharmacological properties and are useful in the treatment of protein kinase dependent diseases, e.g., as drugs to treat proliferative diseases.

## Preferred Embodiments of the Invention

With the groups of preferred compounds of formula (I) mentioned hereinafter, definitions of substituents from the general definitions mentioned hereinbefore may reasonably be used, e.g., to replace more general definitions with more specific definitions or especially with definitions characterized as being preferred. The invention relates especially to a compound of the formula (I)
wherein
each of x and y is, independently of the other, 0 or 1;

$R_1$ is substituted or unsubstituted phenyl where the phenyl is substituted with up to 4 substituents, preferably up to 3 substituents, where the substituents are the same or different and are independently selected from halo, e.g., F or Cl; $C_1$-$C_7$lower alkyl, which may be unsubstituted or substituted with halo, especially methyl, ethyl, propyl or trifluoromethyl; cyano; cyano-lower alkyl, e.g., cyanomethyl, cyanoethyl or cyanopropyl; amino; amino-lower alkyl, wherein the amino group can be mono- or di-substituted, e.g., $-(C_1\text{-}C_7)_mNR_8R_9$ or $-O-(C_1\text{-}C_7)_mNR_8R_9$,
wherein
m is 0 or 1; an
$R_8$ and $R_9$ can be the same or different and are independently H; lower alkyl, e.g., methyl, ethyl or propyl; lower cycloalkyl, e.g., cyclopropyl, or
$R_8$ and $R_9$ , together with the N atom, form a 3- to 8-membered heterocyclic ring containing 1-4 nitrogen, oxygen or sulfur atoms, e.g., imidazolinyl, imidazolinylethyl, piperazinyl or lower alkyl-piperazinyl; amino-carbonyl-lower alkyl, e.g., $R_8R_9$-N-C(O)-$CH_2$-, wherein $R_8$ and $R_9$ are as defined above; heterocyclyl; heterocyclyl-lower alkyl; heterocyclyl-lower alkoxy; or hoterocyclyl-lower alkanesulfanyl, wherein the heterocyclyl is a 3- to 8-membered heterocyclic ring containing 1-4 nitrogen, oxygen or sulfur atoms, e.g., imidazolinyl, imidazolinyl, imidazolinyl-ethyl, piperazinyl or lower alkyl-piperazinyl; substituted or unsubstituted amide; amide-lower alkyl, e.g., $-CH_2-CH(NH_2)-C(O)-NH_2$, wherein alkyl may be linear or cyclic, e.g., cyclopropylene; and the alkyl in any of the substituents above may optionally be substituted with $-NR_8R_9$, wherein $R_8$ and $R_9$ are as defined above;

X is ($CR_7$), wherein $R_7$ is hydrogen; lower alkyl, e.g., methyl or ethyl; amino; or amino alkyl, with the proviso that then the dashed line bonding X to N is a bond, so that X is bound to the adjacent N via a double bond, and with the proviso that then y is zero, or y is 1 and then -R is $\rightarrow$O;

$R_2$ is hydrogen;

$R_3$ is unsubstituted or substituted $C_5$-$C_{14}$heterocyclyl, e.g., thienyl, benzo[1,3]dioxolo, indolyl, benzofuranyl or pyridiyl; unsubstituted or substituted $C_5$-$C_{14}$aryl, e.g., phenyl or phenyl substituted with up to 4 substituents, preferably up to 3 substituents, which are the same or different and are selected from halo, e.g., Cl or F; hydroxy; $C_1$-$C_4$lower alkoxy, e.g., methoxy; lower alkyl, e.g., methyl; or $-(C_1\text{-}C_4)_mNR_8R_9$.
wherein

m is 0 or 1; and
$R_8$ and $R_9$ are as defined above, e.g., piperazinyl, methylpiperazinyl, morpholinyl or pyrrolidinyl;

$R_4$ is hydrogen or halo, (e.g. fluoro or chloro);

$R_5$ is hydrogen; and

$R_6$ is hydrogen, with the proviso that $R_3$ cannot be unsubstituted phenyl unless $R_1$ is phenyl substituted with an heterocyclic ring;

or a pharmaceutically acceptable salt thereof, as such or especially for use in the diagnostic or therapeutic treatment of a warm-blooded animal, especially a human.

Also preferred are pharmaceutical preparations comprising an imidazoquinoline compound of the formula (I), or a pharmaceutically acceptable salt thereof, especially for the treatment of a protein kinase dependent disease; a process for the manufacture of the novel imidazoquinoline compounds of the formula (I), or a pharmaceutically acceptable salt thereof and novel starting materials and intermediates for their manufacture. Especially preferred is the use of a compound of formula (I), or a pharmaceutically acceptable salt thereof, in the manufacture of a pharmaceutical preparation for the treatment of a protein kinase dependent disease.

Also preferred is a compound of the formula (I), or a pharmaceutically acceptable salt thereof, as shown above for use in the treatment of a protein kinase dependent disease, especially one depending on PKB, ALK, S6K1 or RET and (especially aberrantly highly expressed or activated) PKB, ALK, S6K1 or RET-dependent disease or disease dependent on the activation of the PKB, ALK, S6K1 or RET pathways or disease dependent on any two or more of the kinases just mentioned.

Especially preferred is a compound of the formula (I), or a pharmaceutically acceptable salt thereof,
wherein
X is C=O; and
the other moieties are as defined under formula (I),
for use in the diagnostic or therapeutic treatment of a warm-blooded animal, especially a human.

Most preferred is the use in accordance with the present invention of a compound of the formula (I), or a pharmaceutically acceptable salt thereof, as exemplified hereinbelow under "Examples".

*RET kinase inhibition is determined as follows:* The baculovirus donor vector pFB-GSTX3 is used to generate a recombinant baculovirus that expresses the amino acid region 658-1072 (Swiss Prot No. Q9BTB0) of the intra-cytoplasmic kinase domain of human RET-Men2A which corresponds to the wild-type kinase domain of RET (wtRET) and RET-Men2B, which differs from the wtRET by the activating mutation in the activation loop M918T. The coding sequences for the cytoplasmic domain of wtRET and RET-Men2B are amplified by PCR from the plasmids pBABEpuro RET-Men2A and pBABEpuro RET-Men2B, respectively which are received from Dr. James Fagin, College of Medicine, University of Cincinnati. The amplified DNA fragments and the pFB-GSTX3 vector are made compatible for ligation by digestion with SalI and KpnI. Ligation of these DNA fragments results in the baculovirus donor plasmid pFB-GX3-HET-Men2A and pFB-GX3-RET-Men2B, respectively.

*Production of virus:* Transfer vectors containing the kinase domains are transfected into the DH10Bac cell line (GIBCO) and plated on selective agar plates. Colonies without insertion of the fusion sequence into the viral genome (carried by the bacteria) are blue. Single, white colonies are picked and viral DNA (bacmid) isolated from the bacteria by standard plasmid purification procedures. Sf9 cells or Sf21 (American Type Culture Collection) cells are then transfected in 25 $cm^2$ flasks with the viral DNA using Cellfectin reagent.

*Determination of small scale protein expression in Sf9 cells:* Virus-containing media is collected from the transfected cell culture and used for infection to increase its titer. Virus-containing media obtained after two rounds of infection is used for large-scale protein expression. For large-scale protein expression 100 $cm^2$ round tissue culture plates are seeded with $5x10^7$ cells/plate and infected with 1 mL of virus-containing media (approximately 5 MOIs). After 3 days, the cells are scraped off the plate and centrifuged at 500 rpm for 5 minutes. Cell pellets from 10-20, 100 $cm^2$ plates, are re-suspended in 50 mL of ice-cold lysis buffer (25 mM tris-HCl, pH 7.5, 2 mM EDTA, 1% NP-40, 1 mM DTT, 1 mM P MSF). The cells are stirred on ice for 15 minutes and then centrifuged at 5,000 rpms for 20 minutes.

*Purification of GST-tagged proteins:* The centrifuged cell lysate is loaded onto a 2 mL glutathione-sepharose column (Pharmacia) and washed 3 x with 10 mL of 25 mM tris-HCl, pH 7.5, 2 mM EDTA, 1 mM DTT, 200 mM NaCl. The GST-tagged proteins are then eluted by 10 applications (1 mL each) of 25 mM tris-HCl, pH 7.5, 10 mM reduced-glutathione, 100 mM NaCl, 1 mM DTT, 10% glycerol and stored at -70°C.

*Measure of enzyme activity:* Tyrosine protein kinase assays with either purified GST-wtRET or GST-RET-Men2B protein are carried out in a final volume of 30 $\mu$L containing 15 ng of either GST-wtRET or GST-RET-Men2B protein, 20 mM tris-HCl, pH 7.5, 1 mM $MnCl_2$, 10 mM $MgCl_2$, 1 mM DTT, 3 $\mu$g/mL poly(Glu,Tyr) 4:1, 1% dimethyl sulfoxide

(DMSO), 2.0 μM ATP (γ-[$^{33}$P]-ATP 0.1 μCi). The activity is assayed in the presence or absence of inhibitors, by measuring the incorporation of $^{33}$P from (γ$^{33}$P] ATP into poly(Glu,Tyr) 4:1. The assay is carried out in 96-well plates at ambient temperature for 15 minutes under conditions described below and terminated by the addition of 20 μL of 125 mM EDTA. Subsequently, 40 μL of the reaction mixture are transferred onto Immobilon-PVDF membrane (Millipore) previously soaked for 5 minutes with methanol, rinsed with water, then soaked for 5 minutes with 0.5% $H_3PO_4$ and mounted on vacuum manifold with disconnected vacuum source. After spotting all samples, vacuum is connected and each well-rinsed with 200 μL 0.5% $H_3PO_4$. Membranes were removed and washed 4 x on a shaker with 1.0% $H_3PO_4$, once with ethanol. Membranes are counted after drying at ambient temperature, mounting in Packard TopCount 96-well frame, and addition of10 μL/well of Microscint TM (Packard). $IC_{50}$ values are calculated by linear regression analysis of the percentage inhibition of each compound in duplicate, at 4 concentrations (usually 0.01, 0.1, 1 and 10 μM). One unit of protein kinase activity is defined as 1 nmole of $^{33}$P ATP transferred from [γ$^{33}$P] ATP to the substrate protein/minute/mg of protein at 37°C.

| *$IC_{50}$ calculations* | |
|---|---|
| input | 3 x 4 μL stopped assay on Immobilon membrane, not washed |
| background (3 wells) | assay with $H_2O$ instead of enzyme |
| positive control (4 wells) | 3% DMSO instead of compound |
| bath control (1 well) | no reaction mix |

$IC_{50}$ values are calculated by logarithmic regression analysis of the percentage inhibition of each compound at 4 concentrations (usually 3- or 10-fold dilution series starting at 10 μM). In each experiment, the actual inhibition by reference compound is used for normalization of $IC_{50}$ values to the basis of an average value of the reference inhibitor:

$$\text{Normalized } IC_{50} = \text{measured } IC_{50} \text{ average ref. } IC_{50} / \text{measured ref. } IC_{50}$$

Example: Reference inhibitor in experiment 0.4 μM, average 0.3 μM

Test compound in experiment 1.0 μM, normalization: 0.3/0.4 = 0.75 μM

For example, staurosporine or a synthetic staurosporine derivative are used as reference compounds.

Using this protocol, the compounds of the formula (I) are found to show $IC_{50}$ values for RET inhibition in the range from 0.001-20 μM, preferably in the range from 0.01-2 μM.

*The efficacy of the compounds of formula (I) as inhibitors of PKB kinase activity can be demonstrated* as *follows:* To fully activate PKB, the enzyme is exposed to catalytic amounts of PDK1. GST-PKB [100 ng, specific activity (SA): 0.2 nmole/mg/min.] is incubated for 30 minutes at room temperature (RT) with purified recombinant GST-PDK1 (1 ng, SA: 2 nmole/min./mg). The activation is performed as follows: 0.1 μg of GST-PDK1 (0.05 μL) and 10 μg of GST-PKB (0.45 μL) are mixed in a total volume of 0.75 μL containing 15 μM ATP, 3 mM $MgCl_2$, 20 mM hepes (pH 7.6) for 30 minutes at RT. The reaction is subsequently stopped by adding 0.25 μL containing 30% gycerol ($^{w}/_{w}$) and 0.06 μL of 500 mM EDTA. 100-500 ng (0.01-0.05 μL) activated GST-PKB is incubated in a final volume of 30 μL with 10 μM of the RRPRTRSFS peptide, 10 mM Mg-acetate, 50 mM MOPS (PH 7.5), 1 mM DTT and 300 μg/mL BSA, 20 μM ATP (0.1 μCi γ-$^{33}$P-ATP). The reaction is carried out for 30 minutes at RT in the presence of either 1 % DMSO or the text compound of the formula (I) at the required concentration in 1% DMSO. The reaction is terminated by the addition of 20 μL 125 mM EDTA. Thirty (30) μL of each sample is spotted onto P81 Whatman and the paper squares processed. See Ferrari and Thomas, Methods Enzymol, Vol. 200, pp. 159-169 (1991). $IC_{50}$ values are calculated by linear regression analysis of the percentage inhibition of each compound in duplicate. $IC_{50}$ values for compounds of the formula (I) are in the range from 0.005-100 μM, for preferred compounds between 0.01 μM and 2 μM.

*The efficacy* of *the compounds of formula (I)* as *inhibitors of S6K1 activity can be demonstrated as follows:* The DNA fragment encoding ORF of human S6K1 (+28-1605, Genbank NM_003161) is amplified from human muscle cDNA library and modified by PCR to generate a C-terminal truncated form (amino acid region 1-421) with an activating mutation of T412E, hereinafter S6K1ΔCT/T412E. The DNA fragment encoding S6K1ΔCT/T412E is subcloned to the baculovirus donor vector pFastBacHT A to generate a recombinant baculovirus that expresses S6K1ΔCT/T412E. Transfer vectors for S6K1ΔCT/T412E are transfected into the DH1 0Bac cell line (GIBCO). After selection of white colonies, they are used for viral DNA (bacmid) preparation. Sf9 cells (American Type Culture Collection) cells are then transfected with the viral DNA using Cellfectin reagent. Virus-containing media is collected from the transfected cell culture and is used for infection to increase its titer. For large-scale protein expression, Sf9 cells are co-infected with S6K1ΔCT/T412E

and GST-PDK1 virus-containing media with MOI of 2.25 and 1, respectively. After 3 days, the cells expressing the recombinant enzyme are resuspended in the culture medium with a sterilized cell scraper and harvested by centrifugation. The cells are resuspended in 5 volumes of cell pellet of Extraction Buffer (50 mM MOPS pH 7.0, containing 300 mM NaCl, 0.05% Tween-20, 10 mM sodium pyrophosphate, 50 mM β-glycerophosphate, 0.2 mM cantharidic acid and 1 x complete-EDTA protease inhibitor cocktail) and then disrupted with 15 strokes of a Dounce homogenizer followed by sonication on ice. The homogenate is centrifuged at 4°C for 60 minutes at 48,000 x g. The supernatant is clarified with a 5 μm filter and subjected to purification with TALON Superflow Resin (Clontech). First, 1 mL of TALON resin per 5 mL of the supernatant was equilibrated with the Extraction Buffer and resuspended in the supernatant followed by gentle agitation for 20 minutes at RT. After centrifugafion at 700 x g for 5 minutes, supernatant is removed. The resin is washed twice by adding 10 bed volumes of the Extraction Buffer, agitating for 10 minutes at RT, centrifugating at 700 x g for 5 minutes, and discarding the supernatant. The resin is then resuspended with the same volume of the Extraction Buffer and transferred to an disposable chromatography column. The packed column is washed once with 10 bed volumes of the Extraction Buffer and additionally with the same volume of Wash Buffer (5 mM imidazole in the Extraction Buffer). Then, the His-tagged S6K1ΔCT/T412E is eluted with four bed volumes of Elution Buffer (150 mM imidazole in the Extraction Buffer).

*Measure of enzyme activity:* The kinase assay with purified S6K1ΔCT/T412E is performed with TR-FRET (Time-Resolved Fluorescene Resonance Energy Transfer) technology. First, ATP-dependent phosphorylation of the biotinylated substrate peptide corresponding to residues 229-242 of S6 ribosomal protein (Biot-AKRRRLSSLRASTS) is carried out for 1 hour at 37°C in 382-well plates under humidified air. The kinase reaction mixture of the final volume of 9 μL contains 2.57 ng of purified S6K1ΔCT/T412E protein, 50 mM MOPS (pH 7.0), 0.1 mM EGTA, 0.01 mM $Na_3VO_4$, 0.05% Tween-20, 0.01% BSA, 1 mM DTT, 10 mM $MgCl_2$. 3 μM ATP, 0.5 μM biotinylated substrate peptide, 1% of DMSO or the text compounds of the formula (I) at the required concentration in 1% DMSO. For measurement of background, 20 mM of EDTA is added to the reaction mixture. The kinase reaction is terminated by addition of 6 μL of Detection Mixture [50 mM MOPS (pH 7.0), 25 mM EGTA, 200 mM NaCl, 0.05% Tween-20, 0.01% BSA, 5 μg/mL SureLight Allophycocyanin-streptavidin (Perkin Elmer), 2 nM LANCE Eu-W1024 anti-rabbit IgG (Perkin Elmer), 0.2 μg/mL Phospho-S6 Ribosomal Protein (Ser235/236) Antibody (Cell Signaling Technology)] and the plates are incubated for 2 hours. Phosphorylated peptide indicative of kinase activity is quantitated by exciting the sample at 330 nm and comparing donor emission at 615 nm and acceptor emission at 665 nm by 2101 EnVision HTS. multilabel plate reader (Perkin Elmer). The ratio of acceptor counts divided by donor counts is used as a normalized parameter for both signal and background. $IC_{50}$ values are calculated by linear regression analysis of the percentage inhibition of each compound in triplicate, at 7 concentrations (usually 0.01, 0.001, 0.03, 0.1, 0.3, 1 and 10 μM).

*The efficacy of the compounds of formula (I) as inhibitors of ALK tyrosine kinase activity can be demonstrated as follows:* The inhibition of ALK tyrosine kinase activity can be demonstrated using known methods, e.g., using the recombinant kinase domain of the ALK in analogy to the VEGF-R kinase assay. See Wood et al., Cancer Res, Vol. 60, No. 8, pp. 2178-2189 (2000). *In vitro* enzyme assays using GST-ALK protein tyrosine kinase are performed in 96-well plates as a filter binding assay in 20 mM tris·HCl, pH=7.5, 3 mM $MgCl_2$, 10 mM $MnCl_2$, 1 mM DTT, 0.1 μCi/assay (=30 μL) [γ-$^{33}$P]-ATP, 2 μM ATP, 3 μg/mL poly (Glu, Tyr 4:1) Poly-EY (Sigma P-0275), 1% DMSO, 25 ng ALK enzyme. Assays are incubated for 10 minutes at ambient temperature. Reactions are terminated by adding 50 μL of 125 mM EDTA, and the reaction mixture is transferred onto a MAIP Multiscreen plate (Millipore, Bedford, MA, USA), previously wet with methanol, and re-hydrated for 5 minutes with $H_2O$. Following washing (0.5% $H_3PO_4$), plates are counted in a liquid scintillation counter. $IC_{50}$ values are calculated by linear regression analysis of the percentage inhibition. Compared with the control without inhibitor, the compounds of formula (I) inhibit the enzyme activity by 50% ($IC_{50}$), e.g., in a concentration of from 0.001-0.5 μM, especially from 0.1-0.8 μM.

The compounds of formula (I) that inhibit the protein kinase activities mentioned, especially tyrosine and/or the serine/threonine protein kinases mentioned above, can therefore be used in the treatment of protein kinase dependent diseases, especially diseases depending on PKB, ALK, S6K1 or RET and (especially aberrantly highly-expressed or activated) PKB, ALK, S6K1 or RET-dependent disease or disease dependent on the activation of the PKB, ALK, S6K1 or RET pathways or any combination of two or more of the mentioned kinases.

Protein kinase dependent diseases are especially proliferative diseases, preferably a benign or especially malignant tumor, more preferably carcinoma of the brain, kidney, liver, adrenal gland, bladder, breast, stomach (especially gastric tumors), ovaries, colon, rectum, prostate, pancreas, lung, vagina, thyroid, sarcoma, glioblastomas, multiple myeloma or gastrointestinal cancer, especially colon carcinoma or colorectal adenoma, or a tumor of the neck and head, an epidermal hyperproliferation, especially psoriasis, prostate hyperplasia, a neoplasia, especially of epithelial character, preferably mammary carcinoma, or a leukemia, especially as far as c-Met is involved. They are able to bring about the regression of tumors and to prevent the formation of tumor metastases and the growth of (also micro)metastases. In addition, they can be used in epidermal hyperproliferation, e.g., psoriasis; in prostate hyperplasia; in the treatment of neoplasias, especially of epithelial character, e.g., mammary carcinoma; and in leukemias. It is also possible to use the compounds of formula (I) in the treatment of diseases of the immune system insofar as several or, especially, individual

tyrosine protein kinases and/or (further) serine/threonine protein kinases are involved; furthermore, the compounds of formula (I) can be used also in the treatment of diseases of the central or peripheral nervous system where signal transmission by at least one tyrosine protein kinase and/or (further) serine/threonine protein kinase is involved.

There are also experiments to demonstrate the antitumor activity of compounds of the formula (I) *in vivo.*

Female Balb/c hairless mice with subcutaneously (s.c.) transplanted human bladder tumors T24 can be used to determine the anti-tumor activity. On day 0, with the animals under peroral forene narcosis, approximately 25 mg of a solid tumor are placed under the skin on the animals' left flank and the small incised wound is closed by means of suture clips. On day 6 after the transplantation, the mice are divided at random into groups of 6 animals and treatment commences. The treatment is carried out for 15 days with peroral, intravenous or intraperitoneal administration once daily (or less frequently) of a compound of formula (I) in DMSO/Tween80/sodium chloride solution in the various doses. The tumors are measured twice a week with a slide gauge and the volume of the tumors is calculated.

As an alternative to cell line A-431, other cell lines may also be used in the same manner, e.g.,

- The MDA-MB 468 breast adenocarcinoma cell line [ATCC No. HTB 132; see also In Vitro, Vol. 14, pp. 911-915 (1978)];
- The MDA-MB 231 breast carcinoma cell line [ATCC No. HTB-26; see also In Vitro, Vol. 12, p. 331 (1976)];
- The Colo 205 colon carcinoma cell line [ATCC No. CCL 222; see also Cancer Res, Vol. 38, pp. 1345-1355 (1978)];
- The DU145 prostate carcinoma cell line DU 145 [ATCC No. HTB 81; see also Cancer Res, Vol. 37, pp. 4049-4058 (1978)];
- The PC-3 prostate carcinoma cell line PC-3 [especially preferred; ATCC No. CRL 1435; see also Cancer Res, Vol. 40, pp. 524-534 (1980)].
- The A549 human lung adenocarcinoma [ATCC No. CCL 185; see also Int J Cancer, Vol. 17, pp. 62-70 (1976)];
- The NCI-H596 cell line [ATCC No. HTB 178; see also Science, Vol. 246, pp. 491-494 (1989)]; and
- The pancreatic cancer cell line SUIT-2 [see Tomioka et al., Cancer Res, Vol. 61, pp. 7518-7524 (2001)].

The compounds of the formula (I) can be prepared according to the methods:

In one preferred embodiment, a compound of formula (I) is prepared by reacting a compound of the formula (II)

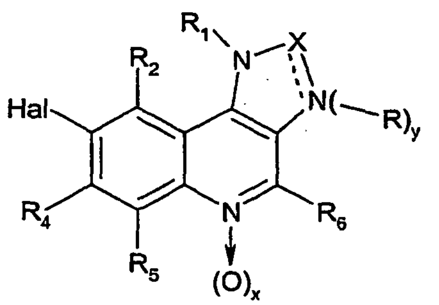

**(II)**

with a boronic acid,
wherein
Hal refers to halogen preferably bromine; and
x, y, X, $R_1$, $R_2$, $R_4$, $R_5$, R and $R_6$ are as defined above; and
if desired, transforming an obtainable compound of formula (I) into a different compound of formula (I), transforming a salt of an obtainable compound of formula (I) into the free compound or a different salt, or an obtainable free compound of formula (I) into a salt; and/or separating an obtainable mixture of isomers of compounds of formula (I) into the individual isomers.

In the following, more detailed description of the preferred process conditions, x, y, $R_1$, $R_2$, $R_3$, $R_4$, $R_5$, $R_6$, $R_7$, X and R have the meanings given for compounds of the formula (I), if not indicated otherwise.

**Starting materials**

A compound of formula (II) of the first preferred embodiment is prepared by reacting a compound of formula (XVI)

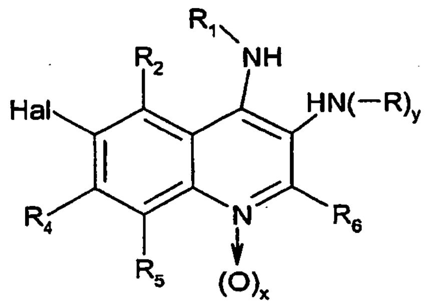

(XVI)

,

wherein
x, y, $R_1$, $R_2$, $R_4$, $R_5$ and $R_6$ are as mentioned for a compound of the formula (I); and
R are as defined below under a), b) or c), respectively,

a) for the manufacture of a compound of the formula II wherein X is C=O and the dashed line in formula (I) bonding X to N is absent, y is 1 and R is hydrogen or an organic moiety that can be bound to nitrogen, with an active derivative of a compound of the formula (III)

A-X-A (III),

wherein
X is C=O; and
each A, independently of the other, is a carbonyl-activating group;

b) for the manufacture of a compound of the formula (II), wherein X is C=S and the dashed line in formula (I) bonding X to N is absent, y is 1 and R is hydrogen or an organic moiety that can be bound to nitrogen, with $CS_2$ or Cl-C(=S)-Cl; or

c) for the manufacture of a compound of the formula (II), wherein X is ($CR_7$), wherein $R_7$ is hydrogen or an organic or inorganic moiety with the proviso that then the dashed line bonding X to N is a bond, so that X is bound to the adjacent N via a double bond, with an activated derivative of a compound of formula (IVa), (IVb) or (IVc) or a derivative of one of these compounds:

$R_7$-COOH **(IVa),**

$R_7$-CN **(IVb),**

or

$R_7$-CHO **(IVc),**

wherein $R_7$ is hydrogen, an organic or inorganic moiety, e.g., $C_1$-$C_7$lower alkyl, amino or amino-lower alkyl;
wherein functional groups which are present in the starting compounds in processes a) to c) and are not intended to take part in the reaction, are present in protected form if necessary, and protecting groups that are present are cleaved, wherein said starting compounds may also exist in the form of salts provided that a salt-forming group is present and a reaction in salt form is possible.

A compound of the formula (II),
wherein

R is hydrogen; and
y is 1 is preferably prepared by hydrogenation of a compound of the formula (V)

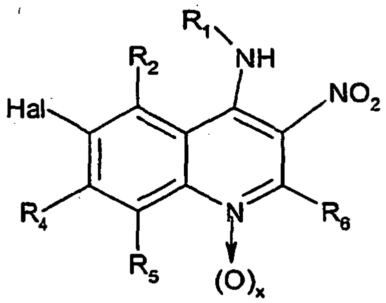

(V)

,

wherein the substituents and symbols are defined as for compounds of the formula (I) (x is preferably zero) in the presence of an appropriate catalyst, e.g., a skeleton based catalyst, such as Raney-Ni with hydrogen in an appropriate solvent, e.g., an alcohol, such as methanol; at preferred temperatures between 0°C and 50°C, e.g., at RT.

The corresponding compounds of the formula (II), wherein R is an organic moiety that can be bound to nitrogen, especially a carbon-bound one, can be prepared by reaction of a compound of formula (11),
wherein

R is hydrogen; and
y is 1 (see preceding paragraph), with a compound of the formula (VI)

R-L **(VI),**

wherein
R is an organic moiety bound to L via a carbon atom; and
L is a leaving group, especially halo, such as chloro, bromo or iodo; or arylsulfonyl, e.g., toluenesulfonyl in an appropriate solvent; preferably in the presence of a tertiary nitrogen base, such as pyridine or triethylamine.

Alternatively, a compound of the formula (II),
wherein

R is hydrogen; and
y is 1 can be reacted with an aldehyde of the formula (VI*) or (VI**)

R*-CHO **(VI*)**

or

R*-CO-R** **(VI**),**

wherein R* and R** are the same or different and each is as an organic moiety bound to the moiety -CHO via a carbon atom; followed by reduction of the resulting enamine with an appropriate reductant, e.g., a complex hydride, such as an alkalimetal cyanoborohydride, e.g., sodium-cyanoborohydride, e.g., in the same solvent and at temperatures between -10°C and 40°C, e.g., at 10°C, the total reaction summing up to reductive amination.

A compound of formula (V) is preferably prepared by reacting a compound of the formula (VII)

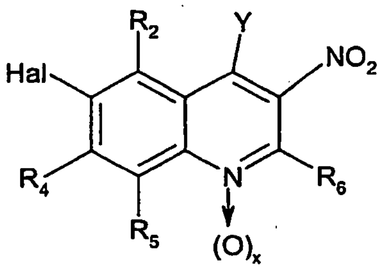

(VII)

,

wherein

Y is halo, especially chloro; and

the other moieties and symbols have the meanings indicated for compounds of the formula (I) (x is preferably zero), with a compound of the formula (VIII)

$R_1$-$NH_2$ **(VIII),**

wherein $R_1$ is as defined for a compound of the formula (1) in an appropriate solvent; preferably a lower alkylcarboxylic acid, such as acetic acid; at preferred temperatures between 10°C and reflux temperature of the reaction mixture, e.g., between 20°C and 140°C.

A compound of the formula (VII) can be prepared by reacting a compound of the formula (IX)

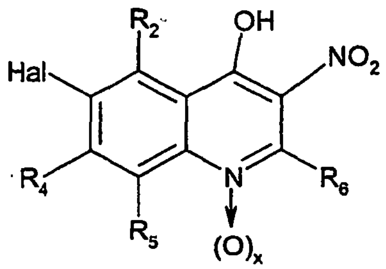

**(IX)**

,

wherein the moieties and symbols have the meanings indicated for a compound of the formula (I) (x is preferably zero), with an inorganic acid halogenide, especially $POCl_3$ (preferably without solvent) at elevated temperatures, e.g., between 100°C and 150°C or under reflux.

A compound of the formula (IX) is known in the art, can be synthesized according to methods known in the art and/or is commercially-available. For example, it can be synthesized by reacting a compound of the formula (X)

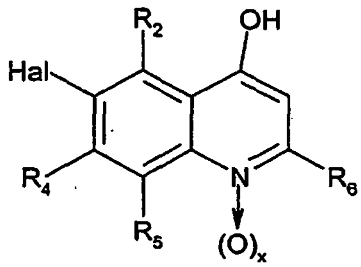

**(X)**

,

wherein the moieties and symbols have the meanings indicated for a compound of the formula (I) (x is preferably zero), with nitric acid (aqueous) at a preferred temperature between 50°C and 100°C, e.g., at 85°C.

A compound of the formula (IX), can alternatively be synthesized by reacting a compound of the formula (XI)

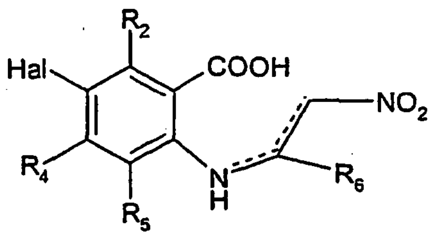

**(XI)**

,

wherein the moieties and symbols have the meanings indicated for a compound of the formula (I), with an anhydride of a carbonic acid, especially acetic anhydride, preferably in the presence of an alkali metal salt of a carboxylic acid, e.g.,

potassium acetate, at a preferred temperature between 50°C and 150°C, e.g., at ca. 100-140°C.

A compound of the formula (XI) can be obtained, e.g., by converting a compound of the formula (XII)

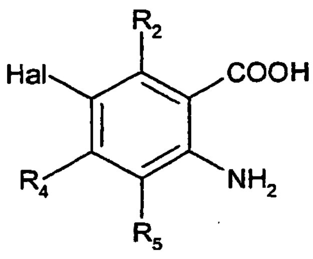

(XII)

,

to the corresponding compound of the formula (XI) by reacting nitromethane in the presence of an alkali metal hydroxide, especially sodium hydroxide, at preferred temperatures between approximately 0°C and 60°C, e.g., between 0°C and RT; then pouring the product under cooling to approximately 0°C into concentrated HCl and adding the compound of the formula (XII) and further concentrated HCl, subsequently allowing for further reaction at preferred temperatures between 0°C and RT to result in the corresponding compound of formula (XI).

Other starting materials are either known in the art, can be prepared according to methods that are known in the art, e.g., in analogy to the methods described hereinabove or in the examples, and/or are commercially-available.

Disclosed herein are also novel starting materials and/or intermediates and to processes for their preparation. The starting materials used and the reaction conditions selected are preferably those that result in the compounds described as being preferred.

Other starting materials are either known in the art, can be prepared according to methods that are known in the art, e.g., in analogy to the methods described hereinabove or in the examples, and/or are commercially-available.

The present invention relates also to novel starting materials and/or intermediates and to processes for their preparation. The starting materials used and the reaction conditions selected are preferably those that result in the compounds described as being preferred.

## Detailed Description of Preferred Reaction Conditions

The reaction described under (a) preferably takes place under conditions known in the art, especially in an appropriate solvent, such as a halo-lower alkane, e.g., dichloromethane; or a lower alkylnitrile, such as acetonitrile and under elevated temperatures, preferably in the range from 40°C to the reflux temperature of the reaction mixture, especially under reflux. In the compound of the formula (III), each A is, independently of the other, preferably halo, trichloromethyl, succinimido or 1-imidazolo. For example, if the compound of the formula (III) is trichloromethyl chloroformate, the reaction preferably takes place under anhydrous conditions in an appropriate aprotic solvent, e.g., a halogenated hydrocarbon, such as dichloromethane, at preferred temperatures between 0°C and 50°C, e.g., at RT.

The reaction described under (b) with $CS_2$ or Cl-C(=S)-Cl preferably takes place in the presence of a base, especially a tertiary amine, such as tri-lower alkylamine, preferably triethylamine or pyridine; an alkalimetal carbonate or -bicarbonate, e.g., sodium bicarbonate; or a metal hydroxide, especially an alkali metal hydroxide, such as sodium or potassium hydroxide, in a polar organic solvent, especially an alcohol, at temperatures between 10°C and the reflux temperature, more preferably between 20°C and 100°C.

The reaction described under (c) preferably takes place in the presence of an active derivative of a compound of the formulae (IVa), (IVb) and (IVc) as solvent or other appropriate solvents or solvent mixtures at preferred temperatures between 30°C and the reflux temperature of the reaction mixture, more preferably under reflux. An activated derivative of a compound of the formula (IVa) is especially a tri-lower alkyl orthoester of the carbonic acid of formula (IVa), especially a tri-ethyl derivative, such as triethylorthoformate or a tetramethyl, derivative, such as tetramethyl orthocarbonate. Alternatively, the respective reactive derivative of an acid of the formula (IVa) is formed *in situ,* e.g., in the presence of polyphosphoric acid (also as solvent) at elevated temperatures, e.g., between 100°C and 140°C. An activated derivative of a compound of formula (IVb) is especially a halo derivative, such as cyanogen bromide.

Compounds of formula (I) can be transformed into different compounds of formula (I).

Especially, the following transformations are of interest:

In compounds of the formula (I), wherein $R_1$ carries a cyano or cyano-lower alkyl substituent, this substituent can be converted into an aminomethyl or aminomethyl-lower alkyl group, respectively, by hydrogenation, e.g., with hydrogen in the presence of an appropriate catalyst, such as a Raney catalyst, especially Raney-Ni; in an appropriate

solvent, such as an alcohol, especially methanol or ethanol; or a cyclic ether, such as tetrahydrofuran, or a mixture thereof, in the presence of ammonia, preferably at temperatures between 0°C and 50°C, e.g., at RT.

In compounds of the formula (I), wherein $R_1$ carries a cyano or cyano-lower alkyl substituent or $A_7$ is any one of these substituents, this substituent can be converted into a *N*-hydroxyamidino or *N*-hydroxyamidino-lower alkyl group, respectively, by reaction with a hydroxylamine salt of an organic or inorganic acid, e.g., a hydroxylamine halogenide; in a polar solvent, e.g., a di-lower alkyl lower alkanoylamide, especially dimethyl formamide; in the presence of water at preferred temperatures between 10°C and 100°C, e.g., at 20-75°C; in the presence of a base, especially an alkali metal carbonate, such as sodium carbonate.

In compounds of the formula (I), wherein $R_1$ is 2-haloaryl, e.g., 2-chlorophenyl; the halogen can be removed by hydrogenation with hydrogen in an appropriate solvent, e.g., in an alcohol, such as methanol; or a *N*,*N*-di-lower alkyl-loweralkanoylamide, such as dimethylformamide or a mixture thereof; and a catalyst, such as a noble metal on a carrier material, e.g., palladium on charcoal (Pd-C), at preferred temperatures between 0°C and 50°C, e.g., at RT, to the corresponding compound wherein $R_1$ is aryl, e.g., phenyl.

In a compound of the formula (I), wherein a hydroxyamidino substituent is present, e.g., as mentioned in the last paragraph; this substituent can be converted into the corresponding amidino substituent by hydrogenation in the presence of an acid, such as HCl; and a catalyst, preferably a Raney metal catalyst, such as Raney-Ni; preferably at elevated temperatures, e.g., between 30°C and 70°C, e.g., at 50°C.

Compounds of the formula (I), wherein x and y or one of them are zero can be converted into the corresponding *N*-oxide compounds (x, y or both = 1, R = $\rightarrow$O) by oxidation in the presence of a peroxide, especially a peroxybenzoic acid derivative, such as 3-chloroperoxybenzoic acid; in the presence of a base, e.g., an alkali metal carbonate, such as sodium carbonate; and in an appropriate solvent, e.g., a halogenated hydrocarbon, such as chloroform or methylene chloride.

Compound of formula (I),
wherein
X is $CR_7$; and
$R_7$ is $NH_2$,
is prepared from the corresponding di-amino compound and cyanogen bromide in an appropriate solvent, e.g. ethanol; at temperatures between 0°C and 50°C, e.g., RT.

A compound of formula (I),
wherein
X is $CR_7$; and
$R_7$ is $OCH_3$,
is prepared from the corresponding di-amino compound and tetramethyl orthocarbonate in the presence of an appropriate solvent, e.g., acetic acid; at elevated temperatures, e.g., 75°C.

A compound of formula (I),
wherein
X is $CR_7$; and
$R_7$ is $CF_3$,
is prepared from the di-amino compound and trifluoroacetic acid in the presence of an appropriate solvent, e.g., 4 N HCl; at elevated temperatures, e.g., 100°C.

A compound of formula (I),
wherein
X is $CR_7$; and
$R_7$ is $CH_3$,
is prepared from the corresponding di-amino compound and triethylorthoacetate at elevated temperatures, e.g., 130°C.

A compound of formula (I),
wherein
X is $CR_7$; and
$R_7$ is lower alkyl,
is prepared from the corresponding di-amino compound and the corresponding aldehyde using catalytic amounts of acetic acid in an appropriate solvent, e.g., DCM; at temperatures between 0°C and 50°C, e.g., RT.

A compound of formula (I), wherein $R_3$ is unsubstituted or substituted aryl or heterocyclyl is prepared by reacting the Br-derivative and the corresponding boronic acid in the presence of bis(triphenylphosphine)palladium (II) dichloride, 1 M solution of sodium carbonate in an appropriate solvent, e.g., DMF at elevated temperatures, e.g., 100°C. This is a Pd catalyzed cross-coupling reaction of aryl, alkynyl or vinyl halides with aryl or vinyl boronic acids. See Suzuki, Tetrahedron Lett, Vol. 20, p. 3437 (1979); or J Am Chem Soc, Vol. 107 p. 972 (1985).

A compound of the formula (I),
wherein

X is ($CR_7$); and
$R_7$ is halogen,
can be obtained from the corresponding compound, wherein $R_7$ is hydrogen by reaction with the corresponding halogen succinimide, especially *N*-bromosuccinimide, in the presence of the corresponding iron(III)halogenide, especially $FeBr_3$; in the absence or presence of an appropriate solvent at elevated temperatures, preferably under reflux.

A compound of the formula (I),
wherein
X is C=O;
y is 1; and
R is aryl, especially phenyl,
can be obtained by converting the corresponding compound of the formula (I), wherein R is H with an arylboronic acid, especially phenylboronic acid; in the presence of anhydrous cupric acetate and a tertiary amine, e.g., a tri-lower alkylamine, such as triethylamine; in an appropriate aprotic solvent, especially a halogenated hydrocarbon, such as dichloromethylene; at preferred temperatures between 0°C and 50°C, e.g., at RT, into said compound.

Salts of compounds of formula (I) having at least one salt-forming group may be prepared in a manner known *per se.* For example, salts of compounds of formula (I) having acid groups may be formed, e.g., by treating the compounds with metal compounds, such as alkali metal salts of suitable organic carboxylic acids, e.g., the sodium salt of 2-ethylhexanoic acid; with organic alkali metal or alkaline earth metal compounds, such as the corresponding hydroxides, carbonates or hydrogen carbonates, such as sodium or potassium hydroxide, carbonate or hydrogen carbonate; with corresponding calcium compounds or with ammonia or a suitable organic amine, stoichiometric amounts or only a small excess of the salt-forming agent preferably being used. Acid addition salts of compounds of formula (I) are obtained in customary manner, e.g., by treating the compounds with an acid or a suitable anion exchange reagent. Internal salts of compounds of formula (I) containing acid and basic salt-forming groups, e.g., a free carboxy group and a free amino group, may be formed, e.g., by the neutralization of salts, such as acid addition salts, to the isoelectric point, e.g., with weak bases, or by treatment with ion exchangers.

Salts can be converted in customary manner into the free compounds; metal and ammonium salts can be converted, e.g., by treatment with suitable acids; and acid addition salts, e.g., by treatment with a suitable basic agent.

Mixtures of isomers obtainable according to the invention can be separated in a manner known *per se* into the individual isomers; diastereoisomers can be separated, e.g., by partitioning between polyphasic solvent mixtures, recrystallization and/or chromatographic separation, e.g., over silica gel or by, e.g., medium pressure liquid chromatography over a reversed phase column; and racemates can be separated, e.g., by the formation of salts with optically pure salt-forming reagents and separation of the mixture of diastereoisomers so obtainable, e.g., by means of fractional crystallization, or by chromatography over optically active column materials.

Intermediates and final products can be worked up and/or purified according to standard methods, e.g., using chromatographic methods, distribution methods, re-crystallization and the like.

## Additional process steps

In the additional process steps, carried out as desired, functional groups of the starting compounds which should not take part in the reaction may be present in unprotected form or may be protected, e.g., by one or more protecting groups. The protecting groups are then wholly or partly removed according to one of the known methods.

Protecting groups, and the manner in which they are introduced and removed are described, e.g., Protective Groups in Organic Chemistry, Plenum Press, London, NY (1973); Methoden derorganischen Chemie, Houben-Weyl, 4th Edition, Vol. 15/1, Georg-Thieme-Verlag, Stuttgart (1974); and Theodora W. Greene, Protective Groups in Organic Synthesis, John Wiley & Sons, NY (1981). A characteristic of protecting groups is that they can be removed readily, i.e., without the, occurrence of undesired secondary reactions, e.g., by solvolysis, reduction, photolysis or alternatively under physiological conditions.

The end products of formula (I) may however also contain substituents that can also be used as protecting groups in starting materials for the preparation of other end products of formula (I). Thus, within the scope of this text, only a readily removable group that is not a constituent of the particular desired end product of formula (I) is designated a "protecting group", unless the context indicates otherwise.

## General process conditions

The following applies in general to all processes mentioned hereinbefore and hereinafter, while reaction conditions specifically mentioned above or below are preferred:

All the above-mentioned process steps can be carried out under reaction conditions that are known *per se,* preferably those mentioned specifically, in the absence or, customarily, in the presence of solvents or diluents, preferably

solvents or diluents that are inert towards the reagents used and dissolve them, in the absence or presence of catalysts, condensation or neutralizing agents, e.g., ion exchangers, such as cation exchangers, e.g., in the $H^+$ form; depending on the nature of the reaction and/or of the reactants at reduced, normal or elevated temperature, e.g., in a temperature range of from about -100°C to about 190°C; preferably from approximately -80°C to approximately 150°C, e.g., at from -80°C to -60°C at RT, at from -20°C to 40°C or at reflux temperature; under atmospheric pressure or in a closed vessel, where appropriate under pressure and/or in an inert atmosphere, e.g., under an argon or nitrogen atmosphere.

At all stages of the reactions, mixtures of isomers that are formed can be separated into the individual isomers, e.g., diastereoisomers or enantiomers; or into any desired mixtures of isomers, e.g., racemates or mixtures of diastereoisomers, e.g., analogously to the methods described under "additional process steps".

The solvents from which those solvents that are suitable for any particular reaction may be selected include those mentioned specifically or, e.g., water; esters, such as lower alkyl-lower alkanoates, e.g., ethyl acetate; ethers, such as aliphatic ethers, e.g., diethyl ether; or cyclic ethers, e.g., tetrahydrofuran or dioxane; liquid aromatic hydrocarbons, such as benzene or toluene; alcohols, such as methanol, ethanol or 1- or 2-propanol; nitriles, such as acetonitrile; halogenated hydrocarbons, such as methylene chloride or chloroform; acid amides, such as dimethylformamide or dimethyl acetamide; bases, such as heterocyclic nitrogen bases, e.g., pyridine or *N*-methylpyrrolidin-2-one; carboxylic acid anhydrides, such as lower alkanoic acid anhydrides, e.g., acetic anhydride; cyclic, linear or branched hydrocarbons, such as cyclohexane, hexane or isopentane; or mixtures of those solvents, e.g., aqueous solutions, unless otherwise indicated in the description of the processes. Such solvent mixtures may also be used in working up, e.g., by chromatography or partitioning.

The compounds, including their salts, may also be obtained in the form of hydrates, or their crystals may, e.g., include the solvent used for crystallization. Different crystalline forms may be present.

The invention relates also to those forms of the process in which a compound obtainable as intermediate at any stage of the process is used as starting material and the remaining process steps are carried out, or in which a starting material is formed under the reaction conditions or is used in the form of a derivative, e.g., in protected form or in the form of a salt, or a compound obtainable by the process according to the invention is produced under the process conditions and processed further *in situ.* In the process of the present invention those starting materials are preferably used which result in new compounds of formula (I) described at the beginning as being especially valuable. Special preference is given to reaction conditions that are analogous to those mentioned in the examples.

## Pharmaceutical Compositions

The invention relates also to pharmaceutical compositions comprising a compound of formula (I), to the compounds for use in the therapeutic (in a broader aspect of the invention also prophylactic) treatment of a protein kinase dependent disease, and describes also the preparation of pharmaceutical preparations, especially for said uses.

The pharmacologically acceptable compounds of the present invention may be used, e.g., for the preparation of pharmaceutical compositions that comprise an effective amount of a compound of the formula (I), or a pharmaceutically acceptable salt thereof, as active ingredient together or in admixture with a significant amount of one or more inorganic or organic, solid or liquid, pharmaceutically acceptable carriers.

The invention relates also to a pharmaceutical composition that is suitable for administration to a warm-blooded animal, especially a human (or to cells or cell lines derived from a warm-blooded animal, especially a human, e.g., lymphocytes), for the treatment or, in a broader aspect of the invention, prevention of (= prophylaxis against) a disease that responds to inhibition of protein kinase activity, comprising an amount of a compound of formula (I) or a pharmaceutically acceptable salt thereof, which is effective for said inhibition, especially the in, together with at least one pharmaceutically acceptable carrier.

The pharmaceutical compositions according to the invention are those for enteral, such as nasal; rectal or oral; or parenteral, such as intramuscular or intravenous, administration to warm-bloodect animals (especially a human), that comprise an effective dose of the pharmacologically active ingredient, alone or together with a significant amount of a pharmaceutically acceptable carrier. The dose of the active ingredient depends on the species of warm-blooded animal, the body weight, the age and the individual condition, individual pharmacokinetic data, the disease to be treated and the mode of administration.

The invention allows for a method of treatment for a disease that responds to inhibition of a protein kinase, which comprises administering an (against the mentioned disease) prophylactically or especially therapeutically effective amount of a compound of formula (I) according to the invention, especially to a warm-blooded animal, e.g., a human, that, on account of one of the mentioned diseases, requires such treatment.

The dose of a compound of the formula (I) or a pharmaceutically acceptable salt thereof to be administered to warm-blooded animals, e.g., humans of approximately 70 kg body weight, is preferably from approximately 3 mg to approximately 10 g, more preferably from approximately 10 mg to approximately 1.5 g, most preferably from about 100 mg to about 1000 mg/person/day, divided preferably into 1-3 single doses which may, e.g., be of the same size. Usually,

children receive half of the adult dose.

The pharmaceutical compositions comprise from approximately 1% to approximately 95%, preferably from approximately 20% to approximately 90%, active ingredient. Pharmaceutical compositions according to the invention may be, e.g., in unit dose form, such as in the form of ampoules, vials, suppositories, dragées, tablets or capsules.

The pharmaceutical compositions of the present invention are prepared in a manner known *per se*, e.g., by means of conventional dissolving, lyophilizing, mixing, granulating or confectioning processes.

Solutions of the active ingredient, and also suspensions, and especially isotonic aqueous solutions or suspensions, are preferably used, it being possible, e.g., in the case of lyophilized compositions that comprise the active ingredient alone or together with a carrier, e.g., mannitol, for such solutions or suspensions to be produced prior to use. The pharmaceutical compositions may be sterilized and/or may comprise excipients, e.g., preservatives, stabilizers, wetting and/or emulsifying agents, solubilizers, salts for regulating the osmotic pressure and/or buffers; and are prepared in a manner known *per se*, e.g., by means of conventional dissolving or lyophilizing processes. The said solutions or suspensions may comprise viscosity-increasing substances, such as sodium carboxymethylcellulose, carboxymethylcellulose, dextran, polyvinylpyrrolidone or gelatin.

Suspensions in oil comprise as the oil component the vegetable, synthetic or semi-synthetic oils customary for injection purposes. There may be mentioned as such especially liquid fatty acid esters that contain as the acid component a long-chained fatty acid having from 8-22 carbon atoms, especially from 12-22 carbon atoms, e.g., lauric acid, tridecylic acid, myristic acid, pentadecylic acid, palmitic acid, margaric acid, stearic acid, arachidic acid, behenic acid or corresponding unsaturated acids, e.g., oleic acid, elaidic acid, erucic acid, brasidic acid or linoleic acid, if desired with the addition of antioxidants, e.g., vitamin E, β-carotene or 3,5-di-*tert*-butyl-4-hydroxytoluene. The alcohol component of those fatty acid esters has a maximum of 6 carbon atoms and is a mono- or poly-hydroxy, e.g., a mono-, di- or tri-hydroxy; alcohol, e.g., methanol, ethanol, propanol, butanol or pentanol; or the isomers thereof, but especially glycol and glycerol. The following examples of fatty acid esters are therefore to be mentioned: ethyl oleate, isopropyl myristate, isopropyl palmitate, "Labrafil M 2375" (polyoxyethylene glycerol trioleate, Gattefossé, Paris), "Miglyol 812" (triglyceride of saturated fatty acids with a chain length of $C_8$-$C_{12}$, Hüls AG, Germany), but especially vegetable oils, such as cottonseed oil, almond oil, olive oil, castor oil, sesame oil, soybean oil and more especially groundnut oil.

The injection compositions are prepared in customary manner under sterile conditions; the same applies also to introducing the compositions into ampoules or vials and sealing the containers.

Pharmaceutical compositions for oral administration can be obtained by combining the active ingredient with solid carriers, if desired granulating a resulting mixture, and processing the mixture, if desired or necessary, after the addition of appropriate excipients, into tablets, dragée cores or capsules. It is also possible for them to be incorporated into plastics carriers that allow the active ingredients to diffuse or be released in measured amounts.

Suitable carriers are especially fillers, such as sugars, e.g., lactose, saccharose, mannitol or sorbitol; cellulose preparations and/or calcium phosphates, e.g., tricalcium phosphate or calcium hydrogen phosphate; and binders, such as starch pastes using, e.g., corn, wheat, rice or potato starch, gelatin, tragacanth, methylcellulose, hydroxypropylmethylcellulose, sodium carboxymethylcellulose and/or polyvinylpyrrolidone; and/or, if desired, disintegrators, such as the above-mentioned starches; and/or carboxymethyl starch, crosslinked polyvinylpyrrolidone, agar, alginic acid or a salt thereof, such as sodium alginate. Excipients are especially flow conditioners and lubricants, e.g., silicic acid, talc, stearic acid or salts thereof, such as magnesium or calcium stearate; and/or polyethylene glycol. Dragée cores are provided with suitable, optionally enteric, coatings, there being used, *inter alia,* concentrated sugar solutions which may comprise gum arabic, talc, polyvinylpyrrolidone, polyethylene glycol and/or titanium dioxide; or coating solutions in suitable organic solvents, or, for the preparation of enteric coatings, solutions of suitable cellulose preparations, such as ethylcellulose phthalate or hydroxypropylmethylcellulose phthalate. Capsules are dry-filled capsules made of gelatin and soft sealed capsules made of gelatin and a plasticizer, such as glycerol or sorbitol. The dry-filled capsules may comprise the active ingredient in the form of granules, e.g., with fillers, such as lactose; binders, such as starches; and/or glidants, such as talc or magnesium stearate; and if desired with stabilizers. In soft capsules the active ingredient is preferably dissolved or suspended in suitable oily excipients, such as fatty oils, paraffin oil or liquid polyethylene glycols, it being possible also for stabilizers and/or antibacterial agents to be added. Dyes or pigments may be added to the tablets or dragée coatings or the capsule casings, e.g., for identification purposes or to indicate different doses of active ingredient.

## Combinations

A compound of the formula (I) may also be used to advantage in combination with other antiproliferative agents. Such antiproliferative agents include, but are not limited to, aromatase inhibitors; antiestrogens; topoisomerase I inhibitors; topoisomerase II inhibitors; microtubule active agents; alkylating agents; histone deacetylase inhibitors; compounds, which induce cell differentiation processes; cyclooxygenase inhibitors; MMP inhibitors; mTOR inhibitors; antineoplastic antimetabolites; platin compounds; compounds targeting/decreasing a protein or lipid kinase activity and further anti-angiogenic compounds; compounds which target, decrease or inhibit the activity of a protein or lipid phosphatase;

gonadorelin agonists; anti-androgens; methionine aminopeptidase inhibitors; bisphosphonates; biological response modifiers; antiproliferative antibodies; heparanase inhibitors; inhibitors of Ras oncogenic isoforms; telomerase inhibitors; proteasome inhibitors; agents used in the treatment of hematologic malignancies; compounds which target, decrease or inhibit the activity of Flt-3; Hsp90 inhibitors; temozolomide (TEMODAL®); and leucovorin.

The term "aromatase inhibitor", as used herein, relates to a compound which inhibits the estrogen production, i.e., the conversion of the substrates androstenedione and testosterone to estrone and estradiol, respectively. The term includes, but is not limited to, steroids, especially atamestane, exemestane and formestane; and, in particular, non-steroids, especially aminoglutethimide, roglethimide, pyridoglutethimide, trilostane, testolactone, ketokonazole, vorozole, fadrozole, anastrozole and letrozole. Exemestane can be administered, e.g., in the form as it is marketed, e.g., under the trademark AROMASIN. Formestane can be administered, e.g., in the form as it is marketed, e.g., under the trademark LENTARON. Fadrozole can be administered, e.g., in the form as it is marketed, e.g., under the trademark AFEMA. Anastrozole can be administered, e.g., in the form as it is marketed, e.g., under the trademark ARIMIDEX. Letrozole can be administered, e.g., in the form as it is marketed, e.g., under the trademark FEMARA or FEMAR. Aminoglutethimide can be administered, e.g., in the form as it is marketed, e.g., under the trademark ORIMETEN. A combination of the invention comprising a chemotherapeutic agent which is an aromatase inhibitor is particularly useful for.the treatment of hormone receptor positive tumors, e.g., breast tumors.

The term "anti-estrogen", as used herein, relates to a compound which antagonizes the effect of estrogens at the estrogen receptor level. The term includes, but is not limited to, tamoxifen, fulvestrant, raloxifene and raloxifene hydrochloride. Tamoxifen can be administered, e.g., in the form as it is marketed, e.g., under the trademark NOLVADEX. Raloxifene hydrochloride can be administered, e.g., in the form as it is marketed, e.g., under the trademark EVISTA. Fulvestrant can be formulated as disclosed in U.S. Patent No. 4,659,516 or it can be administered, e.g., in the form as it is marketed, e.g., under the trademark FASLODEX. A combination of the invention comprising a chemotherapeutic agent which is an antiestrogen is particularly useful for the treatment of estrogen receptor positive tumors, e.g., breast tumors.

The term "anti-androgen", as used herein, relates to any substance which is capable of inhibiting the biological effects of androgenic hormones and includes, but is not limited to, bicalutamide (CASODEX), which can be formulated, e.g., as disclosed in U.S. Patent No. 4,636,505.

The term "gonadorelin agonist", as used herein, includes, but is not limited to, abarelix, goserelin and goserelin acetate. Goserelin is disclosed in U.S. Patent No. 4,100,274 and can be administered, e.g., in the form as it is marketed, e.g., under the trademark ZOLADEX. Abarelix can be formulated, e.g., as disclosed in U.S. Patent No. 5,843,901.

The term "topoisomerase I inhibitor", as used herein, includes, but is not limited to, topotecan, gimatecan, irinotecan, camptothecian and its analogues, 9-nitrocamptothecin and the macromolecular camptothecin conjugate PNU-166148 (compound A1 in WO 99/17804). Irinotecan can be administered, e.g., in the form as it is marketed, e.g., under the trademark CAMPTOSAR. Topotecan can be administered, e.g., in the form as it is marketed, e.g., under the trademark HYCAMTIN.

The term "topoisomerase II inhibitor", as used herein, includes, but is not limited to, the anthracyclines, such as doxorubicin, including liposomal formulation, e.g., CAELYX; daunorubicin; epirubicin; idarubicin; nemorubicin; the anthraquinones mitoxantrone and losoxantrone; and the podophillotoxines etoposide and teniposide. Etoposide can be administered, e.g., in the form as it is marketed, e.g., under the trademark ETOPOPHOS. Teniposide can be administered, e.g., in the form as it is marketed, e.g., under the trademark VM 26-BRISTOL. Doxorubicin can be administered, e.g., in the form as it is marketed, e.g., under the trademark ADRIBLASTIN or ADRIAMYCIN. Epirubicin can be administered, e.g., in the form as it is marketed, e.g., under the trademark FARMORUBICIN. Idarubicin can be administered, e.g., in the form as it is marketed, e.g., under the trademark ZAVEDOS. Mitoxantrone can be administered, e.g., in the form as it is marketed, e.g., under the trademark NOVANTRON.

The term "microtubule active agent" relates to microtubule stabilizing, microtubule destabilizing agents and microtublin polymerization inhibitors including, but not limited to, taxanes, e.g., paclitaxel and docetaxel; vinca alkaloids, e.g., vinblastine, especially vinblastine sulfate; vincristine, especially vincristine sulfate and vinorelbine; discodermolides; cochicine; and epothilones and derivatives thereof, e.g., epothilone B or D or derivatives thereof. Paclitaxel may be administered, e.g., in the form as it is marketed, e.g., TAXOL. Docetaxel can be administered, e.g., in the form as it is marketed, e.g., under the trademark TAXOTERE. Vinblastine sulfate can be administered, e.g., in the form as it is marketed, e.g., under the trademark VINBLASTIN R.P. Vincristine sulfate can be administered, e.g., in the form as it is marketed, e.g., under the trademark FARMISTIN. Discodermolide can be obtained, e.g., as disclosed in U.S. Patent No. 5,010,099. Also included are epothilone derivatives which are disclosed in WO 98/10121, U.S. Patent No. 6,194,181, WO 98/25929, WO 98/08849, WO 99/43653, WO 98/22461 and WO 00/31247. Especially preferred are epothilone A and/or B.

The term "alkylating agent", as used herein, includes, but is not limited to, cyclophosphamide, ifosfamide, melphalan or nitrosourea (BCNU or Gliadel). Cyclophosphamide can be administered, e.g., in the form as it is marketed, e.g., under the trademark CYCLOSTIN. Ifosfamide can be administered, e.g., in the form as it is marketed, e.g., under

the trademark HOLOXAN.

The term "histone deacetylase inhibitors" or "HDAC inhibitors" relates to compounds which inhibit the histone deacetylase and which possess antiproliferative activity. This includes compounds disclosed in WO 02/22577, especially N-hydroxy-3-[4-[[(2-hydroxyethyl)[2-(1*H*-indol-3-yl)ethyl]-amino]methyl]phenyl]-2*E*-2-propenamide, *N*-hydroxy-3-(4-[[[2-(2-rnethyl-1*H*-indol-3-yl)-ethyl]-amino]methyl]phenyl]-2*E*-2-propenamide and pharmaceutically acceptable salts thereof. It further especially includes suberoylanilide hydroxamic acid (SAHA).

The term "antineoplastic antimetabolite" includes, but is not limited to, 5-fluorouracil or 5-FU; capecitabine; gemcitabine; DNA demethylating agents, such as 5-azacytidine and decitabine; methotrexate and edatrexate; and folic acid antagonists, such as pemetrexed. Capecitabine can be administered, e.g., in the form as it is marketed, e.g., under the trademark XELODA. Gemcitabine can be administered, e.g., in the form as it is marketed, e.g., under the trademark GEMZAR. Also included is the monoclonal antibody trastuzumab which can be administered, e.g., in the form as it is marketed, e.g., under the trademark HERCEPTIN.

The term "platin compound", as used herein, includes, but is not limited to, carboplatin, *cis*-platin, cisplatinum and oxaliplatin. Carboplatin can be administered, e.g., in the form as it is marketed, e.g., under the trademark CARBOPLAT. Oxaliplatin can be administered, e.g., in the form as it is marketed, e.g., under the trademark ELOXATIN.

The term "compounds targeting/decreasing a protein or lipid kinase activity; or a protein or lipid phosphatase activity; or further anti-angiogenic compounds", as used herein, includes, but is not limited to, protein tyrosine kinase and/or serine and/or threonine kinase inhibitors or lipid kinase inhibitors, e.g.,

a) compounds targeting, decreasing or inhibiting the activity of the platelet-derived growth factor-receptors (PDGFR), such as compounds which target, decrease or inhibit the activity of PDGFR, especially compounds which inhibit the PDGF receptor, e.g., a *N*-phenyl-2-pyrimidine-amine derivative, e.g., imatinib, SU101, SU6668 and GFB-111;

b) compounds targeting, decreasing or inhibiting the activity of the fibroblast growth factor-receptors (FGFR);

c) compounds targeting, decreasing or inhibiting the activity of the insulin-like growth factor receptor I (IGF-IR), such as compounds which target, decrease or inhibit the activity of IGF-IR, especially compounds which inhibit the IGF-IR receptor, such as those compounds disclosed in WO 02/092599;

d) compounds targeting, decreasing or inhibiting the activity of the Trk receptor tyrosine kinase family;

e) compounds targeting, decreasing or inhibiting the activity of the Axl receptor tyrosine kinase family;

f) compounds targeting, decreasing or inhibiting the activity of the c-Met receptor;

g) compounds targeting, decreasing or inhibiting the activity of the Kit/SCFR receptor tyrosine kinase;

h) compounds targeting, decreasing or inhibiting the activity of the C-kit receptor tyrosine kinases - (part of the PDGFR family), such as compounds which target, decrease or inhibit the activity of the c-Kit receptor tyrosine kinase family, especially compounds which inhibit the c-Kit receptor, e.g., imatinib;

i) compounds targeting, decreasing or inhibiting the activity of members of the c-Abl family and their gene-fusion products, e.g., BCR-Abl kinase, such as compounds which target decrease or inhibit the activity of c-Abl family members and their gene fusion products, e.g., a *N*-phenyl-2-pyrimidine-amine derivative, e.g., imatinib, PD180970, AG957, NSC 680410 or PD173955 from ParkeDavis;

j) compounds targeting, decreasing or inhibiting the activity of members of the protein kinase C (PKC) and Raf family of serine/threonine kinases, members of the MEK, SRC, JAK, FAK, PDK and Ras/MAPK family members, or PI(3) kinase family, or of the PI(3)-kinase-related kinase family, and/or members of the cyclin-dependent kinase family (CDK) and are especially those staurosporine derivatives disclosed in U.S. Patent No. 5,093,330, e.g., midostaurin; examples of further compounds include, e.g., UCN-01; safingol; BAY 43-9006; Bryostatin 1; Perifosine; Ilmofosine; RO 318220 and RO 320432; GO 6976; Isis 3521; LY333531/LY379196; isochinoline compounds, such as those disclosed in WO 00/09495; FTIs; PD184352; or QAN697 (a P13K inhibitor);

k) compounds targeting, decreasing or inhibiting the activity of protein-tyrosine kinase inhibitors, such as compounds which target, decrease or inhibit the activity of protein-tyrosine kinase inhibitors include imatinib mesylate (GLEEVEC) or tyrphostin. A tyrphostin is preferably a low molecular weight (Mr < 1500) compound, or a pharmaceutically acceptable salt thereof, especially a compound selected from the benzylidenemalonitrile class or the S-arylbenzenemalonirile or bisubstrate quinoline class of compounds, more especially any compound selected from the group consisting of Tyrphostin A23/RG-50810, AG 99, Tyrphostin AG 213, Tyrphostin AG 1748, Tyrphostin AG 490, Tyrphostin B44, Tyrphostin 844(+) enantiomer, Tyrphostin AG 555, AG 494, Tyrphostin AG 556, AG957 and adaphostin (4-{[(2,5-dihydroxyphenyl)methyl]amino}-benzoic acid adamantyl ester, NSC 680410, adaphostin; and

l) compounds targeting, decreasing or inhibiting the activity of the epidermal growth factor family of receptor tyrosine kinases (EGFR, ErbB2, ErbB3, ErbB4 as homo- or hetero-dimers), such as compounds which target, decrease or inhibit the activity of the epidermal growth factor receptor family are especially compounds, proteins or antibodies which inhibit members of the EGF receptor tyrosine kinase family, e,g., EGF receptor, ErbB2, ErbB3 and ErbB4 or bind to EGF or EGF related ligands, and are in particular those compounds, proteins or monoclonal antibodies generically and specifically disclosed in WO 97/02266, e.g., the compound of Example 39, or in

EP 0 564 409; WO 99/03854; EP 0520722; EP 0 566 226; EP 0 787 722; EP 0 837 063; U.S. Patent No. 5,747,498; WO 98/10767; WO 97/30034; WO 97/49688; WO 97/38983 and, especially, WO 96/30347, e.g., compound known as CP 358774; WO 96/33980, e.g., compound ZD 1839; and WO 95/03283, e.g., compound ZM105180, e.g., trastuzumab (HERCEPTIN), cetuximab, Iressa, Tarceva, OSI-774, CI-1033, EKB-569, GW-2016, E1.1, E2.4, E2.5, E6.2, E6.4, E2.11, E6.3 or E7.6.3; and 7*H*-pyrrolo-[2,3-*d*]pyrimidine derivatives which are disclosed in WO 03/013541.

Further anti-angiogenic compounds include compounds having another mechanism for their activity, e.g., unrelated to protein or lipid kinase inhibition, e.g., thalidomide (THALOMID) and TNP-470.

Compounds which target, decrease or inhibit the activity of a protein or lipid phosphatase are, e.g., inhibitors of phosphatase 1, phosphatase 2A, PTEN or CDC25, e.g., okadaic acid or a derivative thereof.

Compounds which induce cell differentiation processes are e.g. retinoic acid, α- γ- or δ-tocopherol or α- γ- or δ-tocotrienol.

The term cyclooxygenase inhibitor, as used herein, includes, but is not limited to, e.g., Cox-2 inhibitors, 5-alkyl substituted 2-arylaminophenylacetic acid and derivatives, such as celecoxib (CELEBREX), rofecoxib (VIOXX), etoricoxib, valdecoxib or a 5-alkyl-2-arylaminophenylacetic acid, e.g., 5-methyl-2-(2'-chloro-6'-fluoroanilino)phenyl acetic acid or lumiracoxib.

The term "bisphosphonates", as used herein, includes, but is not limited to, etridonic, clodronic, tiludronic, pamidronic, alendronic, ibandronic, risedronic and zoledronic acid. "Etridonic acid" can be administered, e.g., in the form as it is marketed, e.g., under the trademark DIDRONEL. "Clodronic acid" can be administered, e.g., in the form as it is marketed, e.g., under the trademark BONEFOS. "Tiludronic acid" can be administered, e.g., in the form as it is marketed, e.g., under the trademark SKELID. "Pamidronic acid" can be administered, e.g., in the form as it is marketed, e.g., under the trademark AREDIA™. "Alendronic acid" can be administered, e.g., in the form as it is marketed, e.g., under the trademark FOSAMAX. "Ibandronic acid" can be administered, e.g., in the form as it is marketed, e.g., under the trademark BONDRANAT. "Risedronic acid" can be administered, e.g., in the form as it is marketed, e.g., under the trademark ACTONEL. "Zoledronic acid" can be administered, e.g., in the form as it is marketed, e.g., under the trademark ZOMETA.

The term "mTOR inhibitors" relates to compounds which inhibit the mammalian target of rapamycin (mTOR) and which possess antiproliferative activity, such as sirolimus (Rapamune®), everolimus (Certican™), CCI-779 and ABT578.

The term "heparanase inhibitor", as used herein, refers to compounds which target, decrease or inhibit heparin sulphate degradation. The term includes, but is not limited to, PI-88.

The term "biological response modifier", as used herein, refers to a lymphokine or interferons; e.g., interferon γ.

The term "inhibitor of Ras oncogenic isoforms", e.g., H-Ras, K-Ras or N-Ras, as used herein, refers to compounds which target, decrease or inhibit the oncogenic activity of Ras, e.g., a "farnesyl transferase inhibitor", e.g., L-744832, DK8G557 or R115777 (Zamestra).

The term "telomerase inhibitor", as used herein, refers to compounds which target, decrease or inhibit the activity of telomerase. Compounds which target, decrease or inhibit the activity of telomerase are especially compounds which inhibit the telomerase receptor, e.g., telomestatin.

The term "methionine aminopeptidase inhibitor", as used herein, refers to compounds which target, decrease or inhibit the activity of methionine aminopeptidase. Compounds which target, decrease or inhibit the activity of methionine aminopeptidase are, e.g., bengamide or a derivative thereof.

The term "proteasome inhibitor", as used herein, refers to compounds which target, decrease or inhibit the activity of the proteasome. Compounds which target, decrease or inhibit the activity of the proteasome include, e.g., PS-341 and MLN 341.

The term "matrix metalloproteinase inhibitor" or "MMP inhibitor", as used herein, includes, but is not limited to, collagen peptidomimetic and nonpeptidomimetic inhibitors, tetracycline derivatives, e.g., hydroxamate peptidomimetic inhibitor batimastat and its orally bioavailable analogue marimastat (BB-2516), prinomastat (AG3340), metastat (NSC 683551) BMS-279251, BAY 12-9566, TAA211, MMI270B or AAJ996.

The term "agents used in the treatment of hematologic malignancies", as used herein, includes, but is not limited to, FMS-like tyrosine kinase inhibitors, e.g., compounds targeting, decreasing or inhibiting the activity of FMS-like tyrosine kinase receptors (Flt-3R); interferon, 1-b-*D*-arabinofuransylcytosine (ara-c) and bisulfan; and ALK inhibitors, e.g., compounds which target, decrease or inhibit anaplastic lymphoma kinase.

Compounds which target, decrease or inhibit the activity of FMS-like tyrosine kinase receptors (Flt-3R) are especially compounds, proteins or antibodies which inhibit members of the Flt-3R receptor kinase family, e.g., PKC412, midostaurin, a staurosporine derivative, SU11248 and MLN518.

The term "HSP90 inhibitors", as used herein, includes, but is not limited to, compounds targeting, decreasing or inhibiting the intrinsic ATPase activity of HSP90; degrading, targeting, decreasing or inhibiting the HSP90 client proteins via the ubiquitin proteasome pathway. Compounds targeting, decreasing or inhibiting the intrinsic ATPase activity of HSP90 are especially compounds, proteins or antibodies which inhibit the ATPase activity of HSP90, e.g., 17-allylamino,17-demethoxygeldanamycin (17AAG), a geldanamycin derivative, other geldanamycin related compounds, radicicol and HDAC inhibitors.

The term "antiproliferative antibodies", as used herein, includes, but is not limited to, trastuzumab (Herceptin™), Trastuzumab-DM1, erlotinib (Tarceva™), bevacizumab (Avastin™), rituximab (Rituxan®), PRO64553 (anti-CD40) and 2C4 antibody. By antibodies is meant, e.g., intact monoclonal antibodies, polyclonal antibodies, multispecific antibodies formed from at least two intact antibodies, and antibodies fragments so long as they exhibit the desired biological activity.

For the treatment of acute myeloid leukemia (AML), compounds of formula (I) can be used in combination with standard leukemia therapies, especially in combination with therapies used for the treatment of AML. In particular, compounds of formula (I) can be administered in combination with, e.g., famesyl transferase inhibitors and/or other drugs useful for the treatment of AML, such as Daunorubicin, Adriamycin, Ara-C, VP-16, Teniposide, Mitoxantrone, Idarubicin, Carboplatinum and PKC412.

The structure of the active agents identified by code nos., generic or trade names may be taken from the actual edition of the standard compendium "The Merck Index" or from databases, e.g., Patents International, e.g., IMS World Publications.

The above-mentioned compounds, which can be used in combination with a compound of the formula (I), can be prepared and administered as described in the art, such as in the documents cited above.

A compound of the formula (I) may also be used to advantage in combination with known therapeutic processes, e.g., the administration of hormones or especially radiation.

A compound of formula (I) may in particular be used as a radiosensitizer, especially for the treatment of tumors which exhibit poor sensitivity to radiotherapy. By "combination", there is meant either a fixed combination in one dosage unit form, or a kit of parts for the combined administration where a compound of the formula (I) and a combination partner may be administered independently at the same time or separately within time intervals that especially allow that the combination partners show a cooperative, e.g., synergistic, effect or any combination thereof.

The following examples are merely illustrative and not meant to limit the scope of the present claims in any manner.

## EXAMPLES

The following examples serve to illustrate the invention without limiting the scope thereof:

*Abbreviations*

| | | | |
|---|---|---|---|
| Boc | *tert*-butoxycarbonyl | mL | mililiter(s) |
| DCM | dichloromethane | NMR | nuclear magnetic resonance |
| DMF | *N*,*N*-dimethylformamide | PS | polystyrene |
| DMSO | dimethylsulfoxide | RT | room temperature |
| ES-MS | electrospray mass spectrometry | $t_R$ | HPLC retention time in minutes |
| Grad | gradient | TFA | trifluoroacetic acid |
| HCl | hydrochloric acid | THF | tetrahydrofuran |
| HPLC | high-pressure liquid chromatography | | |

Where no temperatures are given, the reaction takes place at ambient (room) temperature. Ratios of solvents, e.g., in eluents or solvent mixtures, are given in volume by volume ($^{v}/_{v}$). The following agents were obtained from Fluka, Buchs, Switzerland: 4-amino-benzonitrile; 2-amino-5-bromo-benzoic acid; nitromethane; ethyl cyanoacetate; (*R*)-2-*tert*-butoxycarbonylamino-3-(4-nitro-phenyl)-propionic acid; *N*-chlorosuccinimide; 4-nitrophenethyl bromide; triethyl orthoacetate; triethyorthoacetate; 2-amino-4-fluorobenzoic acid; 2-amino-4-chlorobenzoic acid; cyanogen bromide; 3-formylphenylboronic acid; aniline 4-fluoroaniline; and phenylboronic acid.

The following agents were obtained from Aldrich, Buchs, Switzerland:

3,4-methylenedioxyphenylboronic acid; 3,4-difluoro-1-nitrobenzene; 2-fluoro-5-nitrotoluene; thiophene-2-boronic acid; thiophene-3-boronic acid; benzo[*b*]furan-2-boronic acid;
2-fluoroaniline; 2,4-dimethoxyphenylboronic acid; 2,5-dimethoxyphenylboronic acid; 3,4-dimethoxyphenylboronic acid; phenylboronic acid;. 2,3-dimethoxyphenylboronic acid; 2,3,4-trimethoxyphenylboronic acid; 3-methoxyphenylboronic acid; 3-fluorophenylboronic acid; and (4-amino-phenyl)-acetonitrile.

The following agents were obtained from Lancaster, Morecambe, U.K: benzo[*b*]thiophene-2-boronic acid; 4-hydroxyphenylboronic acid; 3,4,5-trimethoxyphenylboronic acid; and 4-hydroxyphenylboronic acid.

5-Indolylboronic acid was obtained from Frontier Scientific, Inc., Lancashire, U.K.; pyridine-4-boronic acid was obtained from Maybridge, Cornwall, U.K.; and pyridine-3-boronic acid was obtained from Acros, Morris Plains, New Jersey, USA.

HPLC linear gradient between A = $H_2O$/TFA 1000:1 and B = acetonitrile/TFA 1000:1.

Grad 1: 2-100% B in 7 minutes and 3 minutes at 100% B; column: Nucleosil $C_{18}$ reverse phase; 250 mm x 4.6 mm; particle size 5 μm, 100 A; flow rate: 2.0 mL/min.; detection at 215 nm.

Grad 2: 20-100% B in 5 minutes and 1.5 minutes at 100% B; column: Nucleosil $C_{16}$ reverse phase; 70 mm x 4 mm; particle size 3 μm, 100 A; flow rate: 1.25 mL/min.; detection at 215 nm.

Grad 3: 2-100% B in 4.5 minutes and 1 minute at 100% B; column: Chromolith Performance; 100 mm x 4.5 mm; flow rate: 2 mL/min.; detection at 215 nm.

Grad 4: 12-70% in 2.5 minutes; column: Chromolith SpeedROD RP18e; 500 mm x 4.6 mm; flow rate: 4 mUmin.; detection at 210 nm.

Grad 5: 20-100% B in 5 minutes and 1 minute at 100% B; column: Nucleosil $C_{18}$ reverse phase; 250 mm x 4.6 mm; particle size 5 μm, 100 A; flow rate: 1.0 mL/min.; detection at 215 nm.

Grad 6: 20-100% B in 14 minutes and 5 minutes at 100%; column: Nucleosil $C_{18}$ reverse phase; 250 mm x 4.6 mm; particle size 5 μm, 100 A; flow rate: 1.0 mL/min., detection at 215 nm.

Grad 7: 20-100% B in 2.5 minutes; column: Chromolith SpeedROD RP18e; 500 mm x 4.6 mm; flow rate: 4 mUmin.; detection at 210 nm.

## Example 1

**[4-(8-Benzo[1,3]dioxol-5-yl-imidazo[4,5-*c*]quinolin-1-yl)-phenyl]-acetonitrile**

76 mg (0.45 mmol) of 3,4-methylenedioxyphenylboronic acid, 10 mg of bis(triphenylphosphine)palladium (II) dichloride and 0.75 mL of a 1 M solution of sodium carbonate are added to a solution of 109 mg (0.30 mmol) of [4-(8-bromo-imidazo[4,5-c]quinolin-1-yl)-phenyl]-acetonitrile (Example **1f)** in 3 mL of DMF. The mixture is stirred for 1 hour at 100°C. After filtration, the solution is purified by medium-pressure liquid chromatography to provide [4-(8-benzo[1,3]dioxol-5-yl-imidazo[4,5-*c*]quinolin-1-yl)-phenyl]-acetonitrile, analytical HPLC: $t_R$= 7.19 minutes (Grad 1); $ES^+$-MS: m/$e_o$ = 405.1.

## Example 1a

**5-Bromo-2-(2-nitro-vinylamino)-benzoic acid**

A suspension of 25 g (16 mmol) of 2-amino-5-bromo-benzoic acid in $H_2O$:HCl (37%) (10:1) is stirred for 8 hours and then filtered (Solution **A).** 8.17 g (255 mmol) of nitromethane are added over 10 minutes to an ice-bath cooled mixture of 35 g of ice and 15.3 g (382 mmol) of NaOH. After stirring for 1 hour at 0°C and 1 hour at RT, the solution is added at 0°C to 28 g of ice and 42 mL of HCl (37%) (Solution **B).** Solutions **A** and **B** are combined and the reaction mixture is stirred for 18 hours at RT. The yellow precipitate is filtered-off and washed with water. 5-Bromo-2-(2-nitro-vinylamino)-benzoic acid is dried *in vacuo* at 40°C, analytical HPLC: $t_R$= 3.93 minutes (Grad 1); $ES^+$-MS: m/$e_o$ = 287.0, 289.0, Br pattern.
$^1$H NMR (DMSO-de): δ 13.7-14.6 (br, s, 1H), 12.94 (d, 1H), 8.07 (d, 1 H), 8.03 (dd, 1 H), 7.83 (dd, 1H), 7.71 (d, 1H), 6.76 (d, 1H).

**Example 1b**

**6-Bromo-3-nitro-quinolin-4-ol**

29 g (101 mmol) of 5-bromo-2-(2-nitro-vinylamino)-benzoic acid (Example **1a)** and 11.9 g (121 mmol) of potassium acetate in 129 mL (152 mmol) of acetic anhydride are stirred for 1.5 hours at 120°C. The precipitate is filtered-off and washed with acetic acid until the filtrate is colorless and then with water. 6-Bromo-3-nitro-quinolin-4-ol is dried *in vacuo,* analytical HPLC: $t_R$ = 3.01 minutes (Grad 1); $ES^+$-MS: m/$e_o$ = 269.0, 271.0.

**Example 1c**

**6-Bromo-4-chloro-3-nitro-quinoline**

7.8 g (29 mmol) of 6-bromo-3-nitro-quinolin-4-ol (Example 1 b) in 58 mL (230 mmol) of PO $Cl_3$ are stirred for 2 hours at 120°C. The mixture is cooled to RT and poured slowly into ice-water. The precipitate is filtered-off, washed with ice-cold water and dissolved in $CH_2Cl_2$. The organic phase is washed with cold brine, and the aqueous phase is discarded. After drying over $MgSO_4$, the organic solvent is evaporated to dryness to provide 6-bromo-4-chloro-3-nitro-quinoline, analytical HPLC: $t_R$= 4.32 minutes (Grad 1).
$^1$H NMR ($CDCl_3$): δ 9.20 (s, 1 H), 8.54 (d, 1 H), 8.04 (d, 1 H), 7.96 (dd, 1H).

**Example 1d**

**[4-(6-Bromo-3-nitro-quinolin-4-ylamino)-phenyl]-acetonitrile**

0.38 g (3.11 mmol) of 4-amino-benzonitrile are added to a stirred solution of 0.8 g (2.78 mmol) of 6-bromo-4-chloro-3-nitro-quinoline (Example 1c) in 20 mL of acetic acid. The solution is stirred for 1 hour at RT, and after this time, 600 mL of water are added. The precipitate is filtered-off, washed with water and dried overnight to provide the [4-(6-bromo-3-nitro-quinolin-4-ylamino)-phenyl]-acetonitrile, analytical HPLC: $t_R$= 8.25 mintues (Grad 1); $ES^+$-MS: m/$e_o$ = 369.2.

**Example 1e**

**[4-(3-Amino-6-bromo-quinoline-4-ylamino)-phenyl]-acetonitrile**

1 g (2.7 mmol) of [4-(6-bromo-3-nitro-quinolin-4-ylamino)-phenyl]-acetonitrile are dissolved in 30 mL of MeOH: THF (1:1) and hydrogenated at RT in the presence of 0.5 g of Ni-Raney. The catalyst is filtered-off and washed with methanol. The solvent is evaporated to dryness to provide [4-(3-amino-6-bromo-quinoline-4-ylamino)-phenyl]-acetonitrile, analytical HPLC: $t_R$= 6.99 minutes (Grad 1); $ES^+$-MS: m/$e_o$ = 339.1, 341.1.

**Example 1f**

**[4-(8-Bromo-imidazo[4,5-*c*]quinolin-1-yl)-phenyl]-acetonitrile**

1.0 g (3.1 mmol) of 4-(3-amino-6-bromo-quinoline-4-ylamino-phenyl)-acetonitrile (Example 1 e) in 60 mL of triethyl orthoformate are heated at reflux for 2 hours. The reaction mixture is cooled at RT. The precipitate is collected by filtration and purified by medium-pressure liquid chromatography to provide [4-(8-bromo-imidazo[4,5-*c*]quinolin-1-yl)-phenyl]-acetonitrile, analytical HPLC: $t_R$ = 6.72 minutes (Grad 1); $ES^+$-MS: m/$e_o$ = 349.1, 351.1.

**Example 2**

**[4-(8-Thiophen-2-yl-imidazo[4,5-*c*]quinolin-1-yl)-phenyl]-acetonltrile**

[4-(8-Thiophen-2-yl-imidazo[4,5-c]quinolin-1-yl)-phenyl]-acetonitrile is synthesized as described in Example 1 using thiophene-2-boronoc acid. [4-(8-Thiophen-2-yl-imidazo[4,5-c]quinolin-1-yl)-phenyl]-acetonitrile, analytical HPLC: $t_R$= 7.36 minutes (Grad 1); $ES^+$-MS: m/$e_o$ = 367.0.

**Example 3**

**[4-(8-Benzofuran-2-yl-imidazo[4,5-*c*]quinolin-1-yl)-phenyl]-acetonitrile**

[4-(8-Benzofuran-2-yl-imidazo[4,5-*c*]quinolin-1-yl)-phenyl]-acetonitrile is synthesized as described in Example **1** using benzo[*b*]furan-2-boronic acid. [4-(8-Benzofuran-2-yl-imidazo[4,5-*c*]quinolin-1-yl)-phenyl]-acetonitrile, analytical HPLC: $t_R$= 8.34 minutes (Grad 1); $ES^+$-MS: m/$e_o$ = 401.1.

**Example 4**

**2-[4-(8-Benzo[1,3]dioxol-5-yl-imidazo[4,5-*c*]quinolin-1-yl)-phenyl]-ethylamine**

30 mg (0.074 mmol) of [4-(8-benzo[1,3]dioxol-5-yl-imidazo[4,5-*c*]quinolin-1-yl)-phenyl]-acetonitrile (Example 1) in 4 mL of 10% $NH_3$ in methanol:THF (1:1) are hydrogenated at 40°C in the presence of 10 mg of Ni-Raney. The catalyst is filtered-off and washed with ethyl acetate. The organic solution is washed with water, dried over $MgSO_4$ and concentrated to dryness to provide 2-[4-(8-benzo[1,3]dioxol-5-yl-imidazo[4,5-*c*]quinolin-1-yl)-phenyl]-ethylamine, analytical HPLC: $t_R$= 6.12 minutes (Grad 3); $ES^+$-MS: m/$e_o$ = 409.1.

**Example 5**

**2-[4-(8-Thiophen-2-yl-imidazo[4,5-*c*]quinolin-1-yl)-phenyl]-ethylamine**

2-[4-(8-Thiophen-2-yl-imidazo[4,5-*c*]quinolin-1-yl)-phenyl]-ethylamine is obtained as described in Example **4** using [4-(8-thiophen-2-yl-imidazo[4,5-*c*]quinolin-1-yl)-phenyl]-acetonitrile (Example 2) as starting material. 2-[4-(8-Thiophen-2-yl-imidazo[4,5-c]quinolin-1-yl)-phenyl]-ethylamine, analytical HPLC: $t_R$ = 6.35 minutes (Grad 1); $ES^+$-MS: m/$e_o$ = 371.3.

**Example 6**

**2-[4-(8-Benzofuran-2-yl-imidazo[4,5-c]quinolin-1-yl)-phenyl]-ethylamine**

2-[4-(8-Benzofuran-2-yl-imidazo[4,5-c]quinolin-1-yl)-phenyl]-ethylamine is obtained as described in Example **4** using [4-(8-benzofuran-2-yl-imidazo[4,5-*c*]quinolin-1-yl)-phenyl]-acetonitrile (Example 3) as starting material. 2-[4-(8-Benzofuran-2-yl-imidazo[4,5-*c*]quinolin-1-yl)-phenyl]-ethylamine, analytical HPLC: $t_R$= 6.95 minutes (Grad 1); $ES^+$-MS: m/$e_o$ = 405.2.

**Example 7**

**[3-Chloro-4-(8-thiophen-2-yl-imidazo[4,5-*c*]quinolin-1-yl)-phenyl]-acetonitrile**

[3-Chloro-4-(8-thiophen-2-yl-imidazo[4,5-*c*]quinolin-1-yl)-phenyl]-acetonitrile is synthesized as described in Example **1** using (4-amino-3-chloro-phenyl)-acetonitrile (Example 7a) and thiophene-2-boronoc acid. 3-Chloro-4-(8-thiophen-2-yl-imidazo[4,5-*c*]quinolin-1-yl)-phenyl]-acetonitrile, analytical HPLC: $t_R$ = 6.74 minutes (Grad 1); $ES^+$-MS: m/$e_o$ = 401.0.

**Example 7a**

**(4-Amino-3-chloro-phenyl)-acetonitrile**

2.86 g (21 mmol) of *N*-chlorosuccinimide are added to a stirred solution of 2.67 g (20 mmol) of 4-aminobenzonitrile in 30 mL of isopropanol. The solution is refluxed for 1 hour and then the solvent is removed *in vacuo.* The crude product is dissolved in ethyl acetate and water. The layers are separated and the organic layer is washed with brine, dried over $MgSO_4$ and concentrated *in vacuo.* The crude residue is purified by chromatography on silica eluting with DCM to afford (4-amino-3-chloro-phenyl)-acetonitrile, analytical HPLC: $t_R$ = 6.54 minutes (Grad 1); $ES^+$-MS: me/$e_o$ = 167.2.

**Example 8**

**{3-Chloro-4-[8-(1*H*-indol-5-yl)-imidazo[4,5-*c*]quinolin-1-yl]-phenyl}-acetonitrile**

{3-Chloro-4-[8-(1*H*-indol-5-yl)-imidazo[4,5-*c*]quinolin-1-yl]-phenyl}-acetonitrile is synthesized as described in Example 1 using (4-amino-3-chloro-phenyl)-acetonitrile (Example 7a) and 5-indolylboronic acid. {3-Chloro-4-[8-(1*H*-indol-5-yl)-imidazo[4,5-*c*]quinolin-1-yl]-phenyl}-acetonitrile, analytical HPLC: $t_R$= 6.65 minutes (Grad 1); $ES^+$-MS: m/$e_o$ = 434.1.

**Example 9**

**[3-Chloro-4-(8-thiophen-3-yl-imidazo[4,5-*c*]quinolin-1-yl)-phenyl]-acetonitrile**

[3-Chloro-4-(8-thiophen-3-yl-imidazo[4,5-*c*]quinolin-1-yl)-phenyl]-acetonitrile is synthesized as described in Example **1** using (4-amino-3-chloro-phenyl)-acetonitrile (Example 7a) and thiophene-3-boronic acid. [3-Chloro-4-(8-thiophen-3-yl-imidazo[4,5-*c*]quinolin-1-yl)-phenyl]-acetonitrile, analytical HPLC: $t_R$= 6.69 minutes (Grad 1); $ES^+$-MS: m/$e_o$ = 401.0.

**Example 10**

**2-[3-Chloro-4-(8-thiophen-2-yl-imidazo[4,5-*c*]quinolin-1-yl)-phenyl]-ethylamine**

2-[3-Chloro-4-(8-thiophen-2-yl-imidazo[4,5-*c*]quinolin-1-yl)-phenyl]-ethylamine is obtained as described in Example **4** using 3-chloro-4-(8-thiophen-2-yl-imidazo[4,5-*c*]quino!in-1-y)-phenyl]-acetonitrile (Example **7**) as starting material. 2-[3-Chloro-4-(8-thiophen-2-yl-imidazo[4,5-*c*]quinolin-1-yl)-phenyl]-ethylamine, analytical HPLC: $t_R$ = 5.46 minutes (Grad 1); $ES^+$-MS: m/$e_o$ = 405.1.

**Example 11**

**2-{3-Chloro-4-[8-(1*H*-indol-5-yl)-imidazo[4,5-*c*]quinolin-1-yl]-phenyl}-ethylamine**

2-{3-Chloro-4-[8-(1*H*-indol-5-yl)-imidazo[4,5-*c*]quinotin-1-yl]-phenyl}-ethylamine is obtained as described in Example **4** using {4-[8-(1*H*-Indol-5-yl)-imidazo[4,5-*c*]quinolin-1-yl]-phenyl}-acetonitrile (Example **8**) as starting material. 2-{3-Chloro-4-[8-(1*H*-indol-5-yl)-imidazo[4,5-clquinolin-1-yl]-phenyl}-ethylamine, analytical HPLC: $t_R$= 5.61 minutes (Grad 1); $ES^+$-MS: m/$e_o$ = 438.3.

**Example 12**

**2-[3-Chloro-4-(8-thiophen-3-yl-imidazo[4,5-*c*]quinolin-1-yl)-phenyl]-ethylamine**

2-[3-Chloro-4-(8-thiophen-3-yl-imidazo[4,5-*c*]quinolin-1-yl)-phenyl]-ethylamine is obtained as described in Example **4** using [3-chloro-4-(8-thiophen-3-yl-imidazo[4,5-*c*]quinolin-1-yl)-phenyl]-acetonitrile (Example 9) as starting material. 2-[3-Chloro-4-(8-thiophen-3-yl-imidazo[4,5-*c*]quinolin-1-yl)-phenyl]-ethylamine, analytical HPLC: $t_R$= 5.50 minutes (Grad 1); $ES^+$-MS: m/$e_o$ = 405.1.

**Example 13**

**[2-Fluoro-4-(8-thiophen-2-yl-imidazo[4,5-*c*]quinolin-1-yl)-phenyl]-acetonitrile**

[2-Fluoro-4-(8-thiophen-2-yl-imidazo[4,5-*c*]quinolin-1-yl)-phenyl]-acetonitrile is obtained as described in Example **1** using (4-amino-2-fluoro-phenyl)-acetonitrile (Example 13a) and thiophene-2-boronic acid. [2-Fluoro-4-(8-thiophen-2-yl-imidazo[4,5-*c*]quinolin-1-yl)-phenyl]-acetonitrile, analytical HPLC: $t_R$ = 6.64 minutes (Grad 1); $ES^+$-MS: m/$e_o$ = 385.1.

**Example 13a**

**(4-Amino-2-fluoro-phenyl)-acetonitrile**

1.55 g (8.6 mmol) of (2-fluoro-4-nitro-phenyl)-acetonitrile (Example **13b**) and 160 mg of Pd 5% on charcoal are

shacked in 45 mL of MeOH under 1.1 bar of $H_2$ for 4 hours. After completion of the reaction, the catalyst is filtered-off and the filtrate is evaporated *in vacuo* to dryness to provide (4-amino-2-fluoro-phenyl)-acetonitrile as a brown solid, analytical HPLC: $t_R$= 1.76 minutes (Grad 3).
$^1$H NMR ($CDCl_3$): δ 7.15 (t, 1 H), 6.40-6.48 (m, 2H), 3.88 (br, s, 2H), 3.64 (s, 2H).

**Example 13b**

**(2-Fluoro-4-nitro-phenyl)-acetonitrile**

1.59 g (10 mmol) of 3,4-difluoro-1-nitrobenzene, 1.9 g (13.8 mmol) of finely-powdered $K_2CO_3$, 16.6 mg (0.1 mmol) of KI and 1.24 g (11 mmol) of ethyl cyanoacetate in 10 mL DMF are stirred for 4 hours at RT, and then 1 hour at 50°C and 1 hour at 100°C. The reaction mixture is quenched with aqueous 1 M citric acid and extracted with EtOAc. The combined organic layers are washed with brine, dried over $MgSO_4$, filtered and evaporated *in vacuo.* The residue is treated with 1 mL HCl (37%) in 10 mL $H_2O$:acetic acid (3:1) for 8 hours at 100°C. After this time, the reaction mixture is quenched with saturated aqueous $NaHCO_3$ and extracted with ether. The combined organic layers are washed with aqueous $NaHCO_3$, brine and dried over $MgSO_4$. The organic phase is evaporated *in vacuo* to dryness to give (2-fluoro-4-nitro-phenyl)-acetonitrile as a pale yellow solid, analytical HPLC: $t_R$= 3.69 minutes (Grad 2); ES$^-$-MS: m/$e_o$ = 178.9.

**Example 14**

**[4-(8-Benzofuran-2-yl-imidazo[4,5-*c*]quinolin-1-yl)-2-fluoro-phenyl]-acetonitrile**

[4-(8-Benzofuran-2-yl-imidazo[4,5-c]quinolin-1-yl)-2 fluoro-phenyl]-acetonitrile is obtained as described in Example **1** using (4-amino-2-fluoro-phenyl)-acetonitrile (Example **13a)** and benzo[*b*]furan-2-boronic acid. [4-(8-Benzofuran-2-yl-imidazo[4,5-c]quinolin-1-yl)-2-fluorophenyl]-acetonitrile, analytical HPLC: $t_R$= 7.43 minutes (Grad 1); ES$^+$-MS: m/$e_o$ = 419.1.

**Example 15**

**{2-Fluoro-4-[8-(1*H*-indol-5-yl)-imidazo[4,5-*c*]quinolin-1-yl]-phenyl}-acetonitrile**

{2-Fluoro-4-[8-(1*H*-indol-5-yl)-imidazo[4,5-*c*]quinolin-1-yl]-phenyl}-acetonitrile is obtained as described in Example **1** using (4-amino-2-fluoro-phenyl)-acetonitrile (Example **13a)** and 5-indolylboronic acid. {2-Fluoro-4-[8-(1*H*-indol-5-yl)-imidazo[4,5-*c*]quinolin-1-yl]-phenyl}-acetonitrile, analytical HPLC: $t_R$= 6.57 minutes (Grad 1); ES$^+$-MS: m/$e_o$ = 418.1.

**Example 16**

**2-[2-Fluoro-4-(8-thiophen-2-yl-imidazo[4,5-*c*]quinolin-1-yl)-phenyl]-ethylamine**

2-[2-Fluoro-4-(8-thiophen-2-yl-imidazo[4,5-c]quinolin-1-yl)-phenyl]-ethylamine is obtained as described in Example **4** using [2-fluoro-4-(8-thiophen-2-yl-imidazo[4,5-c]quinolin-1-yl)-phenyl]-acetonitrile (Example **13**) as starting material. 2-[2-Fluoro-4-(8-thiophen-2-yl-imidazo[4,5-c]quinolin-1-yl)-phenyl]-ethylamine, analytical HPLC: $t_R$= 5.44 minutes (Grad 1); ES$^+$-MS: m/$e_o$ = 389.1.

**Example 17**

**2-[4-(8-Benzofuran-2-yl-imidazo[4,5-c]quinolin-1-yl)-2-fluoro-phenyl]-ethylamine**

2-[4-(8-Benzofuran-2-yl-imidazo[4,5-c]quinolin-1-yl)-2-fluoro-phenyl]-ethylamine is obtained as described in Example **4** using [4-(8-benzofuran-2-yl-imidazo[4,5-c]quinolin-1-yl)-2-fluorophenyl]-acetonitrile (Example **14)** as starting material. 2-[4-(8-Benzofuran-2-yl-imidazo[4,5-c]quinolin-1-yl)-2-fluoro-phenyl]-ethylamine, analytical HPLC: $t_R$= 5.93 minutes (Grad 1); ES$^+$-MS: m/$e_o$ = 423.1.

**Example 18**

**2-{2-Fluoro-4-[8-(1*H*-indol-5-yl)-imidazo[4,5-*c*]quinolin-1-yl]-phenyl}-ethylamine**

2-{2-Fluoro-4-[8-(1*H*-indol-5-yl)-imidazo[4,5-c]quinolin-1-yl]-phenyl}-ethylamine is obtained as described in Example **4** using {2-fluoro-4-[8-(1*H*-indol-5-yl)-imidazo[4,5-*c*]quinolin-1-yl]-phenyl}-acetonitrile (Example **15**) as starting material. 2-{2-Fluoro-4-[8-(1*H*-indol-5-yl)-imidazo[4,5-*c*]quinolin-1-yl]-phenyl}-ethylamine, analytical HPLC: $t_R$= 5.54 minutes (Grad 1); $ES^+$-MS: $m/e_o$ = 422.1.

**Example 19**

**[3-Methyl-4-(8-thiophen-3-yl-imidazo[4,8-*c*]quinolin-1-yl)-phenyl]-acetonitrile**

[3-Methyl-4-(8-thiophen-3-yl-imidazo[4,5-*c*]quinolin-1-yl)-phenyl]-acetonitrile is obtained as described in Example **1** using (4-amino-3-methyl-phenyl)-acetonitrile (Example **19a)** and thiophene-3-boronic acid. [3-Methyl-4-(8-thiophen-3-yl-imidazo[4,5-c]quinolin-1-yl)-phenyl]-acetonitrile, analytical HPLC: $t_R$= 1.81 minutes (Grad 4); $ES^+$-MS: $m/e_o$ = 381.2.

**Example 19a**

**(4-Amino-3-methyl-phenyl)-acetonitrile**

(4-Amino-3-methyl-phenyl)-acetonitrile is obtained as described in Example **13a** from (2-methyl-4-nitro-phenyl)-acetonitrile (Example **19b).** (4-Amino-3-methyl-phenyl)-acetonitrile, analytical HPLC: $t_R$= 1.73 minutes (Grad 3); $ES^+$-MS: $m/e_o$ = 146.9.

**Example 19b**

**(2-Methyl-4-nitro-phenyl)-acetonitrile**

0.83 g (13 mmol) of KOH and 1.47 g (13 mmol) of ethyl cyanoacetate in 4 mL DMSO are stirred for 1 hour and then 1.55 g (10 mmol) of 2-fluoro-5-nitrotoluene are added. The reaction mixture is stirred for 8 hours. After this time, 2.5 mL of water, 2.8 mL of acetic acid and 2.5 mL of HCl 37% are added and the reaction mixture is stirred for 2 hours at 100°C. After this time, water is added and the suspension is extracted with ether. The combined organic layers are washed with brine, dried over $MgSO_4$, filtered and evaporated *in vacuo.* The residue is purified by flash chromatography on silica gel (hexane:ethyl acetate 10:1 to 2:1) to give (2-methyl-4-nitro-phenyl)-acetonitrile as a brown solid, analytical HPLC: $t_R$= 3.92 minutes (Grad 2); $ES^+$-MS: $m/e_o$ 174.9.

**Example 20**

**{4-[8-(1*H*-Indol-5-yl)-imidazo[4,5-*c*]quinolin-1-yl]-3-methyl-phenyl}-acetonitrile**

{4-[8-(1*H*-Indol-5-yl)-imidazo[4,5-*c*]quinolin-1-yl]-3-methyl-phenyl}-acetonitrile is obtained as described in Example **1** using (4-amino-3-methyl-phenyl)-acetonitrile (Example **19a)** and 5-indolylboronic acid. {4-[8-(1*H*-Indol-5-yl)-imidazo[4,5-*c*]quinolin-1-yl]-3-methyl-phenyl}-acetonitrile, analytical HPLC: $t_R$= 1.81 minutes (Grad 4); $ES^+$-MS: $m/e_o$ = 414.3.

**Example 21**

**2-[3-Methyl-4-(8-thiophen-3-yl-imidazo[4,5-*c*]quinolin-1-yl)-phenyl]-ethylamine**

2-[3-Methyl-4-(8-thiophen-3-yl-imidazo[4,5-*c*]quinolin-1-yl)-phenyl]-ethylamine is obtained as described in Example **4** using [3-methyl-4-(8-thiophen-3-yl-imidazo[4,5-*c*]quinolin-1-yl)-phenyl]-acetonitrile (Example **19)** as starting material. 2-[3-Methyl-4-(8-thiophen-3-yl-imidazo[4,5-*c*]quinolin-1-yl)-phenyl]-ethylamine, analytical HPLC: $t_R$= 1.30 minutes (Grad 4); $ES^+$-MS: $m/e_o$ = 385.1 $(M+H)^+$.

**Example 22**

**2-{4-[8-(1*H*-Indol-5-yl)-imidazo[4,5-*c*]quinolin-1-yl]-3-methyl-phenyl}-ethylamine**

2-{4-[8-(1*H*-indol-5-yl)-imidazo[4,5-c]quinolin-1-yl]-3-methyl-phenyl}-ethylamine is obtained as described in Example **4** using {4-[8-(1*H*-Indol-5-yl)-imidazo[4,5-*c*]quinolin-1-yl]-3-methylphenyl}-acetonitrile (Example 20) as starting material. 2-{4-[8-(1*H*-Indol-5-yl)-imidazo[4,5-*c*]quinolin-1-yl]-3-methyl-phenyl}-ethylamine, analytical HPLC: $t_R$ = 1.41

minutes (Grad 4); $ES^+$-MS: m/$e_o$ = 418.3.

**Example 23**

**(*R*)-2-Amino-3-[4-(8-thiophen-2-yl-imidazo[4,5-*c*]quinolin-1-yl)-phenyl]-propionamide**

(*R*)-2-Amino-3-[4-(8-thiophen-2-yl-imidazo[4,5-*c*]quinolin-1-yl)-phenyl]-propionamide is obtained as described in Example **1** using (*R*)-[2-(4-amino-phenyl)-1-carbamoyl-ethyl]-carbamic acid *tert*-butyl ester (Example 23a) and thiophene-2-boronic acid, followed by a subsequent treatment with TFA to remove the Boc-protecting group. (*R*)-2-Amino-3-[4-(8-thiophen-2-yl-imidazo[4,5-c]quinolin-1-yl)-phenyl]-propionamide, analytical HPLC: $t_R$ = 2.30 minutes (Grad 3); $ES^+$-MS: m/$e_o$ =414.0.

**Example 23a**

**(*R*)-[2-(4-Amino-phenyl)-1-carbamoyl-ethyl]-carbamic acid *tert*-butyl ester**

(*R*)-[2-(4-Amino-phenyl)-1-carbamoyl-ethyl]-carbamic acid *tert*-butyl ester is obtained by reduction of (*R*)-[1-carbamoyl-2-(4-nitro-phenyl)-ethyl]-carbamic acid *tert*-butyl ester (Example **23b**) as described in Example **13a.** (*R*)-[2-(4-Amino-phenyl)-1-carbamoyl-ethyl]-carbamic acid *tert*-butyl ester, analytical HPLC: $t_R$ = 2.08 minutes (Grad 3); $ES^+$-MS: m/$e_o$ = 280.1.

**Example 23b**

**(*R*)-[1-Carbamoyl-2-(4-nitro-phenyl)-ethyl]-carbamic acid *tert*-butyl ester**

To a solution of 1.0 g (3.2 mmol) of (*R*)-2-*tert* butoxycarbonylamino-3-(4-nitro-phenyl)-propionic acid in 8 mL of dimethylacetamide is added 905 mg (7 mmol) of diisopropylethylamine and 998 mg (3.36 mmol) of *O*-(1,2-dihydro-2-oxo-1-pyridyl)-*N*,*N*,*N'*,*N'*-tetramethyluronium tetrafluoroborate. Ater 5 minutes, $NH_3$ gas is bubbled for 3 minutes in the reaction mixture. The reaction mixture is stirred for 3 minutes, quenched with aqueous 1 M citric acid and extracted with EtOAc. The combined organic layers are washed with water and with brine, dried over $MgSO_4$, filtered and evaporated *in vacuo.* The residue is purified by flash chromatography on silica gel [hexane-EtOAc (2:1)] to give (*R*)-[1-carbamoyl-2-(4-nitro-phenyl)-ethyl]-carbamic acid *tert*-butyl ester as a white solid, analytical HPLC: $t_R$= 3.59 minutes (Grad 2); $ES^+$-MS: m/$e_o$ = 310.0.

**Example 24**

**(*R*)-2-Amino-3-[4-(8-benzo[*b*]thiophen-2-yl-imidazo[4,5-*c*]quinolin-1-yl)-phenyl]-propionamide**

(*R*)-2-Amino-3-[4-(8-benzo[*b*]thiophen-2-yl-imidazo[4,5-c]quinolin-1-yl)-phenyl]-propionamide is obtained as described in Example **1** using (*R*)-[2-(4-amino-phenyl)-1-carbamoyl-ethyl]-carbamic acid *tert*-butyl ester (Example 23a) and benzo[*b*]thiophene-2-boronic acid, followed by a subsequent treatment with TFA to remove the Boc-protecting group. 2-Amino-3-[4-(8-benzo[*b*]thiophen-2-yl-imidazo[4,5-*c*]quinolin-1-yl)-phenyl]-propionamide, analytical HPLC: $t_R$ = 2.83 minutes (Grad 2); $ES^+$-MS: m/$e_o$ = 464.0.

**Example 25**

**[3,5-Dichloro-4-(8-thiophen-2-yl-imidazo[4,5-*c*]quinolin-1-yl)-phenyl]-acetonitrile**

[3,5-Dichloro-4-(8-thiophen-2-yl-imidazo[4,5-*c*]quinolin-1-yl)phenyl]-acetonitrile is prepared as described in Example **1** using (4-amino-3,5-dichloro-phenyl)-acetonitrile (Example 25a) and thiophene-2-boronic acid. [3,5-Dichloro-4-(8-thiophen-2-yl-imidazo[4,5-c]quinolin-1-yl)-phenyl]-acetonitrile, analytical HPLC: $t_R$= 3.94 minutes (Grad 2); $ES^+$-MS: m/$e_o$ = 434.8, 436.7, 438.8.

**Example 25a**

**(4-Amino-3,5-dichloro-phenyl)-acetonitrile**

2 g (15.1 mmol) of (4-amino-phenyl)-acetonitrile and *N*-chlorosuccinimide in 30 mL of methanol are stirred for

2 hours at 0°C and 15 hours at RT. After this time, the reaction mixture is concentrated *in vacuo*, quenched with saturated aqueous $NaHCO_3$ and extracted with EtOAc. The organic layer is washed with brine, dried over $MgSO_4$, filtered and evaporated *in vacuo.* The residue is purified by flash chromatography on silica gel [hexane:EtOAc (4:1) to (3:1)] to give (4-amino-3,5-dichloro-phenyl)-acetonitrile as an off-white solid, 2 g (15.1 mmol) of (4-amino-phenyl)-acetonitrile, analytical HPLC: $t_R$= 4.08 minutes (Grad 2).
$^1$H NMR (DMSO-d6): δ 7.23 (s, 2H), 5.59 (br, s, 2H), 3.85 (s, 2H).

**Example 26**

**[3,5-Dichloro-4-(8-thiophen-3-yl-imidazo[4,5-*c*]quinolin-1-yl)-phenyl]-acetonitrile**

[3,5-Dichloro-4-(8-thiophen-3-yl-imidazo[4,5-*c*]quinolin-1-yl)-phenyl]-acetonitrile is obtained as described in Example **1** using (4-amino-3,5-dichloro-phenyl)-acetonitrile (Example 25a) and thiophene-3-boronic acid. [3,5-Dichloro-4-(8-thiophen-3-yl-imidazo[4,5-*c*]quinolin-1-yl)-phenyl]-acetonitrile, analytical HPLC: $t_R$ = 3.86 minutes (Grad 2); $ES^+$-MS: m/$e_o$ = 434.8, 436.8, 438.8.

**Example 27**

**2-[3,5-Dichloro-4-(8-thlophen-2-yl-imidazo[4,5-*c*]quinolin-1-yl)-phenyl]-ethylamine**

2-[3,5-Dichloro-4-(8-thiophen-2-yl-imidazo[4,5-*c*]quinolin-1-yl)-phenyl]-ethylamine is obtained as described in Example **4** using [3,5-dichloro-4-(8-thiophen-2-yl-imidazo[4,5-*c*]quinolin-1-yl)-phenyl]-acetonitrile (Example **25**) as starting material. 2-[3,5-Dichloro-4-(8-thiophen-2-yl-imidazo[4,5-*c*]quinolin-1-yl)-phenyl]-ethylamine, analytical HPLC: $t_R$= 2.80 minutes (Grad 2); $ES^+$-MS: m/$e_o$ = 438.8, 440.8, 442.8.

**Example 28**

**2-[3,5-Dichloro-4-(8-thiophen-3-yl-imidazo[4,5-*c*]quinolin-1-yl)-phenyl]-ethylamine**

2-[3,5-Dichloro-4-(8-thiophen-3-yl-imidazo[4,5-*c*]quinolin-1-yl)-phenyl]-ethylamine is obtained as described in Example **4** using [3,5-dichloro-4-(8-thiophen-3-yl-imidazo[4,5-*c*]quinolin-1-yl)-phenyl]-acetonitrile (Example **26)** as starting material. 2-[3,5-Dichloro-4-(8-thiophen-3-yl-imidazo[4,5-*c*]quinolin-1-yl)-phenyl]-ethylamine, analytical HPLC: $t_R$ = 2.74 minutes (Grad 2); $ES^+$-MS: m/$e_o$ = 438.8, 440.8, 442.8.

**Example 29**

**{4-[8-(4-Hydroxy-phenyl)-imidazo[4,5-*c*]quinolin-1-yl]-3-trifluoromethyl-phenyl}-acetonitrile**

{4-[8-(4-Hydroxy-phenyl)-imidazo[4,5-*c*]quinolin-1-yl]-3-trifluoromethyl-phenyl}-acetonitrile is obtained as described in Example **1** using (4-amino-3-trifluoromethyl-phenyl)-acetonitrile (prepared after a procedure in Eur J Med Chem, Vol. 31, p. 133 (1996) and 4-hydroxyphenylboronic acid. {4-[8-(4-Hydroxy-phenyl)-imidazo[4,5-*c*]quinolin-1-yl]-3-trifluoromethyl-phenyl}-acetonitrile, analytical HPLC: $t_R$= 2.48 minutes (Grad 5); $ES^+$-MS: m/$e_o$ = 445.0.

**Example 30**

**4-{1-[4-(2-Amino-ethyl)-2-trifluoromethyl-phenyl]-1*H*-imidazo[4,5-*c*]quinolin-8-yl}-phenol**

4-{1-[4-(2-Amino-ethyl)-2-trifluoroinethyl-phenyl]-1*H*-imidazo[4,5-*c*]quinolin-8-yl}-phenol is obtained as described in Example **4** using {4-[8-(4-hydroxy-phenyl)-imidazo[4,5-*c*]quinofin-1-yl]-3-trifluoromethyl-phenyl}-acetonitrile (Example **29)** as starting material. 4-{1-[4-(2-Aminoethyl)-2-trifluoromethyl-phenyl]-1*H*-imidazo[4,5-*c*]quinolin-8-yl}-phenol, analytical HPLC: $t_R$= 6.69 minutes (Grad 6); $ES^+$-MS: m/$e_o$ = 449.1.

**Example 31**

**3-[4-(8-Benzo[1,3]dioxol-5-yl-imidazo[4,5-*c*]quinolin-1-yl)-phenyl]-propionitrile**

3-[4-(8-Benzo[1,3]dioxol-5-yl-imidazo[4,5-*c*]quinolin-1-yl)-phenyl]-propionitrile is obtained as described in Example **1** using 3-(4-amino-phenyl)-propionitrile (Example 31 a). 3-[4-(8-Benzo[1,3]dioxol-5-yl-imidazo[4,5-*c*]quinolin-1-yl)-phenyl]-propionitrile, analytical HPLC: $t_R$= 7.70 minutes (Grad 1); $ES^+$-MS: m/$e_o$ = 419.2 $(M+H)^+$.

**Example 31a**

**3-(4-Amino-phenyl)-propionitrile**

0.78 g (4.4 mmol) of 3-(4-nitro-phenyl)-propionitrile (Example **31b**) are dissolved in 40 mL of MeOH:THF (1:1) and hydrogenated at RT in the presence of 50 mg of Pd-C 10%. After completion of the reaction, the catalyst is filtered-off and washed with methanol. The organic solvent is evaporated to dryness to provide 3-(4-amino-phenyl)-propionitrile, analytical HPLC: $t_R$= 4.81 minutes (Grad 1); $ES^+$-MS: m/$e_o$ = 147.3.

**Example 31b**

**3-(4-Nitro-phenyl)-propionitrile**

3.45 of (15 mmol) of 4-nitrophenethyl bromide are dissolved in 50 mL of ethanol and 0.81 g (16.5 mmol) of sodium cyanide are added. The solution is stirred for 4 hours at RT and then evaporated to dryness. The crude compound is dissolved in 100 mL of ethyl acetate, and the organic solution is extracted with water, brine, dried over $MgSO_4$ and evaporated to dryness. The crude compound is purified by medium-pressure liquid chromatography to provide 3-(4-nitro-phenyl)-propionitrile, analytical HPLC: $t_R$= 7.27 minutes (Grad 1); $ES^-$-MS: m/$e_o$ = 175.3.

**Example 32**

**3-[4-(8-Thiophen-2-yl-imidazo[4,5-*c*]quinolin-1-yl)-phenyl]-propionitrile**

3-[4-(8-Thiophen-2-yl-imidazo[4,5-*c*]quinolin-1-yl)-phenyl]-propionitrile is obtained as described in Example **1** using 3-(4-amino-phenyl)-propionitrile and thiophene-2-boronic acid. 3-[4-(8-Thiophen-2-yl-imidazo[4,5-*c*]quinolin-1-yl)-phenyl]-propionitrile, analytical HPLC: $t_R$= 7.70 minutes (Grad 1); $ES^+$-MS: m/$e_o$ = 381.1.

**Example 33**

**3-[4-(8-Benzo[*b*]thiophen-2-yl-imidazo[4,5-*c*]quinolin-1-yl)-phenyl]-proplonitrile**

3-[4-(8-Benzo[*b*]thiophen-2-yl-imidazo[4,5-*c*]quinolin-1-yl)-phenyl]-propionitrile is obtained as described in Example **1** using 3-(4-amino-phenyl)-propionitrile (Example **31a**) and benzo[*b*]thiophene-2-boronic acid. 3-[4-(8-Benzo[*b*]thiophen-2-yl-imidazo[4,5-*c*]quinolin-1-yl)-phenyl]-propionitrile, analytical HPLC: $t_R$= 8.23 minutes (Grad 1); $ES^+$-MS: m/$e_o$ = 431.1.

**Example 34**

**3-[4-(8-Thiophen-3-yl-imidazo[4,5-*c*]quinolin-1-yl)-phenyl]-propionitrile**

3-[4-(8-Thiophen-3-yl-imidazo[4,5-*c*]quinolin-1-yl)-phenyl]-propionitrile is obtained as described in Example **1** using 3-(4-amino-phenyl)-propionitrile and thiophene-3-boronic acid. 3-[4-(8-Thiophen-3-yl-imidazo[4,5-*c*]quinolin-1-yl)-phenyl]-propionitrile, analytical HPLC: $t_R$= 7.63 minutes (Grad 1); $ES^+$-MS: m/$e_o$ = 381.3 $(M+H)^+$.

**Example 35**

**3-[4-(8-Benzofuran-2-yl-imidazo[4,5-*c*]quinolin-1-yl)-phenyl]-propionitrile**

3-[4-(8-Benzofuran-2-yl-imidazo[4,5-*c*]quinolin-1-yl)-phenyl]-propionitrile is obtained as described in Example **1** using 3-(4-amino-phenyl)-propionitrile (Example 31 a) and benzo[*b*]furan-2-boronic acid. 3-[4-(8-Benzofuran-2-yl-imidazo[4,5-*c*]quinolin-1-yl)-phenyl]-propionitrile, analytical HPLC: $t_R$ = 8.43 minutes (Grad 1); $ES^+$-MS: m/$e_o$ = 415.5.

## Example 36

### 3-[4-(8-Benzo[1,3]dioxol-5-yl-imidazo[4,5-*c*]quinolin-1-yl)-phenyl]-propylamine

3-[4-(8-Benzo[1,3]dioxol-5-yl-imidazo[4,5-*c*]quinolin-1-yl)-phenyl]-propylamine is obtained as described in Example **4** using 3-[4-(8-benzo[1,3]dioxol-5-yl-imidazo[4,5-*c*]quinolin-1-yl)-phenyl]-propionitrile (Example **31**) as starting material. 3-[4-(8-Benzo[1,3]dioxol-5-yl-imidazo[4,5-*c*]quinolin-1-yl)-phenyl]-propylamine, analytical HPLC: $t_R$= 7.02 minutes (Grad 1); $ES^+$-MS: $m/e_o$ = 423.1.

## Example 37

### 3-[4-(8-Thiophen-2-yl-imidazo[4,5-*c*]quinolin-1-yl)-phenyl]-propylamine

3-[4-(8-Thiophen-2-yl-imidazo[4,5-*c*]quinolin-1-yl)-phenyl]-propylamine is obtained as described in Example **4** using 3-[4-(8-thiophen-2-yl-imidazo[4,5-*c*]quinolin-1-yl)-phenyl]-propionitrile (Example **32**) as starting material. 3-[4-(8-Thiophen-2-yl-imidazo[4,5-c]quinolin-1-yl)-phenyl]-propylamine, analytical HPLC: $t_R$= 6.99 minutes (Grad 1); $ES^+$-MS: $m/e_o$= 385.2.

## Example 38

### 3-[4-(8-Benzo[*b*]thiophen-2-yl-imidazo[4,5-*c*]quinolin-1-yl)-phenyl]-propylamine

3-[4-(8-Benzo[*b*]thiophen-2-yl-imidazo[4,5-*c*]quinolin-1-yl)-phenyl]-propylamine is obtained as described in Example **4** using 3-[4-(8-benzo[*b*]thiophen-2-yl-imidazo[4,5-*c*]quinolin-1-yl)-phenyl]-propionitrile (Example **33**) as starting material. 3-[4-(8-Benzo[*b*]thiophen-2-yl-imidazo[4,5-*c*]quinolin-1-yl)-phenyl]-propylamine: analytical HPLC: $t_R$= 7.20 minutes (Grad 1); $ES^+$-MS: $m/e_o$ = 435.1.

## Example 39

### 3-[4-(8-Thiophen-3-yl-imidazo[4,5-*c*]quinolin-1-yl)-phenyl]-propylamine

3-[4-(8-Thiophen-3-yl-imidazo[4,5-*c*]quinolin-1-yl)-phenyl]-propylamine is obtained as described in Example **4** using 3-[4-(8-thiophen-3-yl-imidazo[4,5-c]quinolin-1-yl)-phenyl]-propionitrile (Example **34**) as starting material. 3-[4-(8-Thiophen-3-yl-imidazo[4,5-c]quinolin-1-yl)-phenyl]-propylamine: analytical HPLC: $t_R$ = 5.71 minutes (Grad 1); $ES^+$-MS: $m/e_o$ = 385.2.

## Example 40

### 3-[4-(8-Benzofuran-2-yl-imidazo[4,5-*c*]quinolin-1-yl)-phenyl]-propylamine

3-[4-(8-Benzofuran-2-yl-imidazo[4,5-*c*]quinolin-1-yl)-phenyl]-propylamine is obtained as described in Example **4** using 3-[4-(8-benzofuran-2-yl-imidazo[4,5-*c*]quinolin-1-yl)-phenyl]-propionitrile (Example 35) as starting material. 3-[4-(8-Benzofuran-2-yl-imidazo[4,5-*c*]quinolin-1-yl)-phenyl]-propylamine, analytical HPLC: $t_R$= 6.07 minutes (Grad 1); $ES^+$-MS: $m/e_o$ = 419.2.

## Example 41

### 3-[4-(8-Benzo[1,3]dioxol-5-yl-imidazo[4,5-*c*]quinolin-1-yl)-3-chloro-phenyl]-propionitrile

3-[4-(8-Benzo[1,3]dioxol-5-yl-imidazo[4,5-c]quinolin-1-yl)-3-chloro-phenyl]-propionitrile is obtained as described in Example **1** using 3-(4-amino-3-chloro-phenyl)-propionitrile. 3-[4-(8-Benzo[1,3]dioxol-5-yl-imidazo[4,5-*c*]quinolin-1-yl)-3-chloro-phenyl]-propionitrile, analytical HPLC: $t_R$= 6.90 minutes (Grad 1); $ES^+$-MS: $m/e_o$ = 453.3.

## Example 41 a

### 3-(4-Amino-3-chloro-phenyl)-propionitrile

0.62 mg of 3-(4-amino-phenyl)-propionitrile (Example **31a**) are dissolved in 7 mL of isopropanol and 0.6 g (4.48

mmol) of *N*-chlorosuccinimide are added. The solution is refluxed for 30 minutes and then the solvent is evaporated to dryness. The crude compound is purified by medium-pressure liquid chromatography to provide 3-(4-amino-3-chlorophenyl)-propionitrile, analytical HPLC: $t_R$ = 6.42 minutes (Grad 1); $ES^+$-MS: m/$e_o$= 180.9.

**Example 42**

**3-[3-Chloro-4-(8-thiophen-2-yl-imidazo[4,5-*c*]quinolin-1-yl)-phenyl]-propionitrile**

3-[3-Chloro-4-(8-thiophen-2-yl-imidazo[4,5-*c*]quinolin-1-yl)-phenyl]-propionitrile is obtained as described in Example **1** using 3-(4-amino-3-chloro-phenyl)-propionitrile and thiophene-2-boronic acid. 3-[3-Chloro-4-(8-thiophen-2-yl-imidazo[4,5-*c*]quinolin-1-yl)-phenyl]-propionitrile, analytical HPLC: $t_R$ = 6.85 minutes (Grad 1); $ES^+$-MS: m/$e_o$ = 415.0.

**Example 43**

**3-[3-Chloro-4-(8-thiophen-3-yl-imidazo[4,5-*c*]quinolin-1-yl)-phenyl]-propionitrite**

3-[3-Chloro-4-(8-thiophen-3-yl-imidazo[4,5-*c*]quinolin-1-yl)-phenyl]-propionitrile is obtained as described in Example **1** using 3-(4-amino-3-chloro-phenyl)-propionitrile and thiophene-3-boronic acid. 3-[3-Chloro-4-(8-thiophen-3-yl-imidazo[4,5-*c*]quinolin-1-yl)-phenyl]-propionitrile, analytical HPLC: $t_R$= 6.76 minutes (Grad 1); $ES^+$-MS: m/$e_o$ = 415.0.

**Example 44**

**3-{3-Chloro-4-[8-(1*H*-indol-5-yl)-imidazo[4,5-*c*]quinolin-1-yl]-phenyl}-propionitrile**

3-{3-Chloro-4-[8-(1*H*-indol-5-yl)-imidazo[4,5-*c*]quinolin-1-yl]-phenyl}-propionitrile is obtained as described in Example **1** using 3-(4-amino-3-chloro-phenyl) propionitrile and 5-indolylboronic acid. 3-{3-Chloro-4-[8-(1*H*-indol-5-yl)-imidazo[4,5-*c*]quinolin-1-yl]-phenyl}-propionitrile, analytical HPLC: $t_R$ = 6.73 minutes (Grad 1); $ES^+$-MS: m/$e_o$ = 448.1.

**Example 45**

**3-[4-(8-Benzo[1,3]dioxol-5-yl-imidazo[4,5-*c*]quinolin-1-yl)-3-chloro-phenyl]-propylamine**

3-[4-(8-Benzo[1,3]dioxol-5-yl-imidazo[4,5-*c*]quinolin-1-yl)-3-chloro-phenyl]-propylamine is synthesized as described in Example **4** using 3-[4-(8-benzo[1,3]dioxol-5-yl-imidazo[4,5-*c*]quinolin-1-yl)-3-chloro-phenyl]-propionitrile (Example **41**) as starting material. 3-[4-(8-Benzo[1,3]dioxol-5-yl-imidazo[4,5-*c*]quinolin-1-yl)-3-chloro-phenyl]-propylamine, analytical HPLC: $t_R$ = 5.71 minutes (Grad 1); $ES^+$-MS: m/$e_o$ = 457.3.

**Example 46**

**3-[3-Chloro-4-(8-thiophen-2-yl-imidazo[4,5-*c*]quinolin-1-yl)-phenyl]-propylamine**

3-[3-Chloro-4-(8-thiophen-2-yl-imidazo[4,5-*c*]quinolin-1-yl)-phenyl]-propylamine is synthesized as described in Example **4** using 3-[3-chloro-4-(8-thiophen-2-yl-imidazo[4,5-*c*]quinolin-1-yl)-phenyl]-propionitrile (Example **42**) as starting material. 3-[3-Chloro-4-(8-thiophen-2-yl-imidazo[4,5-*c*]quinolin-1-yl)-phenyl]-propylamine, analytical HPLC: $t_R$= 5.63 minutes (Grad 1); $ES^+$-MS: m/$e_o$ = 419.3.

**Example 47**

**3-[3-Chloro-4-(8-thiophen-3-yl-imidazo[4,5-*c*]quinolin-1-yl)-phenyl]-propylamine**

3-[3-Chloro-4-(8-thiophen-3-yl-imidazo[4,5-*c*]quinolin-1-yl)-phenyl]-propylamine is synthesized as described in Example **4** using 3-[3-chloro-4-(8-thiophen-3-yl-imidazo[4,5-*c*]quinolin-1-yl)-phenyl]-propionitrile (Example **43**) as starting material. 3-[3-Chloro-4-(8-thiophen-3-yl-imidazo[4,5-*c*]quinolin-1-yl)-phenyl]-propylamine, analytical HPLC: $t_R$ = 5.58 minutes (Grad 1); $ES^+$-MS: m/$e_o$ = 419.1.

**Example 48**

**3-{3-Chloro-4-[8-(1*H*-indol-5-yl)-imidazo[4,5-*c*]quinolin-1-yl]-phenyl}-propylamine**

3-{3-Chloro-4-[8-(1*H*-indol-5-yl)-imidazo[4,5-c]quinolin-1-yl]-phenyl}-propylamine is obtained as described in Example **4** using 3-{3-chloro-4-[8-(1*H*-indo)-5-yl)-imidazo[4,5-*c*]quinolin-1-yl]-phenyl}-propionitrile (Example **44**) as starting material. 3-{3-Chloro-4-[8-(1*H*-indol-5-yl)-imidazo[4,5-*c*]quinolin-1-yl]-phenyl}-propylamine, analytical HPLC: $t_R$= 5.69 minutes (Grad 1); $ES^+$-MS: $m/e_o$ = 452.1.

**Example 49**

**8-Benzo[1,3]dioxol-5-yl-1-{4-[2-(4,5-dihydro-1*H*-imidazol-2-yl)-ethyl]-phenyl}-1 *H*-imidazo[4,5-*c*]quinoline**

8-Benzo[1,3]dioxol-5-yl-1-{4-[2-(4,5-dihydro-1*H*-imidazol-2-yl)-ethyl]-phenyl}-1*H*-imidazo[4,5-*c*]quinoline is obtained as described in Example **1** using 4-[2-(4,5-dihydro-1*H*-imidazol-2-yl)-ethyl]-phenylamine (Example **49a**). 8-Benzo[1,3]dioxol-5-yl-1-{4-[2-(4,5-dihydro-1*H*-imidazol-2-yl)-ethyl]-phenyl}-1*H*-imidazo[4,5-*c*]quinoline, analytical HPLC: $t_R$= 6.35 minutes (Grad 1); $ES^+$-MS: $m/e_o$ = 462.2.

**Example 49a**

**4-[2-(4,5-Dihydro-1*H*-imidazol-2-yl)-ethyl]-phenylamine hydrogen chloride salt**

0.66 g (2.6 mmol) of 2-[2-(4-nitro-phenyl)-ethyl]-4,5-dihydro-1*H*-imidazole hydrogen chloride salt (Example **49c**) are dissolved in 30 mL of methanol and hydrogenated at RT in the presence of 100 mg of Pd/C 10%. After completion of the reaction, the catalyst is filtered-off and washed with methanol. The organic solvent is concentrated to dryness to provide 4-[2-(4,5-dihydro-1*H*-imidazol-2-yl)-ethyl]-phenylamine hydrogen chloride salt, analytical HPLC: $t_R$= 4.94 minutes (Grad 1); $ES^+$-MS: $m/e_o$ = 190.1.

**Example 49b**

**3-(4-Nitro-phenyl)-propionitrile**

4.6 g (20 mmol) of 4-nitrophenethyl bromide and 0.98 g (20 mmol) of sodium cyanide are dissolved in 50 mL of ethanol and the solution is refluxed for 18 hours. After this time, the solution is concentrated to dryness and the crude compound is purified by chromatography on silica eluting with DCM to afford 3-(4-nitro-phenyl)-propionitrile, analytical HPLC: $t_R$= 7.27 minutes (Grad 1); $ES^-$-MS: $m/e_o$ = 176.3.

**Example 49c**

**2-[2-(4-Nitro-phenyl)-ethyl]-4,5-dihydro-1*H*-imidazole hydrogen chloride salt**

1.6 g (9.08 mmol) of 3-(4-nitro-phenyl)-propionitrile (Example **49b**) are dissolved in 20.5 mL of a solution of DCM:ethanol (37: 1) at 0°C. HCl-gas is bubbled in the reaction mixture for 20 minutes and then the solution is stirred for 18 hours at RT. After this time, 200 mL of diethyl ether are added and the precipitate is removed by filtration to provide 0.97 g (3.75 mmol) of 3-(4-nitro-phenyl)-propionimidic acid ethyl ester hydrogen chloride salt. This compound is dissolved in 30 mL of ethanol and 0.27 mL (4.01 mmol) of ethylendiamine are added. The solution is refluxed for 18 hours and then 20 mL of water are added. The pH of the solvent is adjusted to pH 1 with concentrated HCl. The solution is extracted with DCM and the organic phase is discarded. The pH of the aqueous phase is adjusted to pH 10 with 1 N NaOH, and extracted with DCM. The organic solution is extracted with brine, dried over $MgSO_4$ and evaporated to dryness to provide 2-[2-(4-nitro-phenyl)-ethyl]-4,5-dihydro-1*H*-imidazole hydrogen chloride salt. The HCl salt is obtained by titration with 1.25 M HCl in methanol. 2-[2-(4-Nitro-phenyl)-ethyl]-4,5-dihydro-1*H*-imidazote hydrogen chloride salt, analytical HPLC: $t_R$= 7.05 minutes (Grad 1); $ES^-$-MS: $m/e_o$ = 220.1.

**Example 50**

**[3-Chloro-4-(2-methyl-8-thiophen-2-yl-imidazo[4,5-*c*]quinolin-1-yl)-phenyl]-acetonitrile**

[3-Chloro-4-(2-methyl-8-thlophen-2-yl-imidazo[4,5-*c*]quinolin-1-yl)-phenyl]-acetonitrile is obtained as described in Example 1 using (4-amino-3-chloro-phenyl)-acetonitrile (Example 7a), triethyorthoacetate and thiophene-2-boronic acid. [3-Chloro-4-(2-methyl-8-thiophen-2-yl-imidazo[4,5-c]quinolin-1-yl)-phenyl]-acetonitrile, analytical HPLC: $t_R$= 3.67 minutes (Grad 2); $ES^+$-MS: $m/e_o$ =414.9.

**Example 51**

**[3-Chloro-4-(2-methyl-8-thiophen-3-yl-imidazo[4,5-*c*]quinolin-1-yl)-phenyl]-acetonitrile**

[3-Chloro-4-(2-methyl-8-thiophen-3-yl-imidazo[4,5-*c*]quinolin-1-yl)-phenyl]-acetonitrile is obtained as described in Example **1** using (4-amino-3-chloro-phenyl)-acetonitrile (Example **7a),** triethyorthoacetate and thiophene-3-boronic acid. [3-Chloro-4-(2-methyl-8-thiophen-3-yl-imidazo[4,5-*c*]quinolin-1-yl)-phenyl]-acetonitrile, analytical HPLC: $t_R$= 3.67 minutes (Grad 2); $ES^+$-MS: $m/e_o$ = 414.9.

**Example 52**

**2-[3-Chloro-4-(2-methyl-8-thiophen-2-yl-imidazo[4,5-*c*]quinolin-1-yl)-phenyl]-ethylamine**

2-[3-Chloro-4-(2-methyl-8-thiophen-2-yl-imidazo[4,5-c]quinolin-1-yl)-phenyl]-ethylamine is obtained as described in Example **4** using [3-chloro-4-(2-methyl-8-thiophen-2-yl-imidazo[4,5-c]quinolin-1-yl)-phenyl]-acetonitrile (Example **50**) as starting material. 2-[3-Chloro-4-(2-methyl-8-thiophen-2-yl-imidazo[4,5-*c*]quinolin-1-yl)-phenyl]-ethylamine, analytical HPLC: $t_R$= 2.61 minutes (Grad 2); $ES^+$-MS: $m/e_o$ = 418.9.

**Example 53**

**2-[3-Chloro-4-(2-methyl-8-thiophen-3-yl-imidazo[4,5-*c*]quinolin-1-yl)-phenyl]-ethylamine**

2-[3-Chloro-4-(2-methyl-8-thiophen-3-yl-imidazo[4,5-*c*]quinolin-1-yl)-phenyl]-ethylarnine is obtained as described in Example **4** using [3-chloro-4-(2-methyl-8-thiophen-3-yl-imidazo[4,5-c]quinolin-1-yl)-phenyl]-acetonitrile (Example **51**) as starting material. 2-[3-Chloro-4-(2-methyl-8-thiophen-3-yl-imidazo[4,5-c]quinolin-1-yl)-phenyl]-ethylamine, analytical HPLC: $t_R$= 2.58 minutes (Grad 2); $ES^+$-MS: $m/e_o$ = 418.9.

**Example 54**

**14-(8-Benzo[1,3]dioxol-5-yl-7-fluoro-imidazo[4,5-*c*]quinolin-1-yl)-phenyl]-acetonitrile**

[4-(8-Benzo[1,3]dioxol-5-yl-7-fluoro-imidazo[4,5-*c*]quinolin-1-yl)-phenyl]-acetonitrile is obtained as described in Example **1** using 6-bromo-4-chloro-7-fluoro-3-nitro-quinoline (Example **54a**). [4-(8-Benzo[1,3]dioxol-5-yl-7-fluoro-imidazo[4,5-*c*]quinolin-1-yl)-phenyl]-acetonitrile, analytical HPLC: $t_R$ = 7.01 minutes (Grad 1); $ES^+$-MS: $m/e_o$ = 423.2.

**Example 54a**

**6-Bromo-4-chloro-7-fluoro-3-nitro-quinoline**

6-Bromo-4-chloro-7-fluoro-3-nitro-quinoline is obtained in analogy to 6-bromo-4-chloro-3-nitro-quinoline (Example **1c)** starting from 2-amino-5-bromo-4-fluoro-benzoic acid (Example **54b).** 6-Bromo-4-chloro-7-fluoro-3-nitro-quinoline, analytical HPLC: $t_R$= 2.30 minutes (Grad 7); $ES^-$-MS: $m/e_o$ = 287.1.

**Example 54b**

**2-amino-5-bromo-4-fluoro-benzoic acid**

20 g (128.9 mmol) of 2-amino-4-fluorobenzoic acid are dissolved in 470 mL of methanol and the solution is cooled at -70°C. To this stirred solution, 6.59 mL (128.2 mmol) of bromine dissolved in 130 mL of methanol are added slowly. After 3 hours, the solution is added to ice-water and the aqueous phase is extracted with ether. The combined organic portions are washed with water, brine, dried over $MgSO_4$ and concentrated *in vacuo* to provide 2-amino-5-bromo-4-fluoro-benzoic acid, analytical HPLC: $t_R$ = 2.96 minutes (Grad 5).
$^1H$ NMR (DMSO-$d_6$): δ 7.85 (d, 1H), 6.64 (d, 1 H).

**Example 55**

**[4-(7-Fluoro-8-thlophen-2-yl-imidazo[4,5-*c*]quinolin-1-yl)-phenyl]-acetonitrile**

[4-(7-Fluoro-8-thiophen-2-yl-imidazo[4,5-*c*]quinolin-1-yl)-phenyl]-acetonitrile is obtained as described in Example **1** using 6-bromo-4-chloro-7-fluoro-3-nitro-quinoline (Example 54a) and thiophene-2-boronic acid. [4-(7-Fluoro-8-thiophen-2-yl-imidazo[4,5-*c*]quinolin-1-yl)-phenyl]-acetonitrile, analytical HPLC: $t_R$= 7.20 minutes (Grad 1); $ES^+$-MS: m/$e_o$ = 385.1.

**Example 56**

**[4-(7-Fluoro-8-thiophen-3-yl-imidazo[4,5-c]quinolin-1-yl)-phenyl]-acetonitrile**

[4-(7-Fluoro-8-thiophen-3-yl-imidazo[4,5-*c*]quinolin-1-yl)-phenyl]-acetonitrile is obtained as described in Example **1** using 6-bromo-4-chloro-7-fluoro-3-nitro-quinoline (Example 54a) and thiophene-3-boronic acid. [4-(7-Fluoro-8-thiophen-3-yl-imidazo[4,5-*c*]quinolin-1-yl)-phenyl]-acetonitrile, analytical HPLC: $t_R$= 7.06 minutes (Grad 1); $ES^+$-MS: m/$e_o$ = 385.0.

**Example 57**

**[4-(8-Benzofuran-2-yl-7-fluoro-imidazo[4,5-*c*]quinolin-1-yl)-phenyl]-acetonitrile**

[4-(8-Benzofuran-2-yl-7-fluoro-imidazo[4.5-*c*]quinolin-1-yl)-phenyl]-acetonitrile is obtained as described in Example **1** using 6-bromo-4-chloro-7-fluoro-3-nitro-quinoline (Example 54a) and benzo[*b*]furan-2-boronic acid. [4-(8-Benzofuran-2-yl-7-fluoro-imidazo[4,5-*c*]quinolin-1-yl)-phenyl]-acetonitrile, analytical HPLC: $t_R$= 8.41 minutes (Grad 1); $ES^+$-MS: m/$e_o$ = 419.1.

**Example 58**

**{4-[7-Fluoro-8-(1*H*-indol-5-yl)-imidazo[4,5-*c*]quinolin-1-yl]-phenyl}-acetonitrile**

{4-[7-Fluoro-8-(1*H*-indol-5-yl)-imidazo[4,5-*c*]quinolin-1-yl]-phenyl}-acetonitrile is obtained as described in Example **1** using 6-bromo-4-chloro-7-fluoro-3-nitro-quinoline (Example **54a)** and 5-indolylboronic acid. {4-[7-Fluoro-8-(1*H*-indol-5-yl)-imidazo[4,5-*c*]quinolin-1-yl]-phenyl}-acetonitrile, analytical HPLC: $t_R$ = 6.79 minutes (Grad 1); $ES^+$-MS: m/$e_o$ = 418.2.

**Example 59**

**[4-(8-Benzo[*b*]thiophen-2-yl-7-fluoro-imidazo[4,5-*c*]quinolin-1-yl)-phenyl]-acetonitrile**

[4-(8-Benzo[*b*]thiophen-2-yl-7-fluoro-irnidazo[4,5-*c*]quinolin-1-yl)-phenyl]-acetonitrile is obtained as described in Example **1** using 6-bromo-4-chloro-7-fluoro-3-nitro-quinoline (Example **54a**) and benzo[*b*]thiophene-2-boronic acid. [4-(8-Benzo[*b*]thiophen-2-yl-7-fluoro-imidazo[4,5-*c*]quinolin-1-yl)-phenyl]-acetonitrile, analytical HPLC: $t_R$= 8.75 minutes (Grad 1); $ES^+$-MS: m/$e_o$ = 435.0.

**Example 60**

**2-[4-(8-Benzo[1,3]dioxol-5-yl-7-fluoro-imidazo[4,5-*c*]quinolin-1-yl)-phenyl]-ethylamine**

2-[4-(8-Benzo[1,3]dioxol-5-yl-7-fluoro-imidazo[4,5-c]quinolin-1-yl)-phenyl]-ethylamine is obtained as described in Example **4** using [4-(8-benzo[1,3]dioxol-5-yl-7-fluoro-imidazo[4,5-c]quinolin-1-yl)-phenyl]-acetonitrile (Example **53**) as starting material. 2-[4-(8-Benzo[1,3]dioxol-5-yl-7-fluoro-imidazo[4,5-*c*]quinolin-1-yl)-phenyl]-ethylamine, analytical HPLC: $t_R$ = 5.72 minutes (Grad 1); $ES^+$-MS: m/$e_o$ = 427.2.

**Example 61**

**2-[4-(7-Fluoro-8-thiophen-2-yl-imidazo[4,5-*c*]quinolin-1-yl)-phenyl]-ethylamine**

2-[4-(7-Fluoro-8-thiophen-2-yl-imidazo[4,5-*c*]quinolin-1-yl)-phenyl]-ethylamine is obtained as described in Example **4** using [4-(7-fluoro-8-thiophen-2-yl-imidazo[4,5-*c*]quinolin-1-yl)-phenyl]-acetonitrile (Example 55) as starting material. 2-[4-(7-Fluoro-8-thiophen-2-yl-imidazo[4,5-*c*]quinolin-1-yl)-phenyl]-ethylamine, analytical HPLC: $t_R$= 5.62 minutes (Grad 1); $ES^+$-MS: m/$e_o$ = 389.2.

**Example 62**

**2-[4-(7-Fluoro-8-thiophen-3-yl-imidazo[4,5-*c*]quinolin-1-yl)-phenyl]-ethylamine**

2-[4-(7-Fluoro-8-thiophen-3-yl-imidazo[4,5-*c*]quinolin-1-yl)-phenyl]-ethylamine is obtained as described in Example **4** using [4-(7-fluoro-8-thiophen-3-yl-imidazo[4,5-*c*]quinolin-1-yl)-phenyl]-acetonitrile (Example **56**) as starting material. 2-[4-(7-Fluoro-8-thiophen-3-yl-imidazo[4,5-*c*]quinolin-1-yl)-phenyl]-ethylamine, analytical HPLC: $t_R$ = 5.73 minutes (Grad 1); $ES^+$-MS: m/$e_o$ = 389.3.

**Example 63**

**2-[4-(8-Benzofuran-2-yl-7-fluoro-imidazo[4,5-*c*]quinolin-1-yl)-phenyl]-ethylamine**

2-[4-(8-Benzofuran-2-yl-7-fluoro-imidazo[4,5-*c*]quinolin-1-yl)-phenyl]-ethylamine is obtained as described in Example **4** using [4-(8-benzofuran-2-yl-7-fluoro-imidazo[4,5-*c*]quinolin-1-yl)-phenyl]-acetonitrile (Example 57) as starting material. 2-[4-(8-Benzofuran-2-yl-7-fluoro-imidazo[4,5-*c*]quinolin-1-yl)-phenyl]-ethylamine, analytical HPLC: $t_R$= 6.35 minutes (Grad 1); $ES^+$-MS: m/$e_o$ = 423.3.

**Example 64**

**2-{4-[7-Fluoro-8-(1*H*-indol-5-yl)-imidazo[4,5-*c*]quinolin-1-yl]-phenyl}-ethylamine**

2-{4-[7-Fluoro-8-(1*H*-indol-5-yl)-imidazo[4,5-*c*]quinolin-1-yl]-phenyl}-ethylamine is obtained as described in Example **4** using {4-[7-fluoro-8-(1*H*-indol-5-yl)-imidazo[4,5-*c*]quinolin-1-yl]-phenyl}-acetonitrile (Example **58)** as starting material. 2-{4-[7-Fluoro-8-(1*H*-indol-5-yl)-imidazo[4,5-c]quinolin-1-yl]-phenyl}-ethylamine, analytical HPLC: $t_R$= 5.60 minutes (Grad 1); $ES^+$-MS: m/$e_o$ = 422.3.

**Example 65**

**2-[4-(8-Benzo[*b*]thiophen-2-yl-7-fluoro-imidazo[4,5-*c*]quinolin-1-yl)-phenyl]-ethylamine**

2-[4-(8-Benzo[*b*]thiophen-2-yl-7-fluoro-imidazo[4,5-*c*]quinolin-1-yl)-phenyl]-ethylamine is obtained as described in Example **4** using [4-(8-benzo[*b*]thiophen-2-yl-7-fluoro-imidazo[4,5-c]quinolin-1-yl)-phenyl]-acetonitrile (Example 59) as starting material. 2-[4-(8-Benzo[*b*]thiophen-2-yl-7-fluoro-imidazo[4,5-*c*]quinolin-1-yl)-phenyl]-ethylamine, analytical HPLC: $t_R$ = 6.29 minutes (Grad 1); $ES^+$-MS: m/$e_o$ = 439.2.

**Example 66**

**3-[4-(8-Benzo[1,3]dioxol-5-yl-7-fluoro-imidazo[4,5-*c*]quinolin-1-yl)-phenyl]-propionitrile.**

3-[4-(8-Benzo[1,3]dioxol-5-yl-7-fluoro-imidazo[4,5-*c*]quinolin-1-yl)-phenyl]-propionltrile is synthesized as described in Example **1** using 6-bromo-4-chloro-7-fluoro-3-nitro-quinoline (Example **54a)** and 3-(4-amino-phenyl)-propionitrile (Example **31a).** 3-[4-(8-Benzo[1,3]dioxol-5-yl-7-fluoro-imidazo[4,5-c]quinolin-1-yl)-phenyl]-propionitrile, analytical HPLC: $t_R$= 6.93 minutes (Grad 1); $ES^+$-MS: m/$e_o$ = 437.1.

**Example 67**

**3-[4-(7-Fluoro-8-thiophen-2-yl-imidazo[4,5-*c*]quinolin-1-yl)-phenyl]-propionitrile**

3-[4-(7-Fluoro-8-thiophen-2-yl-imidazo[4,5-*c*]quinolin-1-yl)phenyl]-propionitrile is synthesized as described in Example **1** using 6-bromo-4-chloro-7-fluoro-3-nitro-quinoline (Example **54a),** 3-(4-amino-phenyl)-propionitrile (Example **31a)** and thiophene-2-boronic acid. 3-[4-(7-Fluoro-8-thiophen-2-yl-imidazo[4,5-c]quinolin-1-yl)-phenyl]-propionitrile, analytical HPLC: $t_R$= 7.30 minutes (Grad 1); $ES^+$-MS: m/$e_o$ = 399.1.

**Example 68**

**3-[4-(7-Fluoro-8-thiophen-3-yl-imidazo[4,5-*c*]quinolin-1-yl)-phenyl]-propionitrile**

3-[4-(7-Fluoro-8-thiophen-3-yl-imidazo[4,5-*c*]quinolin-1-yl)-phenyl]-propionitrile is obtained as described in Example **1** using 6-bromo-4-chloro-7-fluoro-3-nitro-quinoline (Example 54a), 3-(4-amino-phenyl)-propionitrile (Example **31a**) and thiophene-3-boronic acid. 3-[4-(7-Fluoro-8-thiophen-3-yl-imidazo[4,5-*c*]quinolin-1-yl)-phenyl]-propionitrile, analytical HPLC: $t_R$= 7.00 minutes (Grad 1); $ES^+$-MS: m/$e_o$ = 399.1.

**Example 69**

**3-[4-(8-Benzofuran-2-yl-7-fluoro-imidazo[4,5-*c*]quinolin-1-yl)-phenyl]-propionitrile**

3-[4-(8-Benzofuran-2-yl-7-fluoro-imidazo[4,5-*c*]quinolin-1-yl)-phenyl]-propionitrile is obtained as described in Example **1** using using 6-bromo-4-chloro-7-fluoro-3-nitro-quinoline (Example **54a**), 3-(4-amino-phenyl)-propionitrile (Example **31a**) and benzo[*b*]furan-2-boronic acid. 3-[4-(8-Benzofuran-2-yl-7-fluoro-imidazo[4,5-*c*]quinolin-1-yl)-phenyl]-propionitrile, analytical HPLC: $t_R$= 8.38 minutes (Grad 1); $ES^+$-MS: m/$e_o$ = 433.0.

**Example 70**

**3-[4-(8-Benzo[*b*]thiophen-2-yl-7-fluoro-imidazo[4,5-*c*]quinolin-1-yl)-phenyl]-propionitrile**

3-[4-(8-Benzo[*b*]thiophen-2-yl-7-fluoro-imidazo[4,5-*c*]quinolin-1-yl)-phenyl]-propionitrile is obtained as described in Example **1** using 6-bromo-4-chloro-7-fluoro-3-nitro-quinoline (Example **54a**), 3-(4-amino-phenyl)-propionitrile (Example **31a**) and benzo[*b*]thiophene-2-boronic acid. 3-[4-(8-Benzo[*b*]thiophen-2-yl-7-fluoro-imidazo[4,5-*c*]quinolin-1-yl)-phenyl]-propionitrile, analytical HPLC: $t_R$ = 8.58 minutes (Grad 1); $ES^+$-MS: m/$e_o$ = 449.1.

**Example 71**

**3-{4-[7-Fluoro-8-(1*H*-indol-5-yl)-imidazo[4,5-*c*]quinolin-1-yl]-phenyl}-propionitrile**

3-{4-[7-Fluoro-8-(1*H*-indol-5-yl)-imidazo[4,5-*c*]quinolin-1-yl]-phenyl}-propionitrile is obtained as described in Example **1** using using 6-bromo-4-chloro-7-fluoro-3-nitro-quinoline (Example **54a**), 3-(4-amino-phenyl)-propionitrile (Example **31a**) and 5-indolylboronic acid. 3-{4-[7-Fluoro-8-(1*H*-indol-5-yl)-imidazo[4,5-*c*]quinolin-1-yl]-phenyl}propionitrile, analytical HPLC: $t_R$ = 6.87 minutes (Grad 1); $ES^+$-MS: m/$e_o$ = 432.1.

**Example 72**

**3-[4-(8-Benzo[1,3]dioxol-5-yl-7-fluoro-imidazo[4,5-*c*]quinolin-1-yl)-phenyl]-propylamine**

3-[4-(8-Benzo[1,3]dioxol-5-yl-7-fluoro-imidazo[4,5-*c*]quinolin-1-yl)-phenyl]-propylamine is obtained as described in Example **4** using 3-[4-(8-benzo[1,3]dioxol-5-yl-7-fluoro-imidazo[4,5-c]quinolin-1-yl)-phenyl]-propionitrile (Example **66)** as starting material. 3-[4-(8-Benzo[1,3]dioxol-5-yl-7-fluoro-imidazo[4,5-*c*]quinolin-1-yl)-phenyl]-propylamine, analytical HPLC: $t_R$ = 6.36 minutes (Grad 1); $ES^+$-MS: m/$e_o$ = 441.5.

**Example 73**

**3-14-(7-Fluoro-8-thiophen-2-yl-imidazo[4,5-*c*]quinolin-1-yl)-phenyl]-propylamine**

3-[4-(7-Fluoro-8-thiophen-2-yl-imidazo[4,5-*c*]quinolin-1-yl)-phenyl]-propylamine is obtained as described in Example **4** using 3-[4-(7-fluoro-8-thiophen-2-yl-imidazo[4,5-*c*]quinolin-1-yl)-phenyl]-propionitrile (Example **67**) as starting material. 3-[4-(7-Fluoro-8-thiophen-2-yl-imidazo[4,5-*c*]quinolin-1-yl)-phenyl]-propylamine, analytical HPLC: $t_R$= 5.82 minutes (Grad 1); $ES^+$-MS: m/$e_o$ = 403.1.

**Example 74**

**3-[4-(7-Fluoro-8-thiophen-3-yl-imidazo[4,5-*c*]quinolin-1-yl)-phenyl]-propylamine**

3-[4-(7-Fluoro-8-thiophen-3-yl-imidazo[4,5-*c*]quinolin-1-yl)-phenyl]-propylamine is obtained as described in Example **4** using 3-[4-(7-fluoro-8-thiophen-3-yl-imidazo[4,5-*c*]quinolin-1-yl)-phenyl]-propionitrile (Example **68)** as starting material. 3-[4-(7-Fluoro-8-thiophen-3-yl-imidazo[4,5-*c*]quinolin-1-yl)-phenyl]-propylamine, analytical HPLC: $t_R$= 5.71 minutes (Grad 1); $ES^+$-MS: m/$e_o$ = 403.3.

**Example 75**

**3-[4-(8-Benzofuran-2-yl-7-fluoro-imidazo[4,5-*c*]quinolin-1-yl)-phenyl]-propylamine**

3-[4-(8-Benzofuran-2-yl-7-fluoro-imidazo[4,5-*c*]quinolin-1-yl)-phenyl]-propylamine is obtained as described in Example **4** using 3-[4-(8-benzofuran-2-yl-7-fluoro-imidazo[4,5-*c*]quinolin-1-yl)-phenyl]-propionitrile (Example **69)** as starting material. 3-[4-(8-Benzofuran-2-yl-7-fluoro-imidazo[4,5-*c*]quinolin-1-yl)-phenyl]-propylamine, analytical HPLC: $t_R$= 6.38 minutes (Grad 1); $ES^+$-MS: m/$e_o$ = 437.3.

**Example 76**

**3-[4-(8-Benzo[*b*]thiophen-2-yl-7-fluoro-imidazo[4,5-*c*]quinolin-1-yl)-phenyl]-propylamine**

3-[4-(8-Benzo[*b*]thiophen-2-yl-7-fluoro-imidazo[4,5-*c*]quinolin-1-yl)-phenyl]-propylamine is obtained as described in Example **4** using 3-[4-(8-benzo[*b*]thiophen-2-yl-7-fluoro-imidazo[4,5-c]quinolin-1-yl)-phenyl]-propionitrile (Example **70**) as starting material. 3-[4-(8-Benzo[*b*]thiophen-2-yl-7-fluoro-imidazo[4,5-*c*]quinolin-1-yl)-phenyl]-propylamine, analytical HPLC: $t_R$= 6.51 minutes (Grad 1); $ES^+$-MS: m/$e_o$ = 453.3.

**Example 77**

**3-{4-[7-Fluoro-8-(1*H*-indol-5-yl)-imidazo[4,5-*c*]quinolin-1-yl]-phenyl}-propylamine**

3-{4-[7-Fluoro-8-(1*H*-indol-5-yl)-imidazo[4,5-*c*]quinolin-1-yl]-phenyl}-propylamine is obtained as described in Example **4** using 3-{4-[7-fluoro-8-(1H-indol-5-yl)-imidazo[4,5-*c*]quinolin-1-yl]-phenyl}-propionitrile (Example **71**) as starting material. 3-{4-[7-Fluoro-8-(1*H*-indol-5-yl)-imidazo[4,5-*c*]quinolin-1-yl]-phenyl}-propylamine, analytical HPLC: $t_R$= 5.82 minutes (Grad 1); $ES^+$-MS: m/$e_o$ = 436.2.

**Example 78**

**[3-Chloro-4-(7-fluoro-8-thiophen-2-yl-imdazo[4,5-*c*]quinolin-1-yl)-phenyl]-acetonitrile**

[3-Chloro-4-(7-fluoro-8-thiophen-2-yl-irnidazo[4,5-*c*]quinolin-1-yl)-phenyl]-acetonitrile is obtained as described in Example 1 using 6-bromo-4-chloro-7-fluoro-3-nitro-quinoline (Example **54a**), (4-amino-3-chloro-phenyl)-acetonitrile (Example **7a**) and thiophene-2-boronic acid. [3-Chloro-4-(7-fluoro-8-thiophen-2-yl-imidazo[4,5-c]quinolin-1-yl)-phenyl]-acetonitrile, analytical HPLC: $t_R$= 7.50 minutes (Grad 1); $ES^+$-MS: m/$e_o$ = 419.3.

**Example 79**

**[3-Chloro-4-(7-fluoro-8-thiophen-3-yl-imidazo[4,5-*c*]quinolin-1-yl)-phenyl]-acetonitrile**

[3-Chloro-4-(7-fluoro-8-thiophen-3-yl-imidazo[4,5-c]quinolin-1-yl)-phenyl]-acetonitrile is obtained as described in Example **1** using 6-bromo-4-chloro-7-fluoro-3-nitro-quinoline. (Example **54a**), (4-amino-3-chloro-phenyl)-acetonitrile (Example **7a**) and thiophene-3-boronic acid. [3-Chloro-4-(7-fluoro-8-thiophen-3-yl-imidazo[4,5-*c*]quinolin-1-yl)-phenyl]-acetonitrile, analytical HPLC: $t_R$= 7.35 minutes (Grad 1); $ES^+$-MS: m/$e_o$ = 419.2.

**Example 80**

**{3-Chloro-4-[7-fluoro-8-(1*H*-indol-5-yl)-imidazo[4,5-*c*]quinolin-1-yl]-phenyl}-acetonitrile**

{3-Chloro-4-[7-fluoro-8-(1*H*-indol-5-yl)-imidazo[4,5-*c*]quinolin-1-yl]-phenyl}-acetonitrile is obtained as described in Example **1** using 6-bromo-4-chloro-7-fluoro-3-nitro-quinoline (Example 54a), (4-amino-3-chloro-phenyl)-acetonitrile (Example **7a**) and 5-indolylboronic acid. {3-Chloro-4-[7-fluoro-8-(1*H*-indol-5-yl)-imidazo[4,5-*c*]quinolin-1-yl]-phenyl}-acetonitrile, analytical HPLC: $t_R$= 7.03 minutes (Grad 1); $ES^+$-MS: m/$e_o$ = 452.3.

**Example 81**

**2-[3-Chloro-4-(7-fluoro-8-thiophen-2-yl-imidazo[4,5-*c*]quinolin-1-yl)-phenyl]-ethylamine**

2-[3-Chloro-4-(7-fluoro-8-thiophen-2-yl-imidazo[4,5-*c*]quinolin-1-yl)-phenyl]-ethylamine is obtained as described in Example **4** using [3-chloro-4-(7-fluoro-8-thiophen-2-yl-imidazo[4,5-c]quinolin-1-yl)-phenyl]-acetonitrile (Example **78**) as starting material. 2-[3-Chloro-4-(7-fluoro-8-thiophen-2-yl-imidazo[4,5-c]quinolin-1-yl)-phenyl]-ethylamine, analytical HPLC: $t_R$= 6.01 minutes (Grad 1); $ES^+$-MS: m/$e_o$ = 423.3.

**Example 82**

**2-[3-Chloro-4-(7-fluoro-8-thiophen-3-yl-imidazo[4,5-*c*]quinolin-1-yl)-phenyl]-ethylamine**

2-[3-Chloro-4-(7-fluoro-8-thiophen-3-yl-imidazo[4,5-c]quinolin-1-yl)-phenyl]-ethylamine is obtained as described in Example **4** using [3-chloro-4-(7-fluoro-8-thiophen-3-yl-imidazo[4,5-c]quinolin-1-yl)-phenyl]-acetonitrile (Example 79) as starting material. 2-[3-Chloro-4-(7-fluoro-8-thiophen-3-yl-imidazo[4,5-*c*]quinolin-1-yl)-phenyl]-ethylamine, analytical HPLC: $t_R$= 6.01 minutes (Grad 2); $ES^+$-MS: m/$e_o$ = 423.3.

**Example 83**

**2-{3-Chloro-4-[7-fluoro-8-(1*H*-indo)-5-yl)-imidazo[4,5-*c*]quinolin -1-yl]-phenyl}-ethylamine**

2-{3-Chloro-4-[7-fluoro-8-(1*H*-indol-5-yl)-imidazo[4,5-*c*]quinolin-1-yl]-phenyl}-ethylamine is obtained as described in Example **4** using {3-chloro-4-[7-fluoro-8-(1*H*-indol-5-yl)-imidazo[4,5-*c*]quinolin-1-yl]-phenyl)-acetonitrile (Example **80**) as starting material. 2-{3-Chloro-4-[7-fluoro-8-(1*H*-indol-5-yl)-imidazo[4,5-*c*]quinolin-1-yl]-phenyl}-ethylamine, analytical HPLC: $t_R$= 6.03 minutes (Grad 1); $ES^+$-MS: m/$e_o$ = 456.4.

**Example 84**

**[4-(7-Chloro-8-thiophen-2-yl-imidazo[4,5-*c*]quinolin-1-yl)-phenyl]-acetonitrile**

[4-(7-Chtoro-8-thiophen-2-yl-imidazo[4,5-*c*]quinolin-1-yl)-phenyl]-acetonitrite is obtained as described in Example **1** using 6-bromo-4,7-dichloro-3-nitro-quinoline (Example 84a) and thiophene-2-boronic acid. [4-(7-Chloro-8-thiophen-2-yl-imidazo[4,5-*c*]quinolin-1-yl)-phenyl]-acetonitrile, analytical HPLC: $t_R$= 7.44 minutes (Grad 1); $ES^+$-MS: m/$e_o$ = 401.1.

**Example 84a**

**6-Bromo-4,7-dichtoro-3-nitro-quinoline**

6-Bromo-4,7-dichloro-3-nitro-quinoline is obtained as described in analogy to 6-bromo-4-chloro-3-nitro-quinoline (Example **1c**) starting from 2-amino-5-bromo-4-chloro-benzoic acid (Example **84b**). 6-Bromb-4,7-dichloro-3-nitro-quinoline, analytical HPLC: $t_R$= 9.48 minutes (Grad 1); $ES^+$-MS: m/$e_o$ = 323.0.

**Example 84b**

**2-Amino-5-bromo-4-chloro-benzoic acid**

2-Amino-5-bromo-4-chloro-benzoic acid is obtained as described in Example **54b** starting with 2-amino-4-chlorobenzoic acid. 2-Amino-5-bromo-4-chloro-benzoic acid, analytical HPLC: $t_R$= 3.21 minutes (Grad 5).
$^1$H NMR (DMSO-$d_6$): δ 7.85 (s, 1H), 6.95 (s, 1H).

**Example 85**

**[4-(7-Chloro-8-thiophen-3-yl-imidazo[4,5-*c*]quinolin-1-yl)-phenyl]-acetonitrile**

[4-(7-Chloro-8-thiophen-3-yl-imidazo[4,5-*c*]quinolin-1-yl)-phenyl]-acetonitrile is obtained as described in Example **1** using 6-bromo-4,7-dichloro-3-nitro-quinoline (Example 84a) and thiophene-3-boronic acid. [4-(7-Chloro-8-thiophen-3-yl-imidazo[4,5-*c*]quinolin-1-yl)-phenyl]-acetonitrile, analytical HPLC: $t_R$= 7.23 minutes (Grad 1); $ES^+$-MS: m/$e_o$ = 401.1.

**Example 86**

**[4-(8-Benzofuran-2-yl-7-chloro-imidazo[4,5-*c*]quinolin-1-yl)-phenyl]-acetonitrile**

[4-(8-Benzofuran-2-yl-7-chloro-imidazo[4,5-*c*]quinolin-1-yl)-phenyl]-acetonitrile is obtained as described in Example **1** using 6-bromo-4,7-dichloro-3-nitro-quinoline (Example **84a)** and benzo[*b*]furan-2-boronic acid. [4-(8-Benzofuran-2-yl-7-chloro-imidazo[4,5-*c*]quinolin-1-yl)-phenyl]-acetonitrile, analytical HPLC: $t_R$ = 7.44 minutes (Grad 1); $ES^+$-MS: m/$e_o$ = 401.1.

**Example 87**

**{4-[7-Chloro-8-(1*H*-indol-5-yl)-imidazo[4,5-*c*]quinolin-1-yl]-phenyl}-acetonitrile**

{4-[7-Chloro-8-(1*H*-indol-5-yl)-imidazo[4,5-*c*]quinolin-1 -yl]-phenyl}-acetonitrile is obtained as described in Example **1** using 6-bromo-4,7-dichloro-3-nitro-quinoline (Example **84a**) and 5-indolylboronic acid. {4-[7-Chloro-8-(1*H*-indol-5-yl)-imidazo[4,5-*c*]quinolin-1-yl]-phenyl}-acetonitrile, analytical HPLC: $t_R$= 6.93 minutes (Grad 1); $ES^+$-MS: m/$e_o$ = 434.1.

**Example 88**

**[4-(B-Benzo[*b*]thlophen-2-yl-7-chloro-imidazo[4,5-*c*]quinolin-1-yl)-phenyl]-acetonitrile**

[4-(8-Benzo[*b*]thiophen-2-yl-7-chloro-imidazo[4,5-*c*]quinolin-1-yl)-phenyl]-acetonitrile is obtained as described in Example **1** using 6-bromo-4,7-dichloro-3-nitro-quinoline (Example **84a**) and benzo[*b*]thiophene-2-boronic acid. [4-(8-Benzo[*b*]thiophen-2-yl-7-chloro-imidazo[4,5-*c*]quinolin-1-yl)-phenyl]-acetonitrile, analytical HPLC: $t_R$ = 8.83 minutes (Grad 1); $ES^+$-MS: m/$e_o$ = 451.1.

**Example 89**

**2-[4-(7-Chloro-8-thiophen-2-yl-imidazo[4,5-*c*]quinolin-1-yl)-phenyl]-ethylamine**

2-[4-(7-Chloro-8-thiophen-2-yl-imidazo[4,5-*c*]quinolin-1-yl)-phenyl]-ethylamine is obtained as described in Example **4** using [4-(7-chloro-8-thiophen-2-yl-imidazo[4,5-*c*]quinolin-1-yl)-phenyl]-acetonitrile (Example 84) as starting material. 2-[4-(7-Chloro-8-thiophen-2-yl-imidazo[4,5-*c*]quinolin-1-yl)-phenyl]-ethylamine, analytical HPLC: $t_R$= 5.91 minutes

(Grad 1); ES+-MS: m/$e_o$ = 405.2.

**Example 90**

**2-[4-(7-Chloro-8-thiophen-3-yl-imidazo[4,5-*c*]quinolin-1-yl)-phenyl]-ethylamine**

2-[4-(7-Chloro-8-thiophen-3-yl-imidazo[4,5-*c*]quinolin-1-yl)-phenyl]-ethylamine is obtained as described in Example **4** using [4-(7-chloro-8-thiophen-3-yl-imidazo[4,5-*c*]quinolin-1-yl)-phenyl]-acetonitrile (Example **85**) as starting material. 2-[4-(7-Chloro-8-thiophen-3-yl-imidazo[4,5-*c*]quinolin-1-yl)-phenyl]-ethylamine, analytical HPLC: $t_R$= 5.87 minutes (Grad 1); ES+-MS: m/$e_o$ = 405.2.

**Example 91**

**2-[4-(8-Benzofuran-2-yl-7-chloro-imidazo[4,5-*c*]quinolin-1-yl)-phenyl]-ethylamine**

2-[4-(8-Benzofuran-2-yl-7-chloro-imidazo[4,5-*c*]quinolin-1-yl)-phenyl]-ethylamine is obtained as described in Example **4** using [4-(8-benzofuran-2-yl-7-chloro-imidazo[4,5-*c*]quinolin-1-yl)-phenyl]-acetonitrile (Example **86**) as starting material. 2-[4-(8-Benzofuran-2-yl-7-chloro-imidazo[4,5-*c*]quinolin-1-yl)-phenyl]-ethylamine, analytical HPLC: $t_R$= 6.52 minutes (Grad 1); ES+-MS: m/$e_o$ = 439.2.

**Example 92**

**2-{4-[7-Chloro-8-(1*H*-indol-5-yl)-imidazo[4,5-*c*]quinolin-1-yi]-phenyl}-ethylamine**

2-{4-[7-Chloro-8-(1*H*-indol-5-yl)-imidazo[4,5-*c*]quinolin-1-yl]-phenyl}-ethylamine is obtained as described in Example **4** using {4-[7-chloro-8-(1H-indol-5-yl)-imidazo[4,5-*c*]quinolin-1-yl]-phenyl}-acetonitrile (Example **87**) as starting material. 2-{4-[7-Chloro-8-(1*H*-indol-5-yl)-imidazo[4,5-*c*]quinolin-1-yl]-phenyl}-ethylamine, analytical HPLC: $t_R$= 5.83 minutes (Grad 1); ES+-MS: m/$e_o$ = 438.2.

**Example 93**

**2-[4-(8-Benzo[*b*]thiophen-2-yl-7-chloro-imidazo[4,5-*c*]quinolin-1-yl)-phenyl]-ethylamine**

2-[4-(8-Benzo[*b*]thiophen-2-yl-7-chloro-imidazo[4,5-*c*]quinolin-1-yl)-phenyl]-ethylamine is obtained as described in Example **4** using [4-(8-benzo[b]thiophen-2-yl-7-chloro-imidazo[4,5-c]quinolin-1-yl)-phenyl]-acetonitrile (Example 88) as starting material. 2-[4-(8-Benzo[*b*]thiophen-2-yl-7-chloro-imidazo[4,5-*c*]quinolin-1-yl)-phenyl]-ethylamine, analytical HPLC: $t_R$= 6.55 minutes (Grad 1); ES+-MS: m/$e_o$ = 455.3.

**Example 94**

**3-[4-(8-Benzo[1,3]dioxol-5-yl-7-chloro-imidazo[4,5-*c*]quinolin-1-yl)-phenyl]-propionitrile**

3-[4-(8-Benzo[1,3]dioxol-5-yl-7-chtoro-imidazo[4,5-*c*]quinolin-1-yl)-phenyl]-propionltrile is obtained as described in Example **4** using 6-bromo-4,7-dichloro-3-nitro-quinoline (Example **84a**) and 3-(4-amino-phenyl)-propionitrile (Example **31a**). 3-[4-(8-Benzo[1,3]dioxol-5-yl-7-chloro-imidazo[4,5-*c*]quinolin-1-yl)-phenyl]-propionitrile, analytical HPLC: $t_R$ = 7.34 minutes (Grad 1); ES+-MS: m/$e_o$ = 453.1.

**Example 95**

**3-[4-(7-Chloro-8-thiophen-2-yl-imidazo[4,5-*c*]quinolin-1-yl)-phenyl]-propionitrile**

3-[4-(7-Chloro-8-thiophen-2-yl-imidazo[4,5-*c*]quinolin-1-yl)-phenyl]-propionitrile is obtained as described in Example **1** using 6-bromo-4,7-dichloro-3-nitro-quinoline (Example **84a**), 3-(4-amino-phenyl)-propionitrile (Example 31 a) and thiophene-2-boronic acid. 3-[4-(7-Chloro-8-thiophen-2-yl-imidazo[4,5-*c*]quinolin-1-yl)-phenyl]-propionitrile, analytical HPLC: $t_R$ = 7.54 minutes (Grad 1); ES+-MS: m/$e_o$ = 415.1.

**Example 96**

**3-[4-(8-Benzofuran-2-yl-7-chloro-imidazo[4,5-*c*]quinolin-1-yl)-phenyl]-propionitrile**

3-[4-(8-Benzofuran-2-yl-7-chloro-imidazo[4,5-c]quinolin-1-yl)-phenyl]-propionitrile is obtained as described in Example **1** using 6-bromo-4,7-dichloro-3-nitro-quinoline (Example **84a**), 3-(4-amino-phenyl)-propionitrile (Example **31a**) and benzo[*b*]furan-2-boronic acid. 3-[4-(8-Benzofuran-2-yl-7-chloro-imidazo[4,5-*c*]quinolin-1-yl)-phenyl]-propionitrile, analytical HPLC: $t_R$ = 8.93 minutes (Grad 1); $ES^+$-MS: m/$e_o$ = 449.1.

**Example 97**

**3-{4-[7-Chloro-8-(1*H*-indol-5-yl)-imidazo[4,5-*c*]quinolin-1-yl]-phenyl}-propionitrile**

3-{4-[7-Chloro-B-(1*H*-indol-5-yl)-imidazo[4,5-*c*]quinolin-1-yl]-phenyl}-propionitrile is obtained as escribed in Example **1** using 6-bromo-4,7-dichloro-3-nitro-quinoline (Example **84a**), 3-(4-amino-phenyl)-propionitrile (Example **31a**) and 5-indolylboronic acid. 3-{4-[7-Chloro-8-(1*H*-indol-5-yl)-imidazo[4,5-c]quinolin-1-yl]-phenyl}-propionitrile, analytical HPLC: $t_R$= 7.01 minutes (Grad 1); $ES^+$-MS: m/$e_o$ = 448.1.

**Example 98**

**3-[4-(8-Benzo[*b*]thiophen-2-yl-7-chloro-imidazo[4,5-*c*]quinolin-1-yl)-phenyl]-propionitrile**

3-[4-(8-Benzo[*b*]thiophen-2-yl-7-chloro-imidazo[4,5-*c*]quinolin-1-yl)-phenyl]-propionitrile is obtained as described in Example **1** using 6-bromo-4,7-dichloro-3-nitro-quinoline (Example **84a**), 3-(4-amino-phenyl)-propionitrile (Example **31a**) and benzo[*b*]thiophene-2-boronic acid. 3-[4-(8-Benzo[*b*]thiophen-2-yl-7-chloro-imidazo[4,5-*c*]quinolin-1-yl)-phenyl]-propionitrile, analytical HPLC: $t_R$ = 8.74 minutes (Grad 1); $ES^+$-MS: m/$e_o$ = 465.0.

**Example 99**

**3-[4-(8-Benzo[1,3]dioxol-5-yl-7-chloro-imidazo[4,5-*c*]quinolin-1-yl)-phenyl]-propylamine**

3-[4-(8-Benzo[1,3]dioxol-5-yl-7-chloro-imidazo[4,5-c]quinolin-1-yl)-phenyl]-propylamine is obtained as described in Example **4** using 3-[4-(8-benzo[1,3]dioxol-5-yl-7-chloro-imidazo[4,5-c]quinolin-1-yl)-phenyl]-propionitrile (Example **94**) as starting material. 3-[4-(8-Benzo[1,3]dioxol-5-yl-7-chloro-imidazo[4,5-c]quinolin-1-yl)-phenyl]-propylamine, analytical HPLC: $t_R$= 5.98 minutes (Grad 1); $ES^+$-MS: m/$e_o$ = 457.1.

**Example 100**

**3-[4-(7-Chloro-8-thiophen-2-yl-imidazo[4,5-c]quinolin-1-yl)-phenyl]-propylamine**

3-[4-(7-Chloro-8-thiophen-2-yl-imidazo[4,5-*c*]quinotin-1-yl)-phenyl]-propylamine is obtained as described in Example **4** using 3-[4-(7-chloro-8-thiophen-2-yl-imidazo[4,5-c]quinolin-1-yl)-phenyl]-propionitrile (Example **95**) as starting material. 3-[4-(7-Chloro-8-thiophen-2-yl-imidazo[4,5-c]quinolin-1-yl)-phenyl]-propylamine, analytical HPLC: $t_R$= 6.11 minutes (Grad 1); $ES^+$-MS: m/$e_o$ = 419.3.

**Example 101**

**3-[4-(8-Benzofuran-2-yl-7-chloro-imidazo[4,5-*c*]quinolin-1-yl)-phenyl]-propylamine**

3-[4-(8-Benzofuran-2-yl-7-chloro-imidazo[4,5-*c*]quinolin-1-yl)-phenyl]-propylamine is obtained as described in Example **4** using 3-[4-(8-benzofuran-2-yl-7-chloro-imidazo[4,5-*c*]quinolin-1-yl)-phenyl]-propionitrile (Example **96**) as starting material. 3-[4-(8-Benzofuran-2-yl-7-chloro-imidazo[4,5-*c*]quinolin-1-yl)-phenyl]-propylamine, analytical HPLC: $t_R$= 6.62 minutes (Grad 1); $ES^+$-MS: m/$e_o$ = 453.4.

**Example 102**

**3-{4-[7-Chloro-8-(1*H*-indol-5-yl)-imidazo[4,5-c]quinolin-1-yl]-phenyl}-propylamine**

3-{4-[7-Chloro-8-(1*H*-indol-5-yl)-imidazo[4,5-*c*]quinolin-1-yl]-phenyl}-propylamine is obtained as described in Example **4** using 3-{4-[7-chloro-8-(1*H*-indol-5-yl)-imidazo[4,5-*c*]quinolin-1-yl]-phenyl}-propionitrile (Example **97**) as starting material. 3-{4-[7-Chloro-8-(1*H*-indol-5-yl)-imidazo[4,5-c]quinolin-1-yl]-phenyl}-propylamine, analytical HPLC: $t_R$= 6.03 minutes (Grad 1); $ES^+$-MS: m/$e_o$ = 452.3.

**Example 103**

**3-[4-(8-Benzo[*b*]thiophen-2-yl-7-chloro-imidazo[4,5-*c*]quinolin-1-yl)-phenyl]-propylamine**

3-[4-(8-Benzo[*b*]thiophen-2-yl-7-chloro-imidazo[4,5-*c*]quinolin-1-yl)-phenyl]-propylamine is obtained as described in Example **4** using 3-[4-(8-benzo[*b*]thiophen-2-yl-7-chloro-imidazo[4,5-*c*]quinolin-1-yl)-phenyl]-propionitrile (Example **98)** as starting material. 3-[4-(8-Benzo[*b*]thiophen-2-yl-7-chloro-imidazo[4,5-*c*]quinolin-1-yl)-phenyl]-propylamine, analytical HPLC: $t_R$= 6.62 minutes (Grad 1); $ES^+$-MS: m/$e_o$ = 469.3.

**Example 104**

**[3-Chloro-4-(7-chloro-8-thiophen-2-yl-imidazo[4,5-*c*]quinolin-1-yl)-phenyl]-acetonitrile**

[3-Chloro-4-(7-chloro-8-thiophen-2-yl-imidazo[4,5-*c*]quinolin-1-yl)-phenyl]-acetonitrile is obtained as described in Example **1** using 6-bromo-4,7-dichloro-3-nitro-quinoline (Example **84a),** (4-amino-3-chloro-phenyl)-acetonitrile (Example **7a)** and thiophene-2-boronic acid. [3-Chloro-4-(7-chloro-8-thiophen-2-yl-imidazo[4,5-*c*]quinolin-1-yl)-phenyl]-acetonitrile, analytical HPLC: $t_R$= 7.93 minutes (Grad 1); $ES^+$-MS: m/$e_o$ = 434.9.

**Example 105**

**[3-Chloro-4-(7-chloro-8-thiophen-3-yl-imidazo[4,5-*c*]quinolin-1-yl)-phenyl]-acetonitrile**

[3-Chloro-4-(7-chloro-8-thiophen-3-yl-imidazo[4,5-*c*]quinolin-1-yl)-phenyl]-acetonitrile is obtained as described in Example **1** using 6-bromo-4,7-dichloro-3-nitro-quinoline (Example **84a),** (4-amino-3-chloro-phenyl)-acetonitrile (Example **7a**) and thiophene-3-boronic acid. [3-Chloro-4-(7-chloro-8-thiophen-3-yl-imidazo[4,5-*c*]quinolin-1-yl)-phenyl]-acetonitrile, analytical HPLC: $t_R$= 7.51 minutes (Grad 1); $ES^+$-MS: m/$e_o$ = 435.0.

**Example 106**

**2-[3-Chloro-4-(7-chloro-8-thiophen-2-yl-imidazo[4,5-*c*]quinolin-1-yl)-phenyl]-ethylarnine**

2-[3-Chloro-4-(7-chloro-8-thiophen-2-yl-imidazo[4,5-*c*]quinolin-1-yl)-phenyl]-ethylamine is obtained as described in Example **4** using [3-chloro-4-(7-chloro-8-thiophen-2-yl-imidazo[4,5-*c*]quinolin-1-yl)-phenyl]-acetonitrile (Example **104**). 2-[3-Chloro-4-(7-chloro-8-thiophen-2-yl-imidazo[4,5-*c*]quinolin-1-yl)-phenyl]-ethylamine, analytical HPLC: $t_R$= 6.16 minutes (Grad 1); $ES^+$-MS: m/$e_o$ = 439.0.

**Example 107**

**2-[3-Chloro-4-(7-chloro-8-thiophen-3-yl-imidazo[4,5-*c*]quinolin-1-yl)-phenyl]-ethylamine**

2-[3-Chloro-4-(7-chloro-8-thiophen-3-yl-imidazo[4,5-*c*]quinolin-1-yl)-phenyl]-ethylamine is obtained as described in Example **4** using [3-chloro-4-(7-chloro-8-thiophen-3-yl-imidazo[4,5-c]quinolin-1-yl)-phenyl]-acetonitrile (Example **105**) as starting material. 2-[3-Chloro-4-(7-chloro-8-thiophen-3-yl-imidazo[4,5-*c*]quinolin-1-yl)-phenyl]-ethylamine, analytical HPLC: $t_R$= 6.12 minutes (Grad 1); $ES^+$-MS: m/$e_o$ = 438.9.

**Example 108**

**3-[3-Chloro-4-(7-chloro-8-thiophen-2-yl-imidazo[4,5-*c*]quinolin-1-yl)-phenyl]-propionitrile**

3-[3-Chloro-4-(7-chloro-8-thiophen-2-yl-imidazo[4,5-*c*]quinolin-1-yl)-phenyl]-propionitrile is obtained as described in Example **1** using 6-bromo-4,7-dichloro-3-nitro-quinoline (Example **84a**), 3-(4-amino-3-chloro-phenyl)-propionitrile (Example **41a**) and thiophene-2-boronic acid. 3-[3-Chloro-4-(7-chloro-8-thiophen-2-yl-imidazo[4,5-*c*]quinolin-1-yl)-phenyl]-propionitrile, analytical HPLC: $t_R$= 7.98 minutes (Grad 1); $ES^+$-MS: m/$e_o$ = 449.2.

**Example 109**

**3-[3-Chloro-4-(7-chloro-8-thiophen-3-yl-imidazo[4,5-*c*]quinolin-1-yl)-phenyl]-propionitrile**

3-[3-Chloro-4-(7-chloro-8-thiophen-3-yl-imidazo[4,5-*c*]quinolin-1-yl)-phenyl]-propionitrile is obtained as described in Example **1** using 6-bromo-4,7-dichloro-3-nitro-quinoline (Example **84a**), 3-(4-amino-3-chloro-phenyl)-propionitrile (Example **41a**) and thiophene-3-boronic acid. 3-[3-Chloro-4-(7-chloro-8-thiophen-3-yl-imidazo[4,5-*c*]quinolin-1-yl)-phenyl]-propionitrile, analytical HPLC: $t_R$= 7.60 minutes (Grad 1); $ES^+$-MS: m/$e_o$ = 449.2.

**Example 110**

**3-{3-Chloro-4-[7-chloro-8-(1*H*-indol-5-yl)-imidazo[4,5-*c*]quinolin-1-yl]-phenyl}-propionitrile**

3-{3-Chloro-4-[7-chloro-8-(1*H*-indol-5-yl)-imidazo[4,5-*c*]quinolin-1-yl]-phenyl}-propionitrileis obtained as described in Example **1** using 6-bromo-4,7-dichloro-3-nitro-quinoline (Example 84a), 3-(4-amino-3-chloro-phenyl)-propionitrile (Example **41a**) and 5-indolylboronic acid. 3-{3-Chloro-4-[7-chloro-8-(1*H*-indol-5-yl)-imidazo[4,5-*c*]quinolin-1-yl]-phenyl}-propionitrile, analytical HPLC: $t_R$ = 7.29 minutes (Grad 1); $ES^+$-MS: m/$e_o$ = 481.8.

**Example 111**

**3-[3-Chloro-4-(7-chloro-8-thiophen-2-yl-imidazo[4,5-*c*]quinolin-1-yl)-phenyl]-propylamine**

3-[3-Chloro-4-(7-chloro-8-thiophen-2-yl-imidazo[4,5-*c*]quinolin-1-yl)-phenyl]-propylamine is obtained as described in Example **4** using 3-[3-chloro-4-(7-chloro-8-thiophen-2-yl-imidazo[4,5-*c*]quinolin-1-yl)-phenyl]-propionitrile (Example **108**) as starting material. 3-[3-Chloro-4-(7-chloro-8-thiophen-2-yl-imidazo[4,5-*c*]quinolin-1-yl)-phenyl]-propylamine, analytical HPLC: $t_R$= 6.31 minutes (Grad 1); $ES^+$-MS: m/$e_o$ = 453.0.

**Example 112**

**3-[3-Chloro-4-(7-chloro-8-thiophen-3-yl-imidazo[4,5-*c*]quinolin-1-yl)-phenyl]-propylamine**

3-[3-Chloro-4-(7-chloro-8-thiophen-3-yl-imidazo[4,5-*c*]quinotin-1-yl)-phenyl]-propylamine is obtained as described in Example **4** using 3-[3-chloro-4-(7-chloro-8-thiophen-3-yl-imidazo[4,5-*c*]quinolin-1-yl)-phenyl]-propionitrile (Example **109**) as starting material. 3-[3-Chloro-4-(7-chloro-8-thiophen-3-yl-imidazo[4,5-*c*]quinolin-1-yl)-phenyl]-propylamine, analytical HPLC: $t_R$ = 6.22 minutes (Grad 1); $ES^+$-MS: m/$e_o$ = 453.0.

**Example 113**

**3-{3-Chloro-4-[7-chloro-8-(1*H*-indol-5-yl)-imidazo[4,5-*c*]quinolin-1-yl]-phenyl}-propylamine**

3-{3-Chloro-4-[7-chloro-8-(1*H*-indol-5-yl)-imidazo[4,5-*c*]quinolin-1-yl]-phenyl}-propylamine is obtained as described in Example **4** using 3-{3-chloro-4-[7-chloro-8-(1*H*-indol-5-yl)-imidazo[4,5-*c*]quinolin-1-yl]-phenyl}-propionitrile (Example **110**) as starting material. 3-{3-Chloro-4-[7-chloro-8-(*H*-indol-5-yl)-imidazo[4,5-*c*]quinolin-1-yl]-phenyl}-propylamine, analytical HPLC: $t_R$= 6.03 minutes (Grad 1); $ES^+$-MS: m/$e_o$ = 486.0.

**Example 114**

**3-[4-(2-Amino-7-chloro-8-thiophen-3-yl-imidazo[4,5-*c*]quinolin-1-yl)-phenyl]-propionitrile**

3-[4-(2-Amino-7-chloro-8-thiophen-3-yl-imidazo[4,5-*c*]quinolin-1-yl)-phenyl]-propionitrileis obtained as described in Example **1** using 3-[4-(2-amino-8-bromo-7-chloro-imidazo[4,5-*c*]quinolin-1-yl)-phenyl]-propionitrile (Example **114a**) and thiophene-3-boronic acid. 3-[4-(2-Amino-7-chloro-8-thiophen-3-yl-imidazo[4,5-*c*]quinolin-1-yl)-phenyl]-propionitrile, analytical HPLC: $t_R$= 1.77 minutes (Grad 7); $ES^+$-MS: m/$e_o$ = 430.1.

**Example 114a**

**3-[4-(2-Amino-8-bromo-7-chloro-imidazo[4,5-*c*]quinolin-1-yl)-phenyl]-propionitrile**

1.8 g (4.48 mmol) of 3-[4-(3-amino-6-bromo-7-chloro-quinolin-4-ylamino)-phenyl]-propionitrile (Example **114b**) are dissolved in 90 mL of ethanol and 1.4 g (0.75 mmol) of cyanogen bromide are added. The solution is stirred for 3 days at RT. After this time, the solvent is evaporated to dryness and the crude compound is purified by medium-pressure liquid chromatography to obtain 3-[4-(2-amino-8-bromo-7-chloro-imidazo[4,5-*c*]quinolin-1-yl)-phenyl]-propionitrile, analytical HPLC: $t_R$ = 1.68 minutes (Grad 7); $ES^+$-MS: m/$e_o$ = 428.0.

**Example 114b**

**3-[4-(3-Amino-6-bromo-7-chloro-quinolin-4-ylamino)-phenyl]-propionitrile**

3-[4-(3-Amino-6-bromo-7-chloro-quinolin-4-ylamino)-phenyl]-propionitrile is obtained as described in Examples **1a** to **1e** using 6-bromo-4,7-dichloro-3-nitro-quinoline (Example **84a**) and 3-(4-amino-phenyl)-propionitrile (Example **31a**). 3-[4-(3-Amino-6-bromo-7-chloro-quinolin-4-ylamino)-phenyl]-propionitrile, analytical HPLC: $t_R$= 2.11 minutes (Grad 7); $ES^+$-MS: m/$e_o$ = 433.0.

**Example 115**

**3-[4-(2-Amino-8-benzofuran-2-yl-7-chloro-imidazo[4,5-*c*]quinolin-1-yl)-phenyl]-propionitrile**

3-[4-(2-Amino-8-benzofuran-2-yl-7-chloro-imidazo[4,5-*c*]quinolin-1-yl)-phenyl]-propionitrile is obtained as described in Example **1** using 3-[4-(2-amino-8-bromo-7-chloro-imidazo[4,5-c]quinolin-1-yl)-phenyl]-propionitrile (Example **114a**) and benzo[*b*]furan-2-botonic acid. 3-[4-(2-Amino-8-benzofuran-2-yl-7-chloro-imidazo[4,5-*c*]quinolin-1-yl)-phenyl]-propionitrile, analytical HPLC: $t_R$= 1.92 minutes (Grad 7); $ES^+$-MS: m/$e_o$ = 464.4.

**Example 116**

**1-[4-(3-Amino-propyl)-phenyl]-7-chloro-8-thiophen-3-yl-1 *H*-imidazo[4,5-*c*]quinolin-2-ylamine**

1-[4-(3-Amino-propyl)-phenyl]-7-chloro-8-thiophen-3-yl-1*H*-imidazo[4,5-*c*]quinolin-2-ylamine is obtained as described in Example **4** using 3-[4-(2-amino-7-chloro-8-thiophen-3-yl-imidazo[4,5-*c*]quinolin-1-yl)-phenyl]-propionitrile (Example **114**) as starting material. 1-[4-(3-Amino-propyl)-phenyl]-7-chloro-8-thiophen-3-yl-1*H*-imidazo[4,5-*c*]quinolin-2-ylamine, analytical HPLC: $t_R$= 1.56 minutes (Grad 7); $ES^+$-MS: m/$e_o$ = 434.7.

**Example 117**

**1-[4-(3-Amino-propyl)-phenyl]-8-benzofuran-2-yl-7-chloro-1*H*-imidazo[4,5-*c*]quinolin-2-ylamine**

1-[4-(3-Amino-propyl)-phenyl]-8-benzofuran-2-yl-7-chloro-1*H*-imidazo[4,5-*c*]quinolin-2-ylamine is obtained as described in Example **4** using 3-[4-(2-amino-8-benzofuran-2-yl-7-chloro-imidazo[4,5-*c*]quinolin-1-yl)-phenyl]-propionitrile (Example **115)** as starting material. 1-[4-(3-Amino-propyl)-phenyl]-8-benzofuran-2-yl-7-chloro-1*H*-imidazo[4,5-*c*]quinolin-2-ylamine, analytical HPLC: $t_R$ = 1.70 mintues (Grad 7); $ES^+$-MS: m/$e_o$ = 468.7.

**Example 118**

**8-(2,4-Dimethoxy-phenyl)-1-(2-fluoro-phenyl)-1*H*-imidazo[4,5-*c*]quinoline**

8-(2,4-Dimethoxy-phenyl)-1-(2-fluoro-phenyl)-1*H*-imidazo[4,5-*c*]quinoline is obtained as described in Example **1** using 2-fluoroaniline and 2,4-dimethoxyphenylboronic acid. 8-(2,4-Dimethoxy-phenyl)-1-(2-fluoro-phenyl)-1*H*-imidazo [4,5-*c*]quinoline, analytical HPLC: $t_R$ = 7.35 minutes (Grad 1); $ES^+$-MS: m/$e_o$ = 400.4.

**Example 119**

**8-(2,5-Dimethoxy-phenyl)-1-(2-fluoro-phenyl)-1*H*-imidazo[4,5-*c*]quinoline**

8-(2,5-Dimethoxy-phenyl)-1-(2-fluoro-phenyl)-1*H*-imidazo[4,5-*c*]quinoline is obtained as described in Example 1 using 2-fluoroaniline and 2,5-dimethoxyphenylboronic acid. 8-(2,5-Dimethoxy-phenyl)-1-(2-fluoro-phenyl)-1*H*-imidazo [4,5-*c*]quinoline, analytical HPLC: $t_{R\cdot}$= 7.38 mintues (Grad 1); $ES^+$-MS: m/$e_o$ = 400.2.

**Example 120**

**8-(3,4-Dimethoxy-phenyl)-1-(2-fluoro-phenyl)-1*H*-imidazo[4,5-*c*]quinol ine**

8-(3,4-Dimethoxy-phenyl)-1-(2-fluoro-phenyl)-1*H*-imidazo[4,5-*c*]quinoline is obtained as described in Example **1** using 2-fluoroaniline and 3,4-dimethoxyphenylboronic acid. 8-(3,4-Dimethoxy-phenyl)-1-(2-fluoro-phenyl)-1*H*-imidazo [4,5-*c*]quinoline, analytical HPLC: $t_{R-}$= 7.09 minutes (Grad 1); $ES^+$-MS: m/$e_o$ = 400.3.

**Example 121**

**1-(2-Fluoro-phenyl)-8-phenyl-1*H*-imidazo[4,5-*c*]quinoline**

1-(2-Fluoro-phenyl)-8-phenyl-1*H*-imidazo[4,5-*c*]quinoline is obtained as described in Example **1** using 2-fluoroaniline and phenylboronic acid. 1-(2-Fluoro-phenyl)-8-phenyl-1*H*-imidazo[4,5-*c*]quinoline, analytical HPLC: $t_R$= 9.48 minutes (Grad 6); $ES^+$-MS: m/$e_o$ = 340.3.

**Example 122**

**1-(2-Fluoro-phenyl)-8-(3,4,5-trimethoxy-phenyl)-1*H*-imidazo[4,5-*c*]quinoline**

1-(2-Fluoro-phenyl)-8-(3,4,5-trimethoxy-phenyl)-1*H*-imidazo[4,5-*c*]quinoline is obtained as described in Example **1** using 2-fluoroaniline and 3,4,5-trimethoxyphenylboronic acid. 1-(2-Fluoro-phenyl)-8-(3,4,5-trimethoxy-phenyl)-1*H*-imidazo[4,5-*c*]quinoline, analytical HPLC: $t_R$= 7.14 minutes (Grad 1); $ES^+$-MS: m/$e_o$ = 430.4.

**Example 123**

**8-(2,3-Dimethoxy-phenyl)-1-(2-fluoro-phenyl)-1*H*-imidazo[4,5-*c*]quinline**

8-(2,3-Dimethoxy-phenyl)-1-(2-fluoro-phenyl)-1*H*-imidazo[4,5-c]quinoline is obtained as described in Example **1** using 2-fluoroaniline and 2,3-dimethoxyphenylboronic acid. 8-(2,3-Dimethoxy-phenyl)-1-(2-fluoro-phenyl)-1*H*-imidazo [4,5-*c*]quinoline, analytical HPLC: $t_R$= 6.87 minutes (Grad 1); $ES^+$-MS: m/$e_o$ = 400.2.

**Example 124**

**1-(2-Fluoro-phenyl)-8-(2,3,4-trimethoxy-phenyl)-1*H*-imidazo[4,5-*c*]quinoline**

1-(2-Fluoro-phenyl)-8-(2,3,4-trimethoxy-phenyl)-1*H*-imidazo[4,5-*c*]quinoline is obtained as described in Example **1** using 2-fluoroaniline and 2,3,4-trimethoxyphenylboronic acid. 1-(2-Fluoro-phenyl)-8-(2,3,4-trimethoxy-phenyl)-1*H*-imidazo[4,5-*c*]quinoline, analytical HPLC: $t_R$ = 7.24 minutes (Grad 1); $ES^+$-MS: m/$e_o$ = 430.4.

**Example 125**

**1-(2-Fluoro-phenyl)-8-pyridin-4-yl-1*H*-imidazo[4,5-*c*]quinoline**

1-(2-Fluoro-phenyl)-8-pyridin-4-yl-1*H*-imidazo[4,5-*c*]quinoline is obtained as described in Example **1** using 2-fluoroaniline and pyridine-4-boronic acid. 1-(2-Fluoro-phenyl)-8-pyridin-4-yl-1*H*-imidazo[4,5-*c*]quinoline, analytical HPLC: $t_R$= 5.39 minutes (Grad 1); $ES^+$-MS: m/$e_o$ = 341.0.

**Example 126**

**1-(2-Fluoro-phenyl)-8-pyridin-3-yl-1*H*-imidazo[4,5-*c*]quinoline**

1-(2-Fluoro-phenyl)-8-pyridin-3-yl-1*H*-imidazo[4,5-*c*]quinoline is obtained as described in Example **1** using 2-fluoroaniline and pyridine-3-boronic acid. 1-(2-Fluoro-phenyl)-8-pyridin-3-yl-1*H*-imidazo[4,5-*c*]quinoline, analytical HPLC: $t_R$= 5.87 minutes (Grad 1); $ES^+$-MS: m/$e_o$ = 341.0.

**Example 127**

**4-[1-(2-Fluoro-phenyl)-1*H*-imidazo[4,5-*c*]quinolin-8-yl]-phenol**

4-[1-(2-Fluoro-phenyl)-1*H*-imidazo[4,5-*c*]quinolin-8-yl]-phenol is obtained as described in Example **1** using 2-fluoroaniline and 4-hydroxyphenylboronic acid. 4-[1-(2-Fluoro-phenyl)-1*H*-imidazo[4,5-*c*]quinolin-8-yl]-phenol, analytical HPLC: $t_R$ = 6.67 minutes (Grad 1); $ES^+$-MS: m/$e_o$ = 356.4.

**Example 128**

**1-(2-Fluoro-phenyl)-8-(3-methoxy-phenyl)-1*H*-imidazo[4,5-*c*]quinoline**

1-(2-Fluoro-phenyl)-8-(3-methoxy-phenyl)-1*H*-imidazo[4,5-*c*]quinoline is obtained as described in Example **1** using 2-fluoroaniline and 3-methoxyphenylboronic acid. 1-(2-Fluorophenyl)-8-(3-methoxy-phenyl)-1*H*-imidazo[4,5-*c*]quinoline, analytical HPLC: $t_R$= 7.55 minutes (Grad 1); $ES^+$-MS: m/$e_o$ = 370.2.

**Example 129**

**{3-[1-(2-Fluoro-phenyl)-1*H*-imidazo[4,5-*c*]quinolin-8-yl]-benzyl}-dimethyl-arnine**

19 mg (0.23 mmol) of sodium acetate and 22.5 μL (0.13 mmol) of dimethylamine are added to a solution of 42 mg (0.11 mmol) of 3-[1-(2-fluoro-phenyl)-1*H*-imidazo[4,5-*c*]quinolin-8-yl]-benzaldehyde (Example 129a) in 5 mL of MeOH/AcOH (1:1). The solution is stirred for 10 min at RT and then 62 mg (0.13 mmol) of PS-$BH_3CN$ are added. After stirring for 18 hours at RT, the suspension is filtered and evaporated to dryness. The crude product is purified by medium-pressure liquid chromatography to provide {3-[1-(2-Fluoro-phenyl)-1*H*-imidazo[4,5-*c*]quinolin-8-yl]-benzyl}-dimethyl-amine, analytical HPLC: $t_R$= 6.51 minutes (Grad 1); $ES^+$-MS: m/$e_o$ = 397.4.

**Example 129a**

**3-[1-(2-Fluoro-phenyl)-1*H*-imidazo[4,5-*c*]quinolin-8-yl]-benzaldehyde**

3-[1-(2-Fluoro-phenyl)-1*H*-imidazo[4,5-*c*]quinolin-8-yl]-benzaldehyde is obtained as described in Example **1** using 2-fluoroaniline and 3-formylphenylboronic acid. 3-[1-(2-Fluoro-phenyl)-1*H*-imidazo[4,5-*c*]quinolin-8-yl]-benzaldehyde, analytical HPLC: $t_R$= 7.48 minutes (Grad 1); $ES^+$-MS: m/$e_o$ = 368.1.

**Example 130**

**1-(2-Fluoro-phenyl)-8-[3-(4-methyl-piperazin-1-ylmethyl)-phenyl]-1*H*-imidazo[4,5-*c*]quinoline**

1-(2-Fluoro-phenyl)-8-[3-(4-methyl-piperazin-1-ylmethyl)-phenyl]-1*H*-imidazo[4,5-*c*]quinoline is obtained as described in Example **129** using 1-methylpiperazine. 1-(2-Fluoro-phenyl)-8-[3-(4-methyl-piperazin-1-ylmethyl)-phenyl]-1*H*-imidazo[4,5-*c*]quinoline, analytical HPLC: $t_R$= 6.35 minutes (Grad 1); $ES^+$-MS: m/$e_o$ = 452.0.

**Example 131**

**1-(2-Fluoro-phenyl)-8-(3-morpholin-4-ylmethyl-phenyl)-1*H*-imidazo[4,5-c]quinoline**

1-(2-Fluoro-phenyl)-8-(3-morpholin-4-ylmethyl-phenyl)-1*H*-imidazo[4,5-*c*]quinoline is obtained as described in Example **129** using morpholine. 1-(2-Fluoro-phenyl)-8-(3-morpholin-4-ylmethyl-phenyl)-1*H*-imidazo[4,5-*c*]quinoline, analytical HPLC: $t_R$= 6.60 minutes (Grad 1); $ES^+$-MS: m/$e_o$ = 439.0.

**Example 132**

**1-(2-Fluoro-phenyl)-8-(3-piperazin-1-ylmethyl-phenyl)-1*H*-imidazo[4,5-*c*]quinoline**

1-(2-Fluoro-phenyl)-8-(3-piperazin-1-ylmethyl-phenyl)-1*H*-imidazo[4,5-*c*]quinoline is obtained as described in Example **129** using piperazine. 1-(2-Fluoro-phenyl)-8-(3-piperazin-1-ylmethyl-phenyl)-1*H*-imidazo[4,5-*c*]quinoline, analytical HPLC: $t_R$ = 6.27 minutes (Grad 1); $ES^+$-MS: m/$e_o$ = 438.5.

**Example 133**

**1-(2-Fluoro-phenyl)-8-(3-pyrrolidin-1-ylmethyl-phenyl)-1*H*-imidazo[4,5-c]quinoline**

1-(2-Fluoro-phenyl)-8-(3-pyrrolidin-1-ylmethyl-phenyl)-1*H*-imidazo[4,5-*c*]quinoline is obtained as described in Example **129** using pyrrolidine. 1-(2-Fluoro-phenyl)-8-(3-pyrrolidin-1-ylmethyl-phenyl)-1*H*-imidazo[4,5-*c*]quinoline, analytical HPLC: $t_R$= 6.68 minutes (Grad 1); $ES^+$-MS: m/$e_o$ = 423.0.

**Example 134**

**1-Phenyl-8-(3-piperazin-1-yl-phenyl)-1*H*-imidazo[4,5-*c*]quinoline**

75 mg (0.22 mmol) of 8-(3-fluoro-phenyl)-1-phenyl-1*H*-imidazo[4,5-*c*]quinoline (Example 134a) and 0.38 mL of piperazine are added to 1.7 mL of NMP. The solution is heated for 5 hours at 310°C using a Woodsches-metal bath. After this time, the crude compound is purified by medium-pressure liquid chromatography to provide 1-phenyl-8-(3-piperazin-1-yl-phenyl)-1*H*-imidazo[4,5-*c*]quinoline, analytical HPLC: $t_R$= 1.53 minutes (Grad 7); $ES^+$-MS: m/$e_o$ = 406.5.

**Example 134a**

**8-(3-Fluoro-phenyl)-1-phanyl-1*H*-imidazo[4,5-*c*]quinoline**

8-(3-Fluoro-phenyl)-1-phenyl-1*H*-imidazo[4,5-*c*]quinoline is obtained as described in Example **1** using aniline and 3-fluorophenylboronic acid. 8-(3-Fluoro-phenyl)-1-phenyl-1*H*-imidazo[4,5-*c*]quinoline, analytical HPLC: $t_R$= 1.63 minutes (Grad 7); $ES^+$-MS: m/$e_o$ = 340.1.

**Example 135**

**8-[3-(4-Methyl-piperazin-1-yl)-phenyl]-1-phenyl-1*H*-imidazo[4,5-*c*]quinoline**

8-[3-(4-Methyl-piperazin-1-yl)-phenyl]-1-phenyl-1*H*-imidazo[4,5-*c*]quinoline is obtained as described in Example **134** using *N*-methylpiperazine. 8-[3-(4-Methyl-piperazin-1-yl)-phenyl]-1-phenyl-1 *H*-imidazo[4,5-*c*]quinoline, analytical HPLC: $t_R$ = 1.52 minutes (Grad 7); $ES^+$-MS: m/$e_o$ = 420.5.

**Example 136**

**4-{1-[4-(4-Methyl-piperazin-1-yl)-phenyl]-1*H*-imidazo[4,5-*c*]quinolin-8-yl}-phenol**

4-{1-[4-(4-Methyl-piperazin-1-yl)-phenyl]-1*H*-imidazo[4,5-*c*]quinolin-8-yl}-phenol is obtained as described in Example **1** using 8-bromo-1-[4-(4-methyl-piperazin-1-yl)-phenyl]-1*H*-imidazo[4,5-*c*]quinoline (Example **136a**) and 4-hydroxyphenylboronic acid.

**Example 136a**

**8-Bromo-1-[4-(4-methyl-piperazin-1-yl)-phenyl]-1*H*-imidazo[4,5-*c*]quinoline**

1.2 g (3.5 mmol) of 8-bromo-1-(4-fluoro-phenyl)-1*H*-imidazo[4,5-*c*]quinoline (Example **136b**) are dissolved in 20 mL of DMSO, and 1.45 g (10.5 mmol) of potassium carbonate and 7.7 mL (69.7 mmol) of *N*-methylpyperazine are added. The suspension is stirred for 2 days at 120°C and then added to 100 mL of ethyl acetate. After washing with water and brine, the organic solution is evaporated to dryness and the crude compound is purified by medium-pressure liquid chromatography to provide 8-Bromo-1-[4-(4-methyl-piperazin-1-yl)-phenyl]-1*H*-imidazo[4,5-*c*]quinoline.

**Example 136b**

**8-bromo-1-(4-fluoro-phenyl)-1*H*-imidazo[4,5-*c*]quinoline**

8-bromo-1-(4-fluoro-phenyl)-1*H*-imidazo[4,5-*c*]quinoline is obtained as described in Example **1** using 4-fluoroaniline. 8-bromo-1-(4-fluoro-phenyl)-1*H*-imidazo[4,5-*c*]quinoline, analytical HPLC: $t_R$= 7.21 minutes (Grad 1); $ES^+$-MS: $m/e_o$ = 342.1, 344.1.

**Example 137**

**1-[4-(4-Methyl-piperazin-1-yl)-phenyl]-8-phenyl-1*H*-imidazo[4,5-c]quinoline**

1-[4-(4-Methyl-piperazin-1-yl)-phenyl]-8-phenyl-1*H*-imidazo[4,5-*c*]quinoline is obtained as described in Example **136** using phenylboronic acid. 1-[4-(4-Methyl-piperazin-1-yl)-phenyl]-8-phenyl-1*H*-imidazo[4,5-*c*]quinoline, analytical HPLC: $t_R$= 6.10 minutes (Grad 1); $ES^+$-MS: $m/e_o$ = 420.4.

**Example 138**

**[4-(8-Pyridin-4-yl-imidazo[4,5-*c*]quinolin-1-yl)-phenyl]-acetonitrile**

[4-(8-Pyridin-4-yl-imidazo[4,5-*c*]quinolin-1-yl)-phenyl]-acetonitrile is obtained as described in Example **1** using (4-amino-phenyl)-acetonitrile and pyridine-4-boronic acid. [4-(8-Pyridin-4-yl-imidazo[4,5-*c*]quinolin-1-yl)-phenyl]-acetonitrile, analytical HPLC: $t_R$= 6.34 minutes (Grad 1); $ES^+$-MS: $m/e_o$ = 363.3.

**Example 139**

**1,8-Diphenyl-1 *H*-imidazo[4,5-*c*]quinoline**

1,8-Diphenyl-1*H*-imidazo[4,5-*c*]quinoline is obtained as described in Example **1** using aniline and phenylboronic acid. 1,8-Diphenyl-1*H*-imidazo[4,5-*c*]quinoline, analytical HPLC: $t_R$ = 9.64 minutes (Grad 1); $ES^+$-MS: $m/e_o$ = 322.3.

In the following Examples 140-143 providing activity determinations of compounds of the preceding examples, the following letters are intended to symbolize the following $IC_{50}$ values (only examples with concrete measurement results are represented):

| Letter | $IC_{50}$ Range Class |
|---|---|
| A | ≤0.5 μM |
| B | > 0.5 μM up to 1 μM |
| C | >1 μM up to 2 μM |

**Example 140**

**Inhibition of RET by compounds of the present invention**

Using the testing method described above, with the following test compounds of formula (I), the following $IC_{50}$ values for inhibition of RET are obtained:

| Compound of Example | $IC_{50}$ Range Class |
|---|---|
| 119 | B |
| 125 | B/A |
| 127 | A |
| 129 | A |
| 134 | B |

## Example 141

**inhibition of ALK by compounds of the present invention**

Using the test system described above, with the following test compounds of formula (I), the following $IC_{50}$ values for inhibition of ALK are obtained:

| Compound of Example | $IC_{50}$ Range Class |
|---|---|
| 18 | B/A |
| 11 | A |
| 48 | A |

## Example 142

**Inhibition of PKB by compounds of the present invention**

Using the test system described above, with the following test compounds of formula (I), the following $IC_{50}$ values for inhibition of PKB are obtained:

| Compound of Example | $IC_{50}$ Range Class |
|---|---|
| 61 | A |
| 64 | A |
| 74 | A |
| 90 | A |

## Example 143

**Inhibition of S6K1 by compounds of the present invention**

Using the testing method described above, with the following test compounds of formula (I), the following $IC_{50}$ values for inhibition of S6K1 are obtained:

| Compound of Example | $IC_{50}$ Range Class |
|---|---|
| 11 | A |
| 18 | A |
| 22 | A |
| 30 | A |
| 83 | A |

**Example 144**

**Tablets 1 comprising compounds of the formula (I)**

Tablets comprising, as active ingredient, 50 mg of any one of the compounds of formula (I) mentioned in the preceding Examples 1-139 of the following composition are prepared using routine methods:

| Composition | |
|---|---|
| Active Ingredient | 50 mg |
| Wheat starch | 60 mg |
| Lactose | 50 mg |
| Colloidal silica | 5 mg |
| Talcum | 9 mg |
| Magnesium stearate | 1 mg |
| | **175 mg** |

Manufacture: The active ingredient is combined with part of the wheat starch, the lactose and the colloidal silica and the mixture pressed through a sieve. A further part of the wheat starch is mixed with the 5-fold amount of water on a water bath to form a paste and the mixture made first is kneaded with this paste until a weakly plastic mass is formed.

The dry granules are pressed through a sieve having a mesh size of 3 mm, mixed with a pre-sieved mixture (1 mm sieve) of the remaining com starch, magnesium stearate and talcum and compressed to form slightly biconvex tablets.

**Example 145**

**Tablets 2 comprising compounds of the formula (I)**

Tablets, comprising, as active ingredient, 100 mg of any one of the compounds of formula (I) of Examples **1-139** are prepared with the following composition, following standard procedures:

| Composition | |
|---|---|
| Active Ingredient | 100 mg |
| Crystalline lactose | 240 mg |
| Avicel | 80 mg |
| PVPPXL | 20 mg |
| Aerosil | 2 mg |
| Magnesium stearate | 5 mg |
| | **447 mg** |

Manufacture: The active ingredient is mixed with the carrier materials and compressed by means of a tabletting machine (Korsch EKO, Stempeldurchmesser 10 mm).

**Example 146**

**Capsules**

Capsules, comprising, as active ingredient, 100 mg of any one of the compounds of formula (I), given in Examples **1-139**, of the following composition are prepared according to standard procedures:

| Composition | |
|---|---|
| Active Ingredient | 100 mg |
| Avicel | 200 mg |

(continued)

| Composition | |
|---|---|
| PVPPXL | 15 mg |
| Aerosil | 2 mg |
| Magnesium stearate | 1.5 mg |
| | **318.5 mg** |

Manufacturing is done by mixing the components and filling them into hard gelatine capsules, size 1.

**Claims**

**1.** A compound of formula (I):

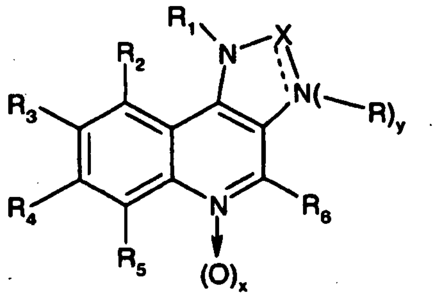

(I)

,

wherein
each of x and y is, independently of the other, 0 or 1;

$R_1$ is substituted or unsubstituted aryl or heteroaryl, especially phenyl, which is substituted with up to 4 substituents, preferably up to 3 substituents, where the substituents are the same or different and are independently selected from halo, e.g., F or Cl; lower alkyl which may be unsubstituted or substituted with halo, especially methyl, ethyl, propyl or trifluoromethyl; cyano; cyano-lower alkyl, e.g., cyanomethyl, cyanoethyl or cyanopropyl; lower alkoxy; amino; amino-lower alkyl; amino-lower alkoxy; amino-lower alkyl sulfanyl; or thiol-lower alkyl, wherein the amino group can be mono- or di-substituted, e.g., $-(C_1-C_7)_mNR_8R_9$ or $-O-(C_1-C_7)_mNR_8R_9$, wherein

m is 0 or 1; and
$R_8$ and $R_9$ can be the same or different and are independently H; lower alkyl, e.g., methyl, ethyl or propyl; lower cycloalkyl, e.g., cyclopropyl, or
$R_8$ and $R_9$, together with the N atom, form a 3- to 8-membered heterocyclic ring containing 1-4 nitrogen, oxygen or sulfur atoms, e.g., azetidinyl, pyrrolidinyl, piperidino, morpholinyl, imidazolinyl, imidazolinyl-ethyl, piperazinyl or lower alkyl-piperazinyl; amino-carbonyl-lower alkyl, e.g., $R_8R_9$-N-C(O)-$CH_2$-, wherein $R_8$ and $R_9$ are as defined above; heterocyclyl; heterocyclyl-lower alkyl; heterocyclyl-lower alkoxy; or heterocyclyl-lower alkanesulfanyl, wherein the heterocyclyl is a 3- to 8-membered heterocyclic ring containing 1-4 nitrogen, oxygen or sulfur atoms, e.g., imidazolyl, imidazolinyl, pyrrolidinyl, morpholinyl, azetidinyl, pyridyl, piperidino, piperidyl, piperazinyl or lower alkyl-piperazinyl; substituted or unsubstituted amide; amide-lower alkyl, e.g., $-CH_2-CH(NH_2)-C(O)-NH_2$), wherein alkyl may be linear or cyclic, e.g., cyclopropylene; and the alkyl in any of the substituents above may optionally be substituted with $-NR_8R_9$, wherein $R_8$ and $R_9$ are as defined above;

X is C=O or C=S, with the proviso that then the dashed line bonding X to N is absent, so that X is bound to the adjacent N via a single bond and with the proviso that then y is 1 and R is hydrogen or an organic moiety that can be bound to nitrogen, or
X is ($CR_7$), wherein $R_7$ is hydrogen or an organic moiety, such as $C_1$-$C_7$lower alkyl; amino; or amino alkyl,

wherein the alkyl may be unsubstituted or substituted with halo, e.g., methyl, ethyl, propyl or trifluoromethyl; lower alkoxy, e.g.; methoxy; or cycloalkyl, e.g., cyclopropyl; with the proviso that then the dashed line bonding X to N is a bond, so that X is bound to the adjacent N via a double bond; and with the proviso that then y is zero, or y is 1 and then -R is →O;

$R_2$ is hydrogen;

$R_3$ is unsubstituted or substituted $C_5$-$C_{14}$heterocyclyl, e.g., thienyl, benzo[1,3]dioxolo, indolyl, benzofuranyl or pyridiyl; unsubstituted or substituted $C_5$-$C_{14}$aryl, e.g., phenyl or phenyl substituted with up to 4 substituents, preferably up to 3 substituents, which are the same or different and are selected from halo, e.g., Cl or F; hydroxy; $C_1$-$C_4$lower alkoxy, e.g., methoxy; lower alkyl, e.g., methyl; or $-(C_1-C_4)_mNR_8R_9$,

wherein

m is 0 or 1; and

$R_8$ and $R_9$, are as defined above, e.g., piperazinyl, methylpiperazinyl, morpholinyl or pyrrolidinyl;

$R_4$ is hydrogen or halo, e.g., fluoro.or chloro;

$R_5$ is hydrogen; and

$R_6$ is hydrogen, amino, amino alkyl or alkylamido, e.g., methylamido $-NHC(O)-CH_3$), with the proviso that $R_3$ cannot be unsubstituted phenyl unless $R_1$ is phenyl substituted with an heterocyclic ring;

wherein the prefix 'lower' denotes a radical having 1 carbon atom up to and including a maximum of 7 carbon atoms or a pharmaceutically acceptable salt thereof.

**2.** A compound according to Claim 1,
wherein
each of x and y is, independently of the other, 0 or 1;

$R_1$ is substituted or unsubstituted phenyl where the phenyl is substituted with up to 4 substituents, preferably up to 3 substituents, where the substituents are the same or different and are independently selected from halo, e.g., F or Cl; $C_1$-$C_7$lower alkyl, which may be unsubstituted or substituted with halo, especially methyl, ethyl, propyl or trifluoromethyl; cyano; cyano-lower alkyl, e.g., cyanomethyl, cyanoethyl or cyanopropyl; amino; amino-lower alkyl, wherein the amino group can be mono- or di-substituted, e.g., $-(C_1-C_7)_mNR_8R_9$ or $-O-(C_1-C_7)_mNR_8R_9$,

wherein

m is 0 or 1; and

$R_8$ and $R_9$ can be the same or different and are independently H; lower alkyl, e.g., methyl, ethyl or propyl; lower cycloalkyl, e.g., cyclopropyl, or

$R_8$ and $R_9$, together with the N atom, form a 3- to 8-membered heterocyclic ring containing 1-4 nitrogen oxygen or sulfur atoms, e.g., imidazolinyl, imidazolinyl-ethyl, piperazinyl or lower alkyl-piperazinyl amino-carbonyl-lower alkyl, e.g., $R_8R_9$-N-C(O)-$CH_2$-, wherein $R_8$ and $R_9$ are as defined above; heterocyclyl; heterocyclyl-lower alkyl; heterocyclyl-lower alkoxy; or heterocyclyl-lower alkanesulfanyl, wherein the heterocyclyl is a 3- to 8-membered heterocyclic ring containing 1-4 nitrogen, oxygen or sulfur atoms, e.g., imidazolinyl, imidazolinyl, imidazolinyl-ethyl, piperazinyl or lower alkyl-piperazinyl; substituted or unsubstituted amide; amide-lower alkyl, e.g., $-CH_2-CH(NH_2)-C(O)-NH_2$, wherein alkyl may be linear or cyclic, e.g., cyclopropylene; and the alkyl in any of the substituents above may optionally be substituted with $-NR_8R_9$, wherein $R_8$ and $R_9$ are as defined above;

X is ($CR_7$), wherein $R_7$ is hydrogen; lower alkyl, e.g., methyl or ethyl; amino; or amino alkyl, with the proviso that then the dashed line bonding X to N is a bond, so that X is bound to the adjacent N via a double bond, and with the proviso that then y is zero, or y is 1 and then -R is →O;

$R_2$ is hydrogen;

$R_3$ is unsubstituted or substituted $C_5$-$C_{14}$heterocyclyl, e.g., thienyl, benzo[1,3]dioxolo, indolyl, benzofuranyl or pyridiyl; unsubstituted or substituted $C_5$-$C_{14}$aryl, e.g., phenyl or phenyl substituted with up to 4 substituents, preferably up to 3 substituents, which are the same or different and are selected from halo, e.g., Cl or F; hydroxy; $C_1$-$C_4$lower alkoxy, e.g., methoxy; lower alkyl, e.g., methyl; or $-(C_1-C_4)_mNR_8R_9$,

wherein

m is 0 or 1; and

$R_8$ and $R_9$ are as defined above, e.g., piperazinyl, methylpiperazinyl, morpholinyl or pyrrolidinyl;

$R_4$ is hydrogen or halo, (e.g. fluoro or chloro);
$R_5$ is hydrogen; and
$R_6$ is hydrogen, with the proviso that $R_3$ cannot be unsubstituted phenyl unless $R_1$ is phenyl substituted with an heterocyclic ring;

or a pharmaceutically acceptable salt thereof.

**3.** A pharmaceutical composition comprising a compound of formula (I), according to any of Claims 1, 2 or 7, or a pharmaceutically acceptable salt thereof and a pharmaceutically acceptable carrier.

**4.** Use of a compound of formula (I), according to any one of Claims 1, 2 or 7, or a pharmaceutically acceptable salt thereof, for the preparation of a pharmaceutical composition for use in the treatment of a protein kinase dependent disease.

**5.** The use according to Claim 4, wherein the protein kinase dependent disease is preferably one depending on PKB, ALK, S6K1 or RET and (especially aberrantly highly-expressed or activated) PKB, ALK, S6K1 or RET-dependent disease or disease dependent on the activation of the PKB, ALK, S6K1 or RET pathways, or a disease dependent on any two or more of the kinases just mentioned.

**6.** The use according to Claim 4, wherein the disease to be treated is a proliferative disease, preferably a benign or especially malignant tumor, more preferably carcinoma of the brain, kidney, liver, adrenal gland, bladder, breast, stomach, gastric tumors, ovaries, colon, rectum, prostate, pancreas, lung, vagina, thyroid, sarcoma, glioblastomas, multiple myeloma, gastrointestinal cancer, colon carcinoma, colorectal adenoma, tumor of the neck and head, an epidermal hyperproliferation, psoriasis, prostate hyperplasia, a neoplasia, especially of epithelial character, mammary carcinoma, leukemia, metabolic diseases, type II diabetes, obesity, hyperlipidemia and atherosclerosis.

**7.** A compound according to Claim 1, selected from the group consisting of:

[4-(8-Benzo[1,3]dioxol-5-yl-imidazo[4,5-*c*]quinolin-1-yl)-phenyl]-acetonitrite;
[4-(8-Thiophen-2-yl-imidazo[4,5-*c*]quinolin-1-yl)-phenyl]-acetonitrile;
[4-(8-Benzofuran-2-yl-imidazo[4,5-*c*]quinolin-1-yl)-phenyl]-acetonitrile;
2-[4-(8-Benzo[1,3]dioxol-5-yl-imidazo[4,5-*c*]quinolin-1-yl)-phenyl]-ethylamine;
2-[4-(8-Thiophen-2-yl-imidazo[4,5-*c*]quinolin-1-yl)-phenyl]-ethylamine;
2-[4-(8-Benzofuran-2-yl-imidazo[4,5-*c*]quinolin-1-yl)-phenyl]-ethylamine;
[3-Chloro-4-(8-thiophen-2-yl-imidazo[4,5-*c*]quinolin-1-yl)-phenyl]-acetonitrile;
(4-Amino-3-chloro-phenyl)-acetonitrile;
{3-Chloro-4-[8-(1*H*-indol-5-yl)-imidazo[4,5-*c*]quinolin-1-yl]-phenyl}-acetonitrile;
[3-Chloro-4-(8-thiophen-3-yl-imidazo[4,5-c]quinolin-1-yl)-phenyl]-acetonitrile;
2-[3-Chloro-4-(8-thiophen-2-yl-imidazo[4,5-*c*]quinolin-1-yl)-phenyl]-ethylamine;
2-{3-Chloro-4-[8-(1*H*-indol-5-yl)-imidazo[4,5-*c*]quinolin-1-yl]-phenyl}-ethylamine;
2-[3-Chloro-4-(8-thiophen-3-yl-imidazo[4,5-*c*]quinolin-1-yl)-phenyl]-ethylamine,
[2-Fluoro-4-(8-thiophen-2-yl-imidazo[4,5-*c*]quinolin-1-yl)-phenyl]-acetonitrile;
[4-(8-Benzofuran-2-yl-imidazo[4,5-*c*]quinolin-1-yl)-2-fluoro-phenyl]-acetonitrile.
{2-Fluoro-4-[8-(1*H*-indol-5-yl)-imidazo[4,5-*c*]quinolin-1-yl]-phenyl}-acetonitrile;
2-[2-Fluoro-4-(8-thiophen-2-yl-imidazo[4,5-*c*]quinolin-1-yl)-phenyl]-ethylamine;
2-[4-(8-Benzofuran-2-yl-imidazo[4,5-*c*]quinolin-1-yl)-2-fluoro-phenyl]-ethylamine;
2-(2-Fluoro-4-[8-(1*H*-indol-5-yl)-imidazo[4.5-*c*]quinolin-1-yl]-phenyl)-ethylamine;
[3-Methyl-4-(8-thiophen-3-yl-imidazo[4,5-*c*]quinolin-1-yl)-phenyl]-acetonitrile;
{4-[8-(1*H*-indol-5-yl)-imidazo[4,5-*c*]quinolin-1-yl]-3-methyl-phenyl}-acetonitrile;
2-[3-Methyl-4-(8-thiophen-3-yl-imidazo[4,5-*c*]quinolin-1-yl)-phenyl]-ethylamine;
2-{4-[8-(1*H*-Indol-5-yl)-imidazo[4,5-*c*]quinolin-1-yl]-3-methyl-phenyl}-ethylamine;
(*R*)-2-Amino-3-[4-(8-thiophen-2-yl-imidazo[4,5-*c*]quinolin-1-yl)-phenyl]-propionamide;
(*R*)-2-Amino-3-[4-(8-benzo[*b*]thiophen-2-yl-imidazo[4,5-*c*]quinolin-1-yl)-phenyl]-propionamide;
[3,5-Dichloro-4-(8-thiophen-2-yl-imidazo[4,5-*c*]quinolin-1-yl)-phenyl]-acetonitrile;
[3,5-Dichloro-4-(8-thiophen-3-yl-imidazo[4,5-*c*]quinolin-1-yl)-phenyl]-acetonitrile;
2-[3,5-Dichloro-4-(8-thiophen-2-yl-imidazo[4,5-*c*]quinolin-1-yl)-phenyl]-ethylamine;

2-[3,5-Dichloro-4-(8-thiophen-3-yl-imidazo[4,5-*c*]quinolin-1-yl)-phenyl]-ethylamine;
{4-[8-(4-Hydroxy-phenyl)-imidazo[4,5-*c*]quinolin-1-yl]-3-trifluoromethyl-phenyl}-acetonitrile;
4-{1-[4-(2-Amino-ethyl)-2-trifluoromethyl-phenyl]-1*H*-bimidazo[4,5-*c*]quinolin-8-yl}-phenol;
3-[4-(8-Benzo[1,3]dioxol-5-yl-imidazo[4,5-*c*]quinolin-1-yl)-phenyl]-propionitrile;
3-[4-(8-Thiophen-2-yl-imidazo[4,5-*c*]quinolin-1-yl)-phenyl]-propionitrile;
3-[4-(8-Benzo[*b*]thiophen-2-yl-imidazo[4,5-*c*]quinolin-1-yl)-phenyl]-propionitrile;
3-[4-(8-Thiophen-3-yl-imidazo[4,5-*c*]quinolin-1-yl)-phenyl]-propionitrile;
3-[4-(8-Benzofuran-2-yl-imidazo[4,5-*c*]quinolin-1-yl)-phenyl]-propionitrile;
3-[4-(8-Benzo[1,3]dioxol-5-yl-imidazo[4.5-*c*]quinolin-1-yl)-phenyl]-propylamine;
3-[4-(8-Thiophen-2-yl-imidazo[4,5-*c*]quinolin-1-yl)-phenyl]-propylamine;
3-[4-(8-Benzo[*b*]thiophen-2-yl-imidazo[4,5-*c*]quinolin-1-yl)-phenyl]-propylamine;
3-[4-(8-Thiophen-3-yl-imidazo[4,5-*c*]quinolin-1-yl)-phenyl]-propylamine;
3-[4-(8-Benzofuran-2-yl-imidazo[4,5-*c*]quinolin-1-yl)-phenyl]-propylamine;
3-[4-(8-Benzo[1,3]dioxol-5-yl-imidazo[4,5-*c*]quinolin-1-yl)-3-chloro-phenyl]-propionitrile;
3-[3-Chloro-4-(8-thiophen-2-yl-imidazo[4,5-*c*]quinollin-1-yl)-phenyl]-propionitrile,
3-[3-Chloro-4-(8-thiophen-3-yl-imidazo[4,5-*c*]quinolin-1-yl)-phenyl]-propionitrile;
3-{3-Chloro-4-[8-(1*H*-indol-5-yl)-imidazo[4,5-*c*]quinolin-1-yl]-phenyl-propionitrile;
3-[4-(8-Benzo[1,3]dioxol-5-yl-imidazo[4,5-*c*]quinolin-1-yl)-3-chloro-phenyl]-propylamine;
3-[3-Chloro-4-(8-thiophen-2-yl-imidazo[4,5-*c*]quinolin-1-yl)-phenyl]-propylamine;
3-[3-Chloro-4-(8-thiophen-3-yl-imidazo[4,5-*c*]quinolin-1-yl)-phenyl]-propylamine;
3-{3-Chloro-4-[8-(1*H*-indol-5-yl)-imidazo[4,5-*c*]quinolin-1-yl]-phenyl}-propylamine;
8-Benzo[1,3]dioxol-5-yl-1-{4-[2-(4,5-dihydro-1*H*-imidazol-2-yl)-ethyl]-phenyl}-1*H*-imidazo[4,5-c]quinoline;
[3-Chloro-4-(2-methyl-8-thiophen-2-yl-imidazo[4,5-*c*]quinolin-1-yl)-phenyl]-acetonitrile;
[3-Chloro-4-(2-methyl-8-thiophen-3-yl-imidazo[4,5-*c*]quinolin-1-yl)-phenyl]-acetonitrile;
2-[3-Chloro-4-(2-methyl-8-thiophen-2-yl-imidazo[4,5-*c*]quinolin-1-yl)-phenyl]-ethylamine;
2-[3-Chloro-4-(2-methyl-8-thiophen-3-yl-imidazo[4,5-*c*]quinolin-1-yl)-phenyl]-ethylamine;
(4-(8-Benzo[1,3]dioxol-5-yl-7-fluoro-imidazo[4,5-*c*]quinolin-1-yl)-phenyl)-acetonitrile;
[4-(7-Fluoro-8-thiophen-2-yl-imidazo[4,5-*c*]quinolin-1-yl)-phenyl]-acetonitrile;
[4-(7-Fluoro-8-thiophen-3-yl-imidazo[4,5-*c*]quinolin-1-yl)-phenyl]-acetonitrile;
[4-(8-Benzofuran-2-yl-7-fluoro-imidazo[4,5-*c*]quinolin-1-yl)-phenyl]-acetonitrile;
{4-[7-Fluoro-8-(1*H*-indol-5-yl)-imidazo[4,5-*c*]quinolin-1-yl]-phenyl}-acetonitrile;
[4-(8-Benzo[*b*]thiophen-2-yl-7-fluoro-imidazo[4,5-*c*]quinolin-1-yl)-phenyl]-acetonitrile;
2-[4-(8-Benzo[1,3]dioxol-5-yl-7-fluoro-imidazo[4,5-*c*]quinolin-1-yl)-phenyl]-ethylamine;
2-[4-(7-Fluoro-8-thiophen-2-yl-imidazo[4,5-*c*]quinolin-1-yl)-phenyl]-ethylamine;
2-[4-(7-Fluoro-8-thiophen-3-yl-imidazo[4,5-*c*]quinolin-1-yl)-phenyl]-ethylamine;
2-[4-(8-Benzofuran-2-yl-7-fluoro-imidazo[4,5-*c*]quinolin-1-yl)-phenyl]-ethylamine;
2-{4-[7-Fluoro-8-(1*H*-indol-5-yl)-imidazo[4,5-*c*]quinolin-1-yl]-phenyl}-ethylamine;
2-[4-(8-Benzo[*b*]thiophen-2-yl-7-fluoro-imidazo[4,5-*c*]quinolin-1-yl)-phenyl]-ethylamine;
3-[4-(8-Benzo[1,3]dioxol-5-yl-7-fluoro-imidazo[4,5-*c*]quinolin-1-yl)-phenyl]-propionitrile;
3-[4-(7-Fluoro-8-thiophen-2-yl-imidazo[4,5-*c*]quinolin-1-yl)-phenyl]-propionitrile;
3-[4-(7-Fluoro-8-thiophen-3-yl-imidazo[4,5-c]quinolin-1-yl)-phenyl]-proplonitrile;
3-[4-(8-Benzofuran-2-yl-7-fluoro-imidazo[4,5-*c*]quinolin-1-yl)-phenyl]-propionitrile;
3-[4-(8-Benzo[*b*]thiophen-2-yl-7-fluoro-imidazo[4,5-*c*]quinolio-1-yl)-phenyl]-propionitrile;
3-{4-[7-Fluoro-8(1*H*-indol-5-yl)-imidazo[4,5-*c*]quinolin-1-yl]-phenyl}-propionitrile,
3-[4-(8-Benzo[1,3]dioxol-5-yl-7-fluoro-imidazo[4,5-*c*]quinolin-1-yl)-phenyl]-propylamine;
3-[4-(7-Fluorcr-8-thiophen-2-yl-imidazo[4,5-*c*]quinolin-1-yl)-phenyl]-propylamine;
3-[4-(7-Fluoro-8-thiophen-3-yl-imidazo[4,5-*c*]quinolin-1-yl)-phonyl]-propylamine;
3-[4-(8-Benzofuran-2-yl-7-fluoro-imidazo[4,5-*c*]quinolin-1-yl)-phenyl]-propylamine;
3-[4-(8-Benzo[*b*]thiophen-2-yl-7-fluoro-imidazo[4,5-*c*]quinolin-1-yl)-phenyl]-propylamine;
3-{4-[7-Fluoro-8-(1*H*-indol-5-yl)-imidazo[4,5-*c*]quinolin-1-yl]-phenyl}-propylamine;
[3-Chloro-4-(7-fluoro-8-thiophen-2-yl-imidazo[4,5-*c*]quinolin-1-yl)-phenyl]-acetonitrile;
[3-Chloro-4-(7-fluoro-8-thlophen-3-yl-imidazo[4,5-*c*]quinolin-1-yl)-phenyl]-acetonitrile;
{3-Chloro-4-[7-fluoro-8-(1*H*-indol-5-yl)-imidazo[4,5-*c*]quinolin-1-yl]-phenyl}-acetonitrile;
2-[3-Chloro-4-(7-fluoro-8-thiophen-2-yl-imidazo[4,5-*c*]quinolin-1-yl)-phenyl]-ethylamine;
2-[3-Chloro-4-(7-fluoro-8-thiophen-3-yl-imidazo[4,5-*c*]quinolin-1-yl)-phenyl]-ethylamine;
2-{3-Chloro-4-[7-fluoro-8-(1*H*-indol-5-yl)-imidazo[4,5-*c*]quinolin-1-yl]-phenyl}-ethylamine;
[4-(7-Chloro-8-thiophen-2-yl-imidazo[4,5-*c*]quinolin-1-yl)-phenyl]-acetonitrile;
[4-(7-Chloro-8-thiophen-3-yl-imidazo[4,5-*c*]quinolin-1-yl)-phenyl]-acetonitrile;

[4-(8-Benzofuran-2-yl-7-chloro-imidazo[4,5-*c*]quinolin-1-yl)-phenyl]-acetonitrile;
{4-[7-Chloro-8-(1*H*-indol-5-yl)-imidazo[4,5-*c*]quinolin-1-yl]-phenyl}-acetonitrile;
[4-(8-Benzo[*b*]thiophen-2-yl-7-chloro-imidazo[4,5-*c*]quinolin-1-yl)-phenyl]-acetonitrile;
2-[4-(7-Chloro-8-thiophen-2-yl-imidazo[4,5-*c*]quinolin-1-yl)-phenyl]-ethylamine;
2-[4-(7-Chloro-8-thiophen-3-yl-imidazo[4,5-*c*]quinolin-1-yl)-phenyl]-ethylamine;
2-[4-(8-Benzofuran-2-yl-7-chloro-imidazo[4,5-*c*]quinolin-1-yl)-phenyl]-ethylamine;
2-{4-[7-Chloro-8-(1*H*-indol-5-yl)-imidazo[4,5-*c*]quinolin-1-yl]-phenyl}-ethylamine;
2-[4-(8-Benzo[*b*]thiophen-2-yl-7-chloro-imidazo[4,5-*c*]quinolin-1-yl)-phenyl]-ethylamine;
3-[4-(8-Benzo[1,3]dioxol-5-yl-7-chloro-imidazo[4,5-*c*]quinolin-1-yl)-phenyl]-propionitrile;
3-[4-(7-Chloro-8-thiophen-2-yl-imidazo[4,5-*c*]quinolin-1-yl)-phenyl]-propionitrile;
3-[4-(8-Benzofuran-2-yl-7-chloro-imidazo[4,5-*c*]quinolin-1-yl)-phenyl]-propionitrile;
3-{4-[7-Chloro-8-(1*H*-indol-5-yl)-imidazo[4,5-*c*]quinolin-1-yl]-phenyl}-propionitrile;
3-[4-(8-Benzo[*b*]thiophen-2-yl-7-chloro-imidazo[4,5-*c*]quinolin-1-yl)-phenyl]-propionitrile;
3-[4-(8-Benzo[1,3]dioxol-5-yl-7-chloro-imidazo[4,5-*c*]quinolin-1-yl)-phenyl]-propylamine;
3-[4-(7-Chloro-8-thiophen-2-yl-imidazo[4,5-*c*]quinolin-1-yl)-phenyl]-propylamine;
3-[4-(8-Benzofuran-2-yl-7-chloro-imidzo[4,5-*c*]quinolin-1-yl)-phenyl]-propylamine;
3-{4-[7-Chloro-8-(1*H*-indol-5-yl)-imidazo[4,5-*c*]quinolin-1-yl]-phenyl}-propylamine;
3-[4-(8-Benzo[*b*]thiophen-2-yl-7-chloro-imidazo[4,5-*c*]quinolin-1-yl)-phenyl]-propylamine;
[3-Chloro-4-(7-chloro-8-thiophen-2-yl-imidazo[4,5-*c*]quinolin-1-yl)-phenyl]-acetonitrile;
[3-Chloro-4-(7-chloro-8-thiophen-3-yl-imidazo[4,5-*c*]quinolin-1-yl)-phenyl]-acetonitrile;
2-[3-Chloro-4-(7-chloro-8-thiophen-2-yl-imidazo[4,5-*c*]quinolin-1-yl)-phenyl]-ethylamine;
2-[3-Chloro-4-(7-chloro-8-thiophen-3-yl-imidazo[4,5-*c*]quinolin-1-yl)-phenyl]-ethylamine;
3-[3-Chloro-4-(7-chloro-8-thiophen-2-yl-imidazo[4,5-*c*]quinolin-1-yl)-phenyl]-propionitrile;
3-[3-Chloro-4-(7-chloro-8-thiophen-3-yl-imidazo[4,5-*c*]quinolin-1-yl)-phenyl]-propionitrile;
3-{3-Chloro-4-[7-chloro-8-(1*H*-indol-5-yl)-imidazo[4,5-*c*]quinolin-1-yl]-phenyl}-propionitrile;
3-[3-Chloro-4-(7-chloro-8-thiophen-2-yl-imidazo[4,5-*c*]quinolin-1-yl)-phenyl]-propylamine;
3-[3-Chloro-4-(7-chloro-8-thiophen-3-yl-imidazo[4,5-*c*]quinolin-1-yl)-phenyl]-propylamine;
3-(3-Chloro-4-[7-chloro-8-(1*H*-indot-5-yl)-imidazo[4,5-*c*]quinolin-1-yl]-phenyl}-propylamine;
3-[4-(2-Amino-7-chloro-8-thiophen-3-yl-imidazo[4,5-*c*]quinolin-1-yl)-phenyl]-propionitrile;
3-[4-(2-Amino-8-bromo-7-chloro-imidazo[4,5-*c*]quinolin-1-yl)-phenyl]-propionitrile;
3-[4-(3-Amino-6-bromo-7-chloro-quinolin-4-ylamino)-phenyl]-propionitrile;
3-[4-(2-Amino-8-benzofuran-2-yl-7-chloro-imidazo[4,5-*c*]quinolin-1-yl)-phenyl]-propionitrile;
1-[4-(3-Amino-propyl)-phenyl]-7-chloro-8-thiophen-3-yl-1*H*-himidazo[4,5-*c*]quinolin-2-ylamine;
1-[4-(3-Amino-propyl)-phenyl]-8-benzoturan-2-yl-7-chloro-1*H*-imidazo[4,5-*c*]quinolin-2-ylamine;
8-(2,4-Dimethoxy-phenyl)-1-(2-fluoro-phenyl)-1 *H*-imidazo[4,5-*c*]quinoline;
8-(2,5-Dimethoxy-phenyl)-1-(2-fluoro-phenyl)-1 *H*-imidazo[4,5-*c*]quinoline;
8-(3,4-Dimethoxy-phenyl)-1-(2-fluoro-phenyl)-1 *H*-imidazo[4,5-*c*]quinoline;
1-(2-Fluoro-phenyl)-8-(3,4,5-trimethoxy-phenyl)-1 *H*-imidazo[4,5-*c*]quinoline;
8-(2,3-Dimethoxy-phenyl)-1-(2-fluoro-phenyl)-1*H*-imidazo[4,5-*c*]quinoline;
1-(2-Fluoro-phenyl)-8-(2,3,4-trimethoxy-phenyl)-1 *H*-imidazo[4,5-*c*]quinoline;
1-(2-Fluoro-phenyl)-8-pyridin-4-yl-1*H*-imidazo[4,5-*c*]quinoline;
1-(2-Fluoro-phenyl)-8-pyridin-3-yl-1*H*-imidazo[4,5-*c*]quinoline;
1-(2-Fluoro-phenyl)-8-(3-methoxy-phenyl)-1*H*-imidazo[4,5-*c*]quinoline;
{3-[1-(2-Fluoro-phenyl)-1*H*-imidazo[4,5-*c*]quinolin-8-yl]-benzyl}-dimethyl-amine;
1-(2-Fluoro-phenyl)-8-[3-(4-methyl-piperazin-1-ylmethyl)-phenyl]-1*H*-imidazo[4,5-*c*]quinoline;
1-(2-Fluoro-phenyl)-8-(3-morpholin-4-ylmethyl-phenyl)-1*H*-imidazo[4,5-*c*]quinoline;
1-(2-Fluoro-phenyl)-8-(3-piperazin-1-ylmethyl-phenyl)-1*H*-imidazo[4,5-*c*]quinoline;
1-(2-Fluoro-phenyl)-8-(3-pyrrolidin-1-ylmethyl-phenyl)-1*H*-imidazo[4,5-*c*]quinoline;
1-Phenyl-8-(3-piperazin-1-yl-phenyl)-1*H*-imidazo[4,5-*c*]quinoline;
8-(3-Fluoro-phenyl)-1-phenyt-1*H*-imidazo[4,5-*c*]quinoline;
8-[3-(4-Methyl-piperazin-1-yl)-phenyl]-1-phenyl-1*H*-imidazo[4,5-*c*]quinoline;
4-{1-[4-(4-Methyl-piperazin-1-yl)-phenyl]-1*H*-imidazo[4,5-*c*]quinolin-8-yl}-phenol;
1-[4-(4-Methyl-piperazin-1-yl)-phenyl]-8-phenyl-1*H*-imidazo[4,5-*c*]quinoline; and
[4-(8-Pyridin-4-yl-imidazo[4,5-c]quinolin-1-yl)-phenyl]-acetonitrile.

**Patentansprüche**

1. Verbindung der Formel (I)

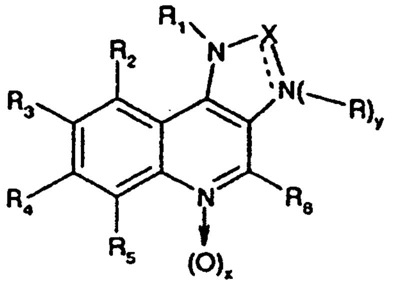

(I)

worin

x und y jeweils unabhängig voneinander für 0 oder 1 stehen,
$R_1$ steht für substituiertes oder unsubstituiertes Aryl oder Heteroaryl, besonders für Phenyl, das mit bis zu 4 Substituenten, vorzugsweise bis zu 3 Substituenten, substituiert ist, worin die Substituenten gleich oder verschieden sind und unabhängig ausgewählt sind aus Halogen, beispielsweise F oder Cl, Niederalkyl, das unsubstituiert oder substituiert sein kann mit Halogen, speziell Methyl, Ethyl, Propyl oder Trifluormethyl, Cyano, Cyanoniederalkyl, beispielsweise Cyanomethyl, Cyanoethyl oder Cyanopropyl, Niederalkoxy, Amino, Aminoniederalkyl, Aminoniederalkoxy, Aminoniederalkylsulfanyl oder Thiolniederalkyl, worin die Aminogruppe monosubstituiert oder disubstituiert sein kann, beispielsweise mit $-(C_1-C_7)_mNR_8R_9$ oder $-O-(C_1-C_7)_mNR_8R_9$.
worin

m für 0 oder 1 steht, und
$R_8$ und $R_9$ gleich oder verschieden sein können und unabhängig für H, Niederalkyl, beispielsweise Methyl, Ethyl oder Propyl oder Niedercycloalkyl, beispielsweise Cyclopropyl, stehen, oder
$R_8$ und $R_9$ zusammen mit dem Stickstoffatom einen 3- bis 8-gliedrigen heterocyclischen Ring mit 1 bis 4 Stickstoffatomen, Sauerstoffatomen oder Schwefelatomen bilden, beispielsweise Azetidinyl, Pyrrolidinyl, Piperidino, Morpholinyl, Imidazolinyl, Imidazolinylethyl, Piperazinyl oder Niederalkylpiperazinyl, Aminocarbonylniederalkyl, beispielsweise $R_8R_9-N-C(O)-CH_2-$, worin $R_8$ und $R_9$ wie oben definiert sind, Heterocyclyl, Heterocyclylniederalkyl, Heterocyclylniederalkoxy oder Heterocyclylniederalkansulfanyl, worin das Heterocyclyl ein 3- bis 8-gliedriger heterocyclischer Ring mit 1 bis 4 Stickstoffatomen, Sauerstoffatomen oder Schwefelatomen ist, beispielsweise Imidazolyl, Imidazolinyl, Pyrrolidinyl, Morpholinyl, Azetidinyl, Pyridyl, Piperidino, Piperidyl, Piperazinyl oder Niederalkylpiperazinyl, substituiertes oder unsubstituiertes Amid, Amidniederalkyl, beispielsweise $-CH_2-CH(NH_2)-C(O)-NH_2$, worin das Alkyl linear oder cyclisch sein kann, beispielsweise Cyclopropylen, und worin das Alkyl an irgendeinem der obigen Substituenten optional substituiert sein kann mit $-NR_8R_9$ , worin $R_8$ und $R_9$ wie oben definiert sind,

X für C=O oder C=S steht, mit der Maßgabe, dass dann die gestrichelte Bindungslinie zwischen X und N fehlt, so dass X an das benachbarte Stickstoffatom über eine Einzelbindung gebunden ist, und mit der weiteren Maßgabe, dass dann y für 1 steht und R für Wasserstoff oder einen organischen Rest steht, der an ein Stickstoffatom gebunden sein kann, oder
X für $(CR_7)$ steht, worin $R_7$ für Wasserstoff oder einen organischen Rest steht, wie $C_1-C_7$-Niederalkyl, Amino oder Aminoalkyl, worin das Alkyl unsubstituiert oder substituiert sein kann mit Halogen, beispielsweise Methyl, Ethyl, Propyl oder Trifluormethyl, Niederalkoxy, beispielsweise Methoxy, oder Cycloalkyl, beispielsweise Cyclopropyl, mit der Maßgabe, dass dann die gestrichelte Bindungslinie zwischen X und N eine Bindung ist, so dass X an das benachbarte Stickstoffatom über eine Doppelbindung gebunden ist, und mit der Maßgabe, dass dann y für Null steht oder y für 1 steht und dann -R für $\rightarrow$ O steht,
$R_2$ für Wasserstoff steht,
$R_3$ für unsubstituiertes oder substituiertes $C_5-C_{14}$-Heterocyclyl, beispielsweise Thienyl, Benzo[1,3]dioxolo, Indolyl, Benzofuranyl oder Pyridyl, unsubstituiertes oder substituiertes $C_5-C_{14}$-Aryl, beispielsweise Phenyl, oder für Phenyl, das mit bis zu 4 Substituenten substituiert ist, vorzugsweise mit bis zu 3 Substituenten, steht, die gleich oder verschieden sind und ausgewählt sind aus Halogen, beispielsweise Cl oder F, Hydroxy, $C_1-C_4$-Niederalkoxy, beispielsweise Methoxy, Niederalkyl, beispielsweise Methyl, oder $-(C_1-C_4)_mNR_8R_9$,

worin

m für 0 oder 1 steht, und
$R_8$ und $R_9$ wie oben definiert sind, und beispielsweise Piperazinyl, Methylpiperazinyl, Morpholinyl oder Pyrrolidinyl sind,

$R_4$ für Wasserstoff oder Halogen, beispielsweise Fluor oder Chlor, steht,
$R_5$ für Wasserstoff steht, und
$R_6$ für Wasserstoff, Amino, Aminoalkyl oder Alkylamido steht, beispielsweise Methylamido-NHC(O)-$CH_3$, mit der Maßgabe, dass $R_3$ nicht unsubstituiertes Phenyl sein kann, es sei denn $R_1$ steht für Phenyl, das mit einem heterocyclischen Ring substituiert ist,

worin das Präfix Nieder für einen Rest mit einem Kohlenstoffatom und bis zu und einschließlich einem Maximum von 7 Kohlenstoffatomen steht,
oder ein pharmazeutisch akzeptables Salz hiervon.

**2.** Verbindung nach Anspruch 1,
worin

x und y jeweils unabhängig voneinander für 0 oder 1 stehen,
$R_1$ steht für substituiertes oder unsubstituiertes Phenyl, worin das Phenyl mit bis zu 4 Substituenten, vorzugsweise bis zu 3 Substituenten, substituiert ist, worin die Substituenten gleich oder verschieden sind und unabhängig ausgewählt sind aus Halogen, beispielsweise F oder Cl, $C_1$-$C_7$-Niederalkyl, das unsubstituiert oder substituiert sein kann mit Halogen, speziell Methyl, Ethyl, Propyl oder Trifluormethyl, Cyano, Cyanoniederalkyl, beispielsweise Cyanomethyl, Cyanoethyl oder Cyanopropyl, Amino, Aminoniederalkyl, worin die Aminogruppe monosubstituiert oder disubstituiert sein kann, beispielsweise mit -$(C_1$-$C_7)_m NR_8R_9$ oder -O-$(C_1$-$C_7)_m NR_8R_9$, worin

m für 0 oder 1 steht, und
$R_8$ und $R_9$ gleich oder verschieden sein können und unabhängig für H, Niederalkyl, beispielsweise Methyl, Ethyl oder Propyl oder Niedercycloalkyl, beispielsweise Cyclopropyl, stehen, oder
$R_8$ und $R_9$ zusammen mit dem Stickstoffatom einen 3- bis 8-gliedrigen heterocyclischen Ring mit 1 bis 4 Stickstoffatomen, Sauerstoffatomen oder Schwefelatomen bilden, beispielsweise Imidazolinyl, Imidazolinylethyl, Piperazinyl oder Niederalkylpiperazinyl, Aminocarbonylniederaikyl, beispielsweise $R_8R_9$-N-C (O)-$CH_2$-, worin $R_8$ und $R_9$ wie oben definiert sind, Heterocyclyl, Heterocyclylniederalkyl, Heterocyclylniederalkoxy oder Heterocyclylniederalkansulfanyl, worin das Heterocyclyl ein 3- bis 8-gliedriger heterocyclischer Ring mit 1 bis 4 Stickstoffatomen, Sauerstoffatomen oder Schwefelatomen ist, beispielsweise Imidazolinyl, Imidazolinyl, Imidazolinylethyl, Piperazinyl oder Niederalkylpiperazinyl, substituiertes oder unsubstituiertes Amid, Amidniederalkyl, beispielsweise -$CH_2$-CH($NH_2$)-C(O)-$NH_2$), worin das Alkyl linear oder cyclisch sein kann, beispielsweise Cyclopropylen, und worin das Alkyl an irgendeinem der obigen Substituenten optional substituiert sein kann mit -$NR_8R_9$, worin $R_8$ und $R_9$ wie oben definiert sind,

X für ($CR_7$) steht, worin $R_7$ für Wasserstoff, Niederalkyl, beispielsweise Methyl oder Ethyl, Amino, oder Aminoalkyl, mit der Maßgabe, dass dann die gestrichelte Bindungslinie zwischen X und N eine Bindung ist, so dass X an das benachbarte Stickstoffatom über eine Doppelbindung gebunden ist, und mit der weiteren Maßgabe, dass dann y für Null steht oder y für 1 steht und dann -R für $\rightarrow$ O steht,
$R_2$ für Wasserstoff steht,
$R_3$ für unsubstituiertes oder substituiertes $C_5$-$C_{14}$-Heterocyclyl, beispielsweise Thienyl, Benzo[1,3]dioxolo, Indolyl, Benzofuranyl oder Pyridyl, unsubstituiertes oder substituiertes $C_5$-$C_{14}$-Aryl, beispielsweise Phenyl, oder für Phenyl, das mit bis zu 4 Substituenten substituiert ist, vorzugsweise mit bis zu 3 Substituenten, steht, die gleich oder verschieden sind und ausgewählt sind aus Halogen, beispielsweise Cl oder F, Hydroxy, $C_1$-$C_4$-Niederalkoxy, beispielsweise Methoxy, Niederalkyl, beispielsweise Methyl, oder -$(C_1$-$C_4)_m NR_8R_9$, steht, worin

m für 0 oder 1 steht, und
$R_8$ und $R_9$ wie oben definiert sind und beispielsweise Piperazinyl, Methylpiperazinyl, Morpholinyl oder Pyrrolidinyl sind,

$R_4$ für Wasserstoff oder Halogen, beispielsweise Fluor oder Chlor, steht,
$R_5$ für Wasserstoff steht, und
$R_6$ für Wasserstoff steht, mit der Maßgabe, dass $R_3$ nicht unsubstituiertes Phenyl sein kann, es sei denn $R_1$ steht für Phenyl, das mit einem heterocyclischen Ring substituiert ist,

oder ein pharmazeutisch akzeptables Salz hiervon.

3. Pharmazeutische Zusammensetzung, umfassend eine Verbindung der Formel (I) nach einem der Ansprüche 1, 2 oder 7, oder ein pharmazeutisch akzeptables Salz hiervon, und einen pharmazeutisch akzeptablen Träger.

4. Verwendung einer Verbindung der Formel (I) nach einem der Ansprüche 1, 2 oder 7, oder eines pharmazeutisch akzeptablen Salzes hiervon, zur Herstellung einer pharmazeutischen Zusammensetzung für die Behandlung einer von Proteinkinase abhängigen Krankheit.

5. Verwendung nach Anspruch 4, worin die von Proteinkinase abhängige Krankheit vorzugsweise eine Krankheit ist, die abhängig ist von PKB, ALK, S6K1 oder RET und die abhängig (speziell aberrant hoch exprimiert oder aktiviert) von PKB, ALK S6K1 oder RET ist, oder eine Krankheit ist, die abhängig ist von der Aktivierung der PKB, ALK, S6K1 oder RET Wege, oder die abhängig ist von zwei oder mehr der soeben erwähnten Kinasen.

6. Verwendung nach Anspruch 4, worin die zu behandelnde Krankheit eine proliferative Krankheit ist, vorzugsweise ein benigner und speziell ein maligner Tumor, bevorzugter ein Karzinom des Hirns, der Niere, der Leber, der Adrenaldrüse, der Blase, der Brust, des Magens, gastrische Tumoren, der Ovarien, des Kolons, des Rektums, der Prostata, des Pankreas, der Lunge, der Vagina, der Schilddrüse, ein Sarkom, Glioblastom, multiples Myelom, Gastrointestinalkrebs, Kolonkarzinom, Kolorektaladenom, Tumor von Hals und Kopf, eine epidermale Hyperproliferation, Psoriasis, Prostatahyperplasie, eine Neoplasie, besonders mit Epithelcharakter, ein Mammarkarzinom, eine Leukämie, metabolische Krankheiten, Diabetes Typ 2, Obesität, Hyperlipidämie und Atherosklerose.

7. Verbindung nach Anspruch 1, die aus der Gruppe ausgewählt ist, die besteht aus
[4-(8-Benzo[1,3]dioxol-5-ylimidazo[4,5-c]chinolin-1-yl)-phenyl]-acetonitril,
[4-(8-Thiophen-2-ylimidazo[4,5-c]chinolin-1-yl)-phenyl]-acetonitril,
[4-(8-Benzofuran-2-ylimidazo[4,5-c]chinolin-1-yl)-phenyl]-acetonitril,
2-[4-(8-Benzo[1,3]dioxol-5-ylimidazo[4,5-c]chinolin-1-yl)-phenyl]-ethylamin,
2-[4-(8-Thiophen-2-ylimidazo[4,5-c]chinolin-1-yl)-phenyl]-ethylamin,
2-[4-(8-Benzofuran-2-ylimidazo[4,5-c]chinolin-1-yl)-phenyl]-ethylamin,
[3-Chlor-4-(8-thiophen-2-ylimidazo[4,5-c]chinolin-1-yl)-phenyl]-acetonitril,
(4-Amino-3-chlorphenyl)-acetonitril,
{3-Chlor-4-[8-(1H-indol-5-yl)-imidazo[4,5-c]chinolin-1-yl]-phenyl}-acetonitril,
[3-Chlor-4-(8-thiophen-3-ylimidazo[4,5-c]chinolin-1-yl)-phenyl]-acetonitril,
2-[3-Chlor-4-(8-thiophen-2-ylimidazo[4,5-c]chinolin-1-yl)-phenyl]-ethylamin,
2-{3-Chlor-4-[8-(1H-indol-5-yl)-imidazo[4,5-c]chinolin-1-yl]-phenyl}-ethylamin,
2-[3-Chlor-4-(8-thiophen-3-ylimidazo[4,5-c]chinolin-1-yl)-phenyl]-ethylamin,
[2-Fluor-4-(8-thiophen-2-ylimidazo[4,5-c]chinolin-1-yl)-phenyl]-acetonitril,
[4-(8-Benzofuran-2-ylimidazo[4,5-*c*]chinolin-1-yl)-2-fluorphenyl]-acetonitril,
{2-Fluor-4-[8-(1H-indol-5-yl)-imidazo[4,5-*c*]chinolin-1-yl]-phenyl}-acetonitril,
2-[2-Fluor-4-(8-thiophen-2-ylimidazo[4,5-*c*]chinolin-1-yl)-phenyl]-ethylamin,
2-[4-(8-Benzofuran-2-yl-imidazo[4,5-*c*]chinolin-1-yl)-2-fluorphenyl]-ethylamin,
2-{2-Fluor-4-[8-(1H-indol-5-yl)-imidazo[4,5-c]chinolin-1-yl]-phenyl}-ethylamin,
[3-Methyl-4-(8-thiophen-3-ylimidazo[4,5-*c*]chinolin-1-yl)-phenyl]-acetonitril,
{4-[8-(1H-Indol-5-yl)-imidazo[4,5-*c*]chinolin-1-yl]-3-methylphenyl}-acetonitril,
2-[3-Methyl-4-(8-thiophen-3-ylimidazo[4,5-*c*]chinolin-1-yl)-phenyl]-ethylamin,
2-{4-[8-(1H-Indol-5-yl)-imidazo[4,5-*c*]chinolin-1-yl]-3-methylphenyl}-ethylamin,
(R)-2-Amino-3-[4-(8-thiophen-2-ylimidazo[4,5-*c*]chinolin-1-yl)-phenyl]-propionamid,
(R)-2-Amino-3-[4-(8-benzo[b]thiophen-2-ylimidazo[4,5-c]chinolin-1-yl)-phenyl]-propionamid,
[3,5-Dichlor-4-(8-thiophen-2-ylimidazo[4,5-c]chinolin-1-yl)-phenyl]-acetonitril,
[3,5-Dichlor-4-(8-thiophen-3-ylimidazo[4,5-*c*]chinolin-1-yl)-phenyl]-acetonitril,
2-[3,5-Dichlor-4-(8-thiophen-2-ylimidazo[4,5-*c*]chinolin-1-yl)-phenyl]-ethylamin,
2-[3,5-Dichlor-4-(8-thiophen-3-ylimidazo[4,5-c]chinolin-1-yl)-phenyl]-ethylamin,
{4-[8-(4-Hydroxyphenyl)-imidazo[4,5-c]chinolin-1-yl]-3-trifluormethylphenyl}-acetonitril,

4-{1-[4-(2-Aminoethyl)-2-trifluormethylphenyl]-1 H-imidazo[4,5-c]chinolin-8-yl}-phenol,
3-[4-(8-Benzo[1,3]dioxol-5-ylimidazo[4,5-c]chinolin-1-yl)-phenyl]-propionitril,
3-[4-(8-Thiophen-2-ylimidazo[4,5-c]chinolin-1-yl)-phenyl]-propionitril,
3-[4-(8-Benzo[b]thiophen-2-ylimidazo[4,5-c]chinolin-1 -yl)-phenyl]-propionitril,
3-[4-(8-Thiophen-3-ylimidazo[4,5-c]chinolin-1-yl)-phenyl]-propionitril,
3-[4-(8-Benzofuran-2-ylimidazo[4,5-c]chinolin-1-yl)-phenyl]-propionitril,
3-[4-(8-Benzo[1,3]dioxol-5-ylimidazo[4,5-c]chinolin-1-yl)-phenyl]-propylamin,
3-[4-(8-Thiophen-2-ylimidazo[4,5-c]chinolin-1-yl)-phenyl]-propylamin,
3-[4-(8-Benzo[b]thiophen-2-ylimidazo[4,5-c]chinolin-1-yl)-phenyl]-propylamin,
3-[4-(8-Thiophen-3-ylimidazo[4,5-c]chinolin-1-yl)-phenyl]-propylamin,
3-[4-(8-Benzofuran-2-ylimidazo[4,5-c]chinolin-1-yl)-phenyl]-propylamin,
3-[4-(8-Benzo[1,3]dioxoi-5-ylimidazo[4,5-c]chinolin-1-yl)-3-chlorphenyl]-propionitril,
3-[3-Chlor-4-(8-thiophen-2-ylimidazo[4,5-c]chinolin-1-yl)-phenyl]-propionitril,
3-[3-Chlor-4-(8-thiophen-3-ylimidazo[4,5-c]chinolin-1-yl)-phenyl]-propionitril,
3-(3-Chlor-4-[8-(1H-indol-5-yl)-imidazo[4,5-c]chinolin-1-yl]-phenyl)-propionitril,
3-[4-(8-Benzo[1,3]dioxol-5-ylimidazo[4,5-c]chinolin-1-yl)-3-chlorphenyl]-propylamin,
3-[3-Chlor-4-(8-thiophen-2-ylimidazo[4,5-c]chinolin-1-yl)-phenyl]-propylamin,
3-[3-Chlor-4-(8-thiophen-3-ylimidazo[4,5-c]chinolin-1-yl)-phenyl]-propylamin,
3-{3-Chlor-4-[8-(1H-indol-5-yl)-imidazo[4,5-c]chinolin-1-yl]-phenyl}-propylamin,
8-Benzo[1,3]dioxol-5-yl-1-{4-[2-(4,5-dihydro-1H-imidazol-2-yl)-ethyl]-phenyl}-1H-imidazo[4,5-c]chinolin,
[3-Chlor-4-(2-methyl-8-thiophen-2-ylimidazo[4,5-c]chinolin-1-yl)-phenyl]-acetonitril,
[3-Chlor-4-(2-methyl-8-thiophen-3-ylimidazo[4,5-c]chinolin-1-yl)-phenyl]-acetonitril,
2-[3-Chlor-4-(2-methyl-8-thiophen-2-ylimidazo[4,5-c]chinolin-1-yl)-phenyl]-ethylamin,
2-[3-Chlor-4-(2-methyl-8-thiophen-3-ylimidazo[4,5-c]chinolin-1-yl)-phenyl]-ethylamin,
[4-(8-Benzo[1,3]dioxol-5-yl-7-fluorimidazo[4,5-c]chinolin-1-yl)-phenyl]-acetonitril,
[4-(7-Fluor-8-thiophen-2-ylimidazo[4,5-c]chinolin-1-yl)-phenyl]-acetonitril,
[4-(7-Fluor-8-thiophen-3-ylimidazo[4,5-c]chinolin-1-yl)-phenyl]-acetonitril,
[4-(8-Benzofuran-2-yl-7-fluorimidazo[4,5-c]chinolin-1-yl)-phenyl]-acetonitril,
{4-[7-Fluor-8-(1H-indol-5-yl)-imidazo[4,5-c]chinolin-1-yl]-phenyl}-acetonitril,
[4-(8-Benzo[b]thiophen-2-yl-7-fluorimidazo[4,5-c]chinolin-1-yl)-phenyl]-acetonitril,
2-[4-(8-Benzo[1,3]dioxol-5-yl-7-fluorimidazo[4,5-c]chinolin-1-yl)-phenyl]-ethylamin,
2-[4-(7-Fluor-8-thiophen-2-ylimidazo[4,5-c]chinolin-1-yl)-phenyl]-ethylamin,
2-[4-(7-Fluor-8-thiophen-3-ylimidazo[4,5-c]chinolin-1-yl)-phenyl]-ethylamin,
2-[4-(8-Benzofuran-2-yl-7-fluorimidazo[4,5-c]chinolin-1-yl)-phenyl]-ethylamin,
2-{4-[7-Fluor-8-(1H-indol-5-yl)-imidazo[4,5-c]chinolin-1-yl]-phenyl}-ethylamin,
2-[4-(8-Benzo[b]thiophen-2-yl-7-fluorimidazo[4,5-c]chinolin-1-yl)-phenyl]-ethylamin,
3-[4-(8-Benzo[1,3]dioxol-5-yl-7-fluorimidazo[4,5-c]chinolin-1-yl)-phenyl]-propionitril,
3-[4-(7-Fluor-8-thiophen-2-ylimidazo[4,5-c]chinolin-1-yl)-phenyl]-propionitril,
3-[4-(7-Fluor-8-thiophen-3-ylimidazo[4,5-c]chinolin-1-yl)-phenyl]-propionitril,
3-[4-(8-Benzofuran-2-yl-7-fluorimidazo[4,5-c]chinolin-1-yl)-phenyl]-propionitril,
3-[4-(8-Benzo[b]thiophen-2-yl-7-fluorimidazo[4,5-c]chinolin-1-yl)-phenyl]-propionitril,
3-{4-[7-Fluor-8-(1H-indol-5-yl)-imidazo[4,5-c]chinolin-1-yl]-phenyl}-propionitril,
3-[4-(8-Benzo[1,3]dioxol-5-yl-7-fluorimidazo[4,5-c]chinolin-1-yl)-phenyl]-propylamin,
3-[4-(7-Fluor-8-thiophen-2-ylimidazo[4,5-c]chinolin-1-yl)-phenyl]-propylamin,
3-[4-(7-Fluor-8-thiophen-3-ylimidazo[4,5-c]chinolin-1-yl)-phenyl]-propylamin,
3-[4-(8-Benzofuran-2-yl-7-fluorimidazo[4,5-c]chinolin-1-yl)-phenyl]-propylamin,
3-[4-(8-Benzo[b]thiophen-2-yl-7-fluorimidazo[4,5-c]chinolin-1-yl)-phenyl]-propylamin,
3-{4-[7-Fluor-8-(1H-indol-5-yl)-imidazo[4,5-c]chinolin-1-yl]-phenyl}-propylamin,
[3-Chlor-4-(7-fluor-8-thiophen-2-ylimidazo[4,5-c]chinolin-1-yl)-phenyl]-acetonitril,
[3-Chlor-4-(7-fluor-8-thiophen-3-ylimidazo[4,5-c]chinolin-1-yl)-phenyl]-acetonitril,
{3-Chlor-4-[7-fluor-8-(1H-indol-5-yl)-imidazo[4,5-c]chinolin-1-yl]-phenyl}-acetonitril,
2-[3-Chlor-4-(7-fluor-8-thiophen-2-ylimidazo[4,5-c]chinolin-1-yl)-phenyl]-ethylamin,
2-[3-Chlor-4-(7-fluor-8-thiophen-3-ylimidazo[4,5-c]chinolin-1-yl)-phenyl]-ethylamin,
2-{3-Chlor-4-[7-fluor-8-(1H-indol-5-yl)-imidazo[4,5-c]chinolin-1-yl]-phenyl}-ethylamin,
[4-(7-Chlor-8-thiophen-2-ylimidazo[4,5-c]chinolin-1-yl)-phenyl]-acetonitril,
[4-(7-Chlor-8-thiophen-3-ylimidazo[4,5-c]chinolin-1-yl)-phenyl]-acetonitril,
[4-(8-Benzofuran-2-yl-7-chlorimidazo[4,5-c]chinolin-1-yl)-phenyl]-acetonitril,
{4-[7-Chlor-8-(1H-indol-5-yl)-imidazo[4,5- *c*]chinolin-1-yl]-phenyl}-acetonitril,

[4-(8-Benzo[b]thiophen-2-yl-7-chlorimidazo[4,5-c]chinolin-1-yl)-phenyl]-acetonitril,
2-[4-(7-Chlor-8-thiophen-2-ylimidazo[4,5-c]chinolin-1-yl)-phenyl]-ethylamin,
2-[4-(7-Chlor-8-thiophen-3-ylimidazo[4,5-c]chinolin-1-yl)-phenyl]-ethylamin,
2-[4-(8-Benzofuran-2-yl-7-chlorimidazo[4,5-c]chinolin-1-yl)-phenyl]-ethylamin,
2-{4-[7-Chlor-8-(1H-indol-5-yl)-imidazo[4,5-c]chinolin-1-yl]-phenyl}-ethylamin,
2-[4-(8-Benzo[b]thiophen-2-yl-7-chlorimidazo[4,5-c]chinolin-1-yl)-phenyl]-ethylamin,
3-[4-(8-Benzo[1,3]dioxol-5-yl-7-chlorimidazo[4,5-c]chinolin-1-yl)-phenyt]-propionitril,
3-[4-(7-Chlor-8-thiophen-2-ylimidazo[4,5-c]chinolin-1-yl)-phenyl]-propionitril,
3-[4-(8-Benzofuran-2-yl-7-chlorimidazo[4,5-c]chinolin-1-yl)-phenyl]-propionitril,
3-{4-[7-Chlor-8-(1H-indol-5-yl)-imidazo[4,5-c]chinolin-1-yl]-phenyl}-propionitril,
3-[4-(8-Benzo[b]thiophen-2-yl-7-chlorimidazo[4,5-c]chinolin-1-yl)-phenyl]-propionitril,
3-[4-(8-Benzo[1,3]dioxol-5-yl-7-chlorimidazo[4,5-c]chinolin-1-yl)-phenyl]-propylamin,
3-[4-(7-Chlor-8-thiophen-2-ylimidazo[4,5-c]chinolin-1-yl)-phenyl]-propylamin,
3-[4-(8-Benzofuran-2-yl-7-chlorimidazo[4,5-c]chinolin-1-yl)-phenyl]-propylamin,
3-{4-[7-Chlor-8-(1H-indol-5-yl)-imidazo[4,5-c]chinolin-1-yl]-phenyl}-propylamin,
3-[4-(8-Benzo[b]thiophen-2-yl-7-chlorimidazo[4,5-c]chinolin-1-yl)-phenyl]-propylamin,
[3-Chlor-4-(7-chlor-8-thiophen-2-ylimidazo[4,5-c]chinolin-1-yl)-phenyl]-acetonitril,
[3-Chlor-4-(7-chlor-8-thiophen-3-ylimidazo[4,5-c]chinolin-1-yl)-phenyl]-acetonitril,
2-[3-Chlor-4-(7-chlor-8-thiophen-2-ylimidazo[4,5-c]chinolin-1-yl)-phenyl]-ethylamin,
2-[3-Chlor-4-(7-chlor-8-thiophen-3-ylimidazo[4,5-c]chinolin-1-yl)-phenyl]-ethylamin,
3-[3-Chlor-4-(7-chlor-8-thiophen-2-ylimidazo[4,5-c]chinolin-1-yl)-phenyl]-propionitril,
3-[3-Chlor-4-(7-chlor-8-thiophen-3-ylimidazo[4,5-c]chinolin-1-yl)-phenyl]-propionitril,
3-{3-Chlor-4-[7-chlor-8-(1H-indol-5-yl)-imidazo[4,5-c]chinolin-1-yl]-phenyl}-propionitril,
3-[3-Chlor-4-(7-chlor-8-thiophen-2-ylimidazo[4,5-c]chinolin-1-yl)-phenyl]-propylamin,
3-[3-Chlor-4-(7-chlor-8-thiophen-3-ylimidazo[4,5-c]chinolin-1-yl)-phenyl]-propylamin,
3-{3-Chlor-4-[7-chlor-8-(1H-indol-5-yl)-imidazo[4,5-c]chinolin-1-yl]-phenyl}-propylamin,
3-[4-(2-Amino-7-chlor-8-thiophen-3-ylimidazo[4,5-c]chinolin-1-yl)-phenyl]-propionitril,
3-[4-(2-Amino-8-brom-7-chlorimidazo[4,5-c]chinolin-1-yl)-phenyl]-propionitril,
3-[4-(3-Amino-6-brom-7-chlorchinolin-4-ylamino)-phenyl]-propionitril,
3-[4-(2-Amino-8-benzofuran-2-yl-7-chlorimidazo[4,5-c]chinolin-1-yl)-phenyl]-propionitril,
1-[4-(3-Aminopropyl)-phenyl]-7-chlor-8-thiophen-3-yl-1H-imidazo[4,5-c]chinolin-2-ylamin,
1-[4-(3-Aminopropyl)-phenyl]-8-benzofuran-2-yl-7-chlor-1H-imidazo[4,5-c]chinolin-2-ylamin,
8-(2,4-Dimethoxyphenyl)-1-(2-fluorphenyl)-1H-imidazo[4,5-c]chinolin,
8-(2,5-Dimethoxyphenyl)-1-(2-fluorphenyl)-1H-imidazo[4,5-c]chinolin,
8-(3,4-Dimethoxyphenyl)-1-(2-fluorphenyl)-1H-imidazo[4,5-c]chinolin,
1-(2-Fluorphenyl)-8-(3,4,5-trimethoxyphenyl)-1H-imidazo[4,5-c]chinolin,
8-(2,3-Dimethoxyphenyl)-1-(2-fluorphenyl)-1H-imidazo[4,5-c]chinolin,
1-(2-Fluorphenyl)-8-(2,3,4-trimethoxyphenyl)-1H-imidazo[4,5-c]chinolin,
1-(2-Fluorphenyl)-8-pyridin-4-yl-1H-imidazo[4,5-c]chinolin,
1-(2-Fluorphenyl)-8-pyridin-3-yl-1H-imidazo[4,5-c]chinolin,
1-(2-Fluorphenyl)-8-(3-methoxyphenyl)-1H-imidazo[4,5-c]chinolin,
{3-[1-(2-Fluorphenyl)-1H-imidazo[4,5-c]chinolin-8-yl]-benzyl}-dimethylamin,
1-(2-Fluorphenyl)-8-[3-(4-methylpiperazin-1-ylmethyl)-phenyl]-1H-imidazo[4,5-c]chinolin,
1-(2-Fluorphenyl)-8-(3-morpholin-4-ylmethylphenyl)-1H-imidazo[4,5-c]chinolin,
1-(2-Fluorphenyl)-8-(3-piperazin-1-ylmethylphenyl)-1H-imidazo[4,5-c]chinolin,
1-(2-Fluorphenyl)-8-(3-pyrrolidin-1-ylmethylphenyl)-1H-imidazo[4,5-c]chinolin,
1-Phenyl-8-(3-piperazin-1-ylphenyl)-1H-imidazo[4,5-c]chinolin,
8-(3-Fluorphenyl)-1-phenyl-1H-imidazo[4,5-c]chinolin,
8-[3-(4-Methylpiperazin-1-yl)-phenyl]-1-phenyl-1H-imidazo[4,5-c]chinolin,
4-{1-[4-(4-Methylpiperazin-1-yl)-phenyl]-1H-imidazo[4,5-c]chinolin-8-yl}-phenol,
1-[4-(4-Methylpiperazin-1-yl)-phenyl]-8-phenyl-1H-imidazo[4,5-c]chinolin, und
[4-(8-Pyridin-4-ylimidazo[4,5-c]chinolin-1-yl)-phenyl]-acetonitril.

## Revendications

**1.** Composé de formule (I)

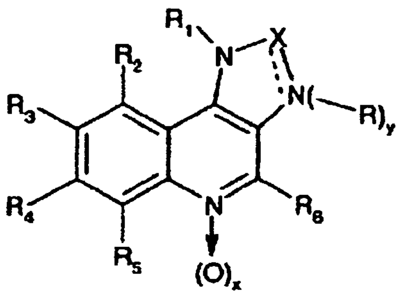

(I)

dans laquelle,
chacun des x et y est, indépendamment l'un de l'autre, 0 ou 1 ;

$R_1$ est aryle ou hétéroaryle substitué ou non substitué, tout particulièrement phényle, qui est substitué par jusqu'à 4 substituants, de préférence par jusqu'à 3 substituants, **caractérisé en ce que** les substituants sont identiques ou différents et sont indépendamment sélectionnés à partir d'halo, p.ex., F ou Cl ; alkyle inférieur qui peut être non substitué ou substitué par halo, tout particulièrement méthyle, éthyle, propyle ou trifluorométhyle ; cyano ; cyano-alkyle inférieur, p.ex.. cyanométhyle, cyanoéthyle ou cyanopropyle ; alcoxy inférieur ; amino ; amino-alkyle inférieur ; amino-alcoxy inférieur ; amino-alkylsulfanyle inférieur ou thiol-alkyle inférieur, **caractérisé en ce que** le groupe amino peut être mono- ou di-substitué, p.ex., $-(C_1-C_7)_m NR_8R_9$ ou $-O-(C_1-C_7)_mNR_8R_9$,
dans laquelle,
m est 0 ou 1 ; et
$R_8$ et $R_9$ peuvent être identiques ou différents et sont indépendamment H; alkyle inférieur, p.ex., méthyle, éthyle ou propyle; cycloalkyle inférieur, p.ex., cyclopropyle, ou
$R_8$ et $R_9$, de pair avec l'atome N, forment un cycle hétérocyclique possédant de 3 à 8 ramifications et contenant de 1 à 4 atomes d'azote, oxygène ou soufre, p.ex., azétidinyle, pyrrolidinyle, pipéridino, morpholinyle, imidazolinyle, imidazolinyl-éthyle, pipérazinyle ou alkyle inférieur-pipérazinyle ; amino-carbonyl-alkyle inférieur, p.ex., $R_8R_9$-N-C(O)-$CH_2$-, dans laquelle $R_8$ et $R_9$ sont tels que définis ci-dessus ; hétérocyclyle ; hétérocyclyl-alkyle inférieur ; hétérocyclyl-alcoxy inférieur ou hétérocyclyl-alkanesulfanyle inférieur, **caractérisé en ce que** le cycle hétérocyclyle possédant de 3 à 6 ramifications et contenant de 1 à 4 atomes d'azote, oxygène ou soufre, p.ex., imidazolyle, imidazolinyle, pyrrolidinyle, morpholinyle, azétidinyle, pyridyle, pipéridino, pipéridyle, pipérazinyle ou alkyle inférieur-pipérazinyle ; amide substitué ou non substitué ; amide-alkyle inférieur, p.ex.. $-CH_2-CH(NH_2)-C(O)-NH_2)$, **caractérisé en ce que** alkyle peut être linéaire ou cyclique, p.ex.. cyclopropylène ; et l'alkyle présent dans n'importe lequel des substituants ci-dessus peut éventuellement être substitué par $-NR_8R_9$, dans lequel $R_8$ et $R_9$ sont tels que définis ci-dessus;
X est C=O ou C=S, à condition que lorsque la liaison en pointillés entre X et N est absente, alors X est lié à l'atome N adjacent par l'intermédiaire d'une liaison unique et à condition que lorsque y est 1 et que R est hydrogène ou un groupement organique, ils puissent être liés à un azote ; ou
X est $(CR_7)$, **caractérisé en ce que** $R_7$ est hydrogène ou un groupement organique, tel que $C_1$-$C_7$alkyle inférieur ; amino ou amino alkyle, **caractérisé en ce que** alkyle peut être non substitué ou substitué par halo, p.ex.. méthyle, éthyle, propyle ou trifluorométhyle ; alcoxy inférieur, p.ex., méthoxy ; ou cycloalkyle, p.ex., cyclopropyle ; à condition que lorsque la ligne en pointillés entre X et N est une liaison, alors X est lié à l'atome N adjacent par l'intermédiaire d'une liaison double ; et à condition que lorsque y est zéro, ou que y est 1, -R soit $\rightarrow$ O ;
$R_2$ est hydrogène ;
$R_3$ est $C_5$-$C_{14}$hétérocyclyle non substitué ou substitué, p.ex. thiényle, benzo[1,3]dioxolo, indolyle, benzofuranyle ou pyridiyle ; $C_5$-$C_{14}$aryle non substitué ou substitué, p.ex., phényle ou phényle substitué par jusqu'à 4 substituants, de préférence par jusqu'à 3 substituants, qui sont identiques ou différents et qui sont sélectionnés à partir d'halo, p.ex.. Cl ou F ; hydroxy ;
$C_1$-$C_4$alcoxy inférieur, p.ex., méthoxy ; alkyle inférieur, p.ex., méthyle ;
ou $-(C_1-C_4)_mN_8R_9$,
dans laquelle,
m est 0 ou 1 ; et
$R_8$ et $R_9$, sont tels que définis ci-dessus, p.ex., pipérazinyle, méthylpipérazinyle, morpholinyle ou pyrrolidinyle ;
$R_4$ est hydrogène ou halo, p.ex.. fluoro ou chloro ;

$R_5$ est hydrogène ; et
$R_6$ est hydrogène, amino, amino alkyle ou alkylamido, p.ex., méthylamido -NHC(O)-$CH_3$), à condition que $R_3$ ne puisse pas être phényle non substitué

sauf si $R_1$ est phényle substitué par un cycle hétérocyclique ;
**caractérisé en ce que** le suffixe « inférieur » désigne un radical possédant de 1 atome de carbone jusqu'à et y compris un maximum de 7 atomes de carbone,
ou un de ses sels pharmaceutiquement acceptables.

**2.** Composé selon la revendication 1,
dans laquelle,
chacun des x et y est, indépendamment l'un de l'autre, 0 ou 1 ;

$R_1$ est phényle substitué ou non substitué **caractérisé en ce que** le phényle est substitué par jusqu'à 4 substituants, de préférence par jusqu'à 3 substituants, **caractérisé en ce que** substituants sont identiques ou différents et sont indépendamment sélectionnés à partir d'halo, p.ex., F ou Cl ; $C_1$-$C_7$alkyle inférieur, qui peut être non substitué ou substitué par halo, tout particulièrement méthyle, éthyle, propyle ou trifluorométhyle ; cyano ; cyano-alkyle inférieur, p.ex., cyanométhyle, cyanoéthyle ou cyanopropyle ; amino ; amino-alkyle inférieur, **caractérisé en ce que** le groupe amino peut être mono- ou di-substitué, p.ex., $-(C_1-C_7)_mNR_8R_9$ or $-O-(C_1-C_7)_mNR_8R_9$,
dans laquelle,
m est 0 ou 1 ; et
$R_8$ et $R_9$ peuvent être identiques ou différents et sont indépendamment H ; alkyle inférieur, p.ex., méthyle, éthyle or propyle ; cycloalkyle inférieur, p.ex., cyclopropyle, ou
$R_8$ et $R_9$, de pair avec l'atome N, forment un cycle hétérocyclique possédant de 3 à 8 ramifications et contenant de 1 à 4 atomes d'azote, d'oxygène ou de soufre ; p.ex. imidazolinyle, imidazolinyl-éthyle, pipérazinyle ou alkyle inférieur-pipérazinyle, amino-carbonyl-alkyle inférieur, p.ex. $R_8R_9$-N-C(O)-$CH_2$-, dans laquelle $R_8$ et $R_9$ sont tels que définis ci-dessus, hétérocyclyle; hétérocyclyl-alkyle inférieur ; hétérocyclyl-alcoxy inférieur ou hétérocyclylalkanesulfanyle inférieur, **caractérisé en ce que** l'hétérocyclyle est un cycle hétérocyclique possédant de 3 à 8 ramifications et contenant de 1 à 4 atomes d'azote, d'oxygène ou de soufre, p.ex., imidazolinyle, imidazolinyle, imidazolinyl-éthyle, pipérazinyle ou alkyle inférieur-pipérazinyle ; amide substitué ou non substitué ; amide-alkyle inférieur, p.ex., -$CH_2$-CH($NH_2$)-C(O)-$NH_2$, **caractérisé en ce que** alkyle peut être linéaire ou cyclique, p.ex., cyclopropylène ; et l'alkyle présent dans n'importe lequel des substituants ci-dessus peut éventuellement être substitué par -$NR_8R_9$, dans lequel $R_8$ et $R_9$ sont tels que définis ci-dessus;
X est ($CR_7$), **caractérisé en ce que** $R_7$ est hydrogène ; alkyle inférieur, p.ex., méthyle ou éthyle ; amino ou amino alkyle, à condition que lorsque la ligne en pointillés entre X et N est une liaison, alors X soit lié à l'atome N adjacent par l'intermédiaire d'une liaison double ; et à condition que lorsque y est zéro, ou que y est 1, -R soit $\rightarrow$ O ;
$R_2$ est hydrogène ;
$R_3$ est $C_5$-$C_{14}$hétérocyclyle non substitué ou substitué, p.ex. thiényle, benzo[1,3]dioxolo, indolyle, benzofuranyle ou pyridiyle ; $C_5$-$C_{14}$aryle non substitué ou substitué, p.ex., phényle ou phényle substitué par jusqu'à 4 substituants, de préférence par jusqu'à 3 substituants, qui sont identiques ou différents et qui sont sélectionnés à partir d'halo, p.ex.. Cl ou F ; hydroxy ; $C_1$-$C_4$alcoxy inférieur, p.ex., méthoxy ; alkyle inférieur, p.ex., méthyle ; ou $-(C_1-C_4)_mNR_8R_9$,
dans laquelle,
m est 0 ou 1 ; et
$R_8$ et $R_9$, sont tels que définis ci-dessus, p.ex., pipérazinyle, méthylpipérazinyle, morpholinyle ou pyrrolidinyle ;
$R_4$ est hydrogène ou halo (p.ex. fluoro ou chloro) ;
$R_5$ est hydrogène ; et
$R_6$ est hydrogène, à condition que $R_3$ ne puisse pas être phényle non substitué sauf si $R_1$ est phényle substitué par un cycle hétérocyclique ;

ou un de ses sels pharmaceutiquement acceptables.

**3.** Composition pharmaceutique comprenant un composé de formule (I), selon l'une quelconque des revendications 1, 2 ou 7, ou un de ses sels pharmaceutiquement acceptables et un véhicule pharmaceutiquement acceptable.

**4.** Utilisation d'un composé de formule (I), selon l'une quelconque des revendications 1, 2 ou 7, ou un de ses sels

pharmaceutiquement acceptables, dans la préparation d'une composition pharmaceutique utilisable pour le traitement d'une maladie dépendant d'une protéine kinase.

5. Utilisation selon la revendication 4, **caractérisée en ce que** la maladie dépendant d'une protéine kinase est de préférence une maladie dépendant de PKB, ALK, S6K1 ou RET et une maladie dépendant de PKB, ALK, S6K1 ou RET (tout particulièrement exprimée ou activée de façon aberrante) ou une maladie dépendant de l'activation des voies PKB, ALK, S6K1 ou RET, ou une maladie dépendant de deux ou plusieurs des kinases mentionnées.

6. Utilisation selon la revendication 4, **caractérisée en ce que** la maladie à traiter est une maladie proliférative, de préférence une tumeur bénigne ou, tout particulièrement maligne, de préférence un carcinome du cerveau, du rein, du foie, des glandes surrénales, de la vessie, du sein, de l'estomac, gastro-intestinal, de l'ovaire, du côlon, du rectum, de la prostate, du pancréas, du poumon, du vagin, de la thyroïde, un sarcome, un glioblastome, un myélome multiple, un cancer gastro-intestinal, un carcinome du côlon, un adénome colorectal, une tumeur de la tête et du cou, une hyperprolifération de l'épiderme, le psoriasis, l'hyperplasie de la prostate, une néoplasie, tout particulièrement à caractère épithélial, un carcinome mammaire, une leucémie, des maladies métaboliques, le diabète de type II, l'obésité, l'hyperlipidémie et l'athérosclérose.

7. Composé selon la revendication 1, sélectionné à partir du groupe composé de :

[4-(8-Benzo[1,3]dioxol-5-yl-imidazo[4,5-*c*]quinolin-1-yl)-phényl]-acétonitrile ;
[4-(8-Thiophen-2-yl-imidazo[4,5-*c*]quinolin-1-yl)-phényl]-acétonitrile ;
[4-(8-Benzofuran-2-yl-imidazo[4,5-*c*]quinolin-1-yl)-phényl)-acétonitrile ;
2-[4-(8-Benzo[1,3]dioxol-5-yl-imidazo[4,5-*c*]quinolin-1-yl)-phényl]-éthylamine ;
2-[4-(8-Thiophen-2-yl-imidazo[4,5-*c*]quinolin-1-yl)-phényl]-éthylamine ;
2-[4-(8-Benzofuran-2-yl-imidazo[4,5-*c*]quinolin-1-yl),-phényl]-éthylamine ;
[3-Chloro-4-(8-thiophen-2-yl-imidazo[4,5-*c*]quinolin-1-yl)-phényl]-acétonitrile ;
(4-Amino-3-chloro-phényl)-acétonitrile ;
{3-Chloro-4-(8-(1*H*-indol-5-yl)-imidazo[4,5-*c*]quinolin-1-yl]-phényl)-acétonitrile ;
[3-Chloro-4-(8-thiophen-3-yl-imidazo[4,5-*c*]quinolin-1-yl)-phényl]-acétonitrile;
2-[3-Chloro-4-(8-thiophen-2-yl-imidazo[4,5-*c*]quinolin-1-yl)-phényl]-éthylamine;
2-{3-Chloro-4-[8-(1H-indol-5-yl)-imidazo[4,5-*c*]quinolin-1-yl]-phényl}éthylamine;
2-[3-Chloro-4-(8-thiophen-3-yl-imidazo[4,5-*c*]quinolin-1-yl)-phényl]-éthylamine;
[2-Fluoro-4-(8-thiophen-2-yl-imidazo[4,5-*c*]quinolin-1-yl)-phényl]-acétonitrile;
[4-(8-Benzofuran-2-yl-imidazo[4,5-*c*]quinolin-1-yl)-2-fluoro-phényl]-acétonitrile;
{2-Fluoro-4-[8-(1 H-indol-5-yl)-imidazo[4,5-*c*]quinolin-1-yl)-phényl}-acétonitrile;
2-[2-Fluoro-4-(8-thiophen-2-yl-imidazo[4,5-*c*]quinolin-1-yl)-phényl]-éthylamine;
2-[4-(8-Benzofuran-2-yl-imidazo[4,5-*c*]quinolin-1-yl)-2-fluoro-phényl]-éthylamine ;
2- {2-Fluoro-4-[8-(1H-indol-5-yl)-imidazo[4,5-*c*]quinolin-1-yl]-phényl]-éthylamine ;
[3-Méthyl-4-(8-thiophen-3-yl-imidazo[4,5-*c*]quinolin-1-yl)-phényl]-acétonitrile ;
(4-[8-(1H-Indol-5-yl)-imidazo[4,5-*c*]quinolin-1-yl]-3-méthyl-phényl)-acétonitrile;
2-[3-Méthyl-4-(8-thiophen-3-yl-imidazo[4,5-*c*]quinolin-1-yl)-phényl]-éthylamine;
2- {4-[8-(1H-Indol-5-yl)-imidazo[4,5-*c*]quinolin-1-yl)-phényl]-3-méthyl-phényl}-éthylamine;
(R)-2-Amino-3-[4-(8-thiophen-2-yl-imidazo[4,5-c]quinolin-1-yl)-phényl]-propionamide ;
(R)-2-Amino-3-[4-(8-benzo[b]thiophen-2-yl-imidazo[4,5-*c*]quinolin-1-yl)-phényl]-propionamide;
[3,5-Dichloro-4-(8-thiophen-2-yl-imidazo[4,5-*c*]quinolin-1-yl)-phényl]-acétonitrile;
[3,5-Dichloro-4-(8-thiophen-3-yl-imidazo[4,5-*c*]quinolin-1-yl)-phényl]-acétonitrile;
2-[3,5-Dichloro-4-(8-thiophen-2-yl-imidazo[4,5-*c*]quinolin-1-yl)-phényl]-éthylamine ;
2-[3,5-Dichloro-4-(8-thiophen-3-yl-imidazo[4,5-*c*]quinolin-1-yl)-phényl]-éthylamine ;
{4-[8-(4-Hydroxy-phényl)-imidazo[4,5-*c*]quinolin-1-yl]-3-trifluorométhyl-phényl}-acétonitrile ;
4- {1-(4-(2-Amino-éthyl)-2-trifluorométhyl-phényl]-1H-imidazo[4,5-*c*]quinolin-8-yl}-phénol ;
3-[4-(8-Benzo[1,3]dioxol-5-yl-imidazo[4,5-*c*]quinolin-1-yl)-phényl]-propionitrile
3-[4-(8-Thiophen-2-yl-imidazo[4,5-*c*]quinolin-1-yl)-phényl)-propionitrile;
3-[4-(8-Benzo[*b*]thiophen-2-yl-imidazo[4,5-*c*]quinolin-1-yl)-phényl]-propionitrile;
3-[4-(8-Thiophen-3-yl-imidazo[4,5-*c*]quinolin-1-yl)-phényl]-propionitrile ;
3-[4-(8-Benzofuran-2-yl-imidazo[4,5-*c*]quinolin-1-yl)-phényl]-propionitrile ;
3-[4-(8-Benzo[1,3]dioxol-5-yl-imidazo[4,5-*c*]quinolin-1-yl)-phényl)-propylamine ;
3-[4-(8-Thiophen-2-yl-imidazo[4,5-*c*]quinolin-1-yl)-phényl)-propylamine ;
3-[4-(8-Benzo[b]thiophen-2-yl-imidazo[4,5-*c*]quinolin-1-yl)-phényl]-propylamine ;

3-[4-(8-Thiophen-3-yl-imidazo[4,5-*c*]quinolin-1-yl)-phényl]-propylamine ;
3-[4-(8-Benzofuran-2-yl-imidazo[4,5-*c*]quinolin-1-yl)-phényl]-propylamine ;
3-[4-(8-Benzo[1,3]dioxol-5-yl-imidazo[4,5-*c*]quinolin-1-yl)-3-chloro-phényl]-propionitrile ;
3-[3-Chloro-4-(8-thiophen-2-yl-imidazo[4,5-*c*]quinolin-1-yl)-phényl]-propionitrile ;
3-[3-Chloro-4-(8-thiophen-3-yl-imidazo[4,5-*c*]quinolin-1-yl)-phényl]-propionitrile ;
3- {3Chtoro-4-[8-(1H-indol-5-yl)-imidazo[4,5-*c*]quinotin-1-yl]-phényl}-propionitrile ;
3-[4-(8-Benzo[1,3]dioxol-5-yl-imidazo[4,5-*c*]quinolin-1-yl)-3-chloro-phényl]-propylamine ;
3-[3-Chloro-4-(8-thiophen-2-yl-imidazo[4,5-*c*]quinolin-1-yl)-phényl]-propylamine ;
3-[3-Chloro-4-(8-thiophen-3-yl-imidazo[4,5-*c*]quinolin-1-yl)-phényl]-propylamine ;
3- {3-Chloro-4-[8-(1H-indol-5-yl)-imidazo[4,5-*c*]quinolin-1-yl]-phényl}-propylamine ;
8-Benzo[1,3]dioxol-5-yl-1-{4-[2-(4,5-dihydro-1H-imidazol-2-yl)-éthyl]-phényl}-1H-imidazo[4,5-*c*]quinoline ;
[3-Chloro-4-(2-méthyl-8-thiophen-2-yl-imidazo[4,5-*c*]quinolin-1-yl)-phényl]-acétonitrile ;
[3-Chloro-4-(2-méthyl-8-thiophen-3-yl-imidazo[4,5-*c*]quinolin-1-yl)-phényl]-acétonitrile ;
2-[3-Chloro-4-(2-méthyl-8-thiophen-2-yl-imidazo[4,5-*c*]quinolin-1-yl)-phényl]-éthylamine ;
2-[3-Chloro-4-(2-méthyl-8-thiophen-3-yl-imidazo[4,5-*c*]quinolin-1-yl)-phényl]-éthylamine ;
[4-(8-Benzo[1,3]dioxol-5-yl-7-fluoro-imidazo[4,5-*c*]quinolin-1-yl)-phényl)-acétonitrile ;
[4-(7-Fluoro-8-thiophen-2-yl-imidazo[4,5-*c*]quinolin-1-yl)-phényl]-acétonitrile;
[4-(7-Fluoro-8-thiophen-3-yl-imidazo[4,5-*c*]quinolin-1-yl)-phényl]-acétonitrile;
[4-(8-Benzofuran-2-yl-7-fluoro-imidazo[4,5-*c*]quinolin-1-yl)-phényl]-acétonitrile;
(4-[7-Fluoro-8-(1H-indol-5-yl)-imidazo[4,5-*c*]quinolin-1-yl]-phényl)-acétonitrile;
[4-(8-Benzo[b]thiophen-2-yl-7-fluoro-imidazo[4,5-*c*]quinolin-1-yl)-phényl]-acétonitrile ;
2-[4-(8-Benzo[1,3]dioxol-5-yl-7-fluoro-imidazo[4,5-*c*]quinolin-1-yl)-phényl]-éthylamine ;
2-[4-(7-Fluoro-8-thiophen-2-yl-imidazo[4,5-*c*]quinolin-1-yl)-phényl]-éthylamine ;
2-[4-(7-Fluoro-8-thiophen-3-yl-imidazo[4,5-*c*]quinolin-1-yl)-phényl)-éthylamine ;
2-[4-(8-Benzokran-2-yl-7-fluoro-imidazo[4,5-*c*]quinolin-1-yl)-phényl]-éthylamine ;
2- {4-(7-Fluoro-8-(1H-indol-5-yl)-imidazo[4,5-*c*]quinolin-1-yl]-phényl}-éthylamine ;
2-[4-(8-Benzo[b]thiophen-2-yl-7-fluoro-imidazo[4,5-*c*]quinolin-1-yl)-phényl]-éthylamine ;
3-[4-(8-Benzo[1,3]dioxol-5-yl-7-fluoro-imidazo[4,5-*c*]quinolin-1-yl)-phényl]-propionitrile ;
3-[4-(7-Fluoro-8-thiophen-2-yl-imidazo[4,5-*c*]quinolin-1-yl)-phényl]-propionitrile;
3-[4-(7-Fluoro-8-thiophen-3-yl-imidazo[4,5-c]quinolin-1-yl)-phényl]-propionitrile ;
3-[4-(8-Benzofuran-2-yl-7-fluoro-imidazo[4,5-*c*]quinolin-1-yl)-phényl]-propionitrile ;
3-[4-(8-Benzo[b]thiophen-2-yl-7-fluoro-imidazo[4,5-*c*]quinolin-1-yl)-phényl]-propionitrile ;
3-{4-(7-Fluoro-8-(1H-indol-5-yl)-imidazo[4,5-*c*]quinolin-1-yl]-phényl}-propionitrile ;
3-[4-(8-Benzo[1,3]dioxol-5-yl-7-fluoro-imidazo[4,5-*c*]quinolin-1-yl)-phényl]-propylamine ;
3-[4-(7-Fluoro-8-thiophen-2-yl-imidazo[4,5-*c*]quinolin-1-yl)-phényl]-propylamine;
3-[4-(7-Fluoro-8-thiophen-3-yl-imidazo[4,5-*c*]quinolin-1-yl)-phényl]-propylamine;
3-[4-(8-Benzofuran-2-yl-7-fluoro-imidazo[4,5-*c*]quinolin-1-yl)-phényl]-propylamine ;
3-[4-(8-Benzo[b]thiophen-2-yl-7-fluoro-imidazo[4,5-*c*]quinolin-1-yl)-phényl]-propylamine ;
3-{4-[7-Fluoro-8-(1H-indol-5-yl)-imidazo[4,5-*c*]quinolin-1-yl]-phényl}-propylamine;
[3-Chloro-4-(7-fluoro-8-thiophen-2-yl-imidazo[4,5-*c*]quinolin-1-yl)-phényl]-acétonitrile ;
[3-Chloro-4-(7-fluoro-8-thiophen-3-yl-imidazo[4,5-*c*]quinolin-1-yl)-phényl]-acétonitrile;
{3-Chloro-4-[7-fluoro-8-(1H-indol-5-yl)-imidazo[4,5-*c*]quinolin-1-yl]-phényl}-acétonitrile ;
2-[3-Chloro-4-(7-fluoro-8-thiophen-2-yl-imidazo[4,5-*c*]quinolin-1-yl)-phényl]-éthylamine ;
2-[3-Chloro-4-(7-fluoro-8-thiophen-3-yl-imidazo[4,5-*c*]quinolin-1-yl)-phényl]-éthylamine ;
2- {3-Chloro-4-[7-fluoro-8-(1H-indol-5-yl)-imidazo[4,5-*c*]quinolin-1-yl]-phényl)-éthylamine ;
[4-(7-Chloro-8-thiophen-2-yl-imidazo[4,5-*c*]quinolin-1-yl)-phényl]-acétonitrile ;
[4-(7-Chloro-8-thiophen-3-yl-imidazo[4,5-*c*]quinolin-1-yl)-phényl]-acétonitrile;
[4-(8-Benzofuran-2-yl-7-chloro-imidazo[4,5-*c*]quinolin-1-yl)-phényl]-acétonitrile;
{4-[7-Chloro-8-(1H-indol-5-yl)-imidazo[4,5-*c*]quinolin-1-yl]-phényl}-acétonitrile ;
[4-(8-Benzo[b]thiophen-2-yl-7-chloro-imidazo[4,5-*c*]quinolin-1-yl)-phényl]-acétonitrile ;
2-[4-(7-Chtoro-8-thiophen-2-yl-imidazo[4,5-*c*]quinolin-1-yl)-phényl]-éthylamine ;
2-[4-(7-Chloro-8-thiophen-3-yl-imidazo[4,5-*c*]quinolin-1-yl)-phényl]-éthylamine ;
2-[4-(8-Benzofuran-2-yl-7-chloro-imidazo[4,5-*c*]quinolin-1-yl)-phényl]-éthylamine ;
2- {4-[7-Chloro-8-(1H-indol-5-yl)-imidazo[4,5-c]quinolin-1-yl]-phényl}-éthylamine ;
2-[4-(8-Benzo[b]thiophen-2-yl-7-chloro-imidazo[4,5-*c*]quinolin-1-yl)-phényl)-éthylamine ;
3-[4-(8-Benzo[1,3]dioxol-5-yl-7-chloro-imidazo[4,5-*c*]quinolin-1-yl)-phényl]-propionitrile ;
3-[4-(7-Chloro-8-thiophen-2-yl-imidazo[4,5-*c*]quinolin-1-yl)-phényl]-propionitrile;
3-[4-(8-Benzofuran-2-yl-7-chloro-imidazo[4,5-*c*]quinolin-1-yl)-phényl]-propionitrile ;

3- {4-(7-Chloro-8-(1H-indol-5-yl)-imidazo[4,5-*c*]quinolin-1-yl]-phényl}-propionitrile;
3-[4-(8-Benzo[b]thiophen-2-yl-7-chloro-imidazo[4,5-*c*]quinolin-1-yl)-phényl]-propionitrile ;
3-[4-(8-Benzo[1,3]dioxol-5-yl-7-chloro-imidazo[4,5-c]quinolin-1-yl)-phényl]-propylamine ;
3-[4-(7-Chloro-8-thiophen-2-yl-imidazo[4,5-*c*]quinolin-1-yl)-phényl]-propylamine;
3-[4-(8-Benzofuran-2-yl-7-chloro-imidazo[4,5-*c*]quinolin-1-yl)-phényl]-propylamine ;
3- {4-[7-Chloro-8-(1H-indol-5-yl)-imidazo[4,5-*c*]quinolin-1-yl)phényl}-propylamine ;
3-[4-(8-Benzo[b]thiophen-2-yl-7-chloro-imidazo[4,5-*c*]quinolin-1-yl)-phényl]-propylamine;
[3-Chloro-4-(7-chloro-8-thiophen-2-yl-imidazo[4,5-c]quinolin-1-yl)-phényl]-acétonitrile;
[3-Chloro-4-(7-chloro-8-thiophen-3-yl-imidazo[4,5-c]quinolin-1-yl)-phényl]-acétonitrile ;
2-[3-Chloro-4-(7-chloro-8-thiophen-2-yl-imidazo[4,5-*c*]quinolin-1-yl)-phényl]-éthylamine ;
2-[3-Chloro-4-(7-chloro-8-thiophen-3-yl-imidazo[4,5-*c*]quinolin-1-yl)-phényl]-éthylamine ;
3-[3-Chloro-4-(7-chloro-8-thiophen-2-yl-imidazo[4,5-*c*]quinolin-1-yl)-phényl]-propionitrile ;
3-[3-Chloro-4-(7-chloro-8-thiophen-3-yl-imidazo[4,5-*c*]quinolin-1-yl)-phényl]-propionitrile ;
3- {3-Chloro-4-[7-chloro-8-(1 H-indol-5-yl)-imidazo[4,5-*c*]quinolin-1-yl]-phényl}-propionitrile ;
3-[3-Chloro-4-(7-chloro-8-thiophen-2-yl-imidazo[4,5-*c*]quinolin-1-yl)-phényl]-propylamine ;
3-[3-Chloro-4-(7-chloro-8-thiophen-3-yl-imidazo[4,5-*c*]quinolin-1-yl)-phényl]-propylamine ;
3- {3-Chloro-4-[7-chloro-8-(1H-indol-5-yl)-imidazo[4,5-*c*]quinolin-1-yl]-phényl}-propylamine ;
3-[4-(2-Amino-7-chloro-8-thiophen-3-yl-imidazo[4,5-*c*]quinolin-1-yl)-phényl]-propionitrile ;
3-[4-(2-Amino-8-bromo-7-chloro-imidazo[4,5-*c*]quinolin-1-yl)-phényl]-propionitrile ;
3-[4-(3-Amino-6-bromo-7-chloro-quinolin-4-ylamino)-phényl]-propionitrile;
3-[4-(2-Amino-8-benzofuran-2-yl-7-chloro-imidazo[4,5-*c*]quinolin-1-yl)-phényl]-propionitrile ;
1-[4-(3-Amino-propyl)-phényl]-7-chloro-8-thiophen-3-yl-1H-imidazo[4,5-c]quinolin-2-ylamine ;
1-[4-(3-Amino-propyl)-phényl]-8-benzofuran-2-yl-7-chloro- 1H-imidazo[4,5-*c*]quinolin-2-ylamine ;
8-(2,4-Diméthoxy-phényl)-1-(2-fluoro-phényl)-1H-imidazo[4,5-*c*]quinoline ;
8-(2,5-Diméthoxy-phényl)-1-(2-fluoro-phényl)-1H-imidazo[4,5-*c*]quinoline ;
8-(3,4-Diméthoxy-phényl)-1-(2-fluoro-phényl)-1H-imidazo[4,5-*c*]quinoline;
1-(2-Fluoro-phényl)-8-(3,4,5-triméthoxy-phényl)-1H-imidazo[4,5-*c*]quinoline ;
8-(2,3-Diméthoxy-phényl)-1-(2-fluoro-phényl)-1H-imidazo[4,5-*c*]quinoline;
1-(2-Fluoro-phényl)-8-(2,3,4-triméthoxy-phényl)-1H-imidazo[4,5-*c*]quinoline ;
1-(2-Fluoro-phényl)-8-pyridin-4-yl-1H-imidazo[4,5-*c*]quinoline ;
1-(2-Fluoro-phényl)-8-pyridin-3-yl-1H-imidazo[4,5-*c*]quinoline ;
1-(2-Fluoro-phényl)-8-(3-méthoxy-phényl)-1H-imidazo[4,5-*c*]quinoline ;
{3-[1-(2-Fluoro-phényl)-1H-imidazo[4,5-*c*]quinoline-8-yl]-benzyl}-diméthylamine ;
1-(2-Fluoro-phényl)-8-[3-(4-méthyl-pipérazin-1-ylméthyl)-phényl]-1H-imidazo [4,5-c] quinoline ;
1-(2-Fluoro-phényl)-8-(3-morpholin-4-ylméthyl-phényl)-1H-imidazo[4,5-*c*] quinoline ;
1-(2-Fluoro-phényl)-8-(3-pipérazin-1-ylméthyl-phényl)-1H-imidazo[4,5-*c*] quinoline ;
1-(2-Fluoro-phényl)-8-(3-pyrrolidin-1-ylméthyl-phényl)-1H-imidazo[4,5-*c*] quinoline ;
1-Phényl-8-(3-pipérazin-1-yl-phényl)-1H-imidazo[4,5-*c*]quinoline;
8-(3-Fluoro-phényl)-1-phényl-1H-imidazo[4,5-c]quinoline ;
8-[3-(4-Méthyl-pipérazin-1-yl)-phényl)-1-phényl-1H-imidazo[4,5-*c*]quinoline;
4- {1-[4-(4-Méthyl-pipérazin-1-yl)-phényl]-1H-imidazo[4,5-*c*]quinoline-8-yl}-phénol ;
1-[4-(4-Méthyl-pipérazin-1-yl)-phényl]-8-phényl-1H-imidazo[4,5-c]quinoline; et
[4-(8-Pyridin-4-yl-imidazo[4,5-c]quinolin-1-yl)-phényl]-acétonitrile.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0246188 A2
- US 5605899 E
- US 6331539 B
- US 4659516 A
- US 4636505 A
- US 4100274 A
- US 5843901 A
- WO 9917804 A
- US 5010099 A
- WO 9810121 A
- US 6194181 B
- WO 9825929 A
- WO 9808849 A
- WO 9943653 A
- WO 9822461 A
- WO 0031247 A
- WO 0222577 A
- WO 02092599 A
- US 5093330 A
- RO 318220
- RO 320432
- WO 0009495 A
- WO 9702266 A
- EP 0564409 A
- WO 9903854 A
- EP 0520722 A
- EP 0566226 A
- EP 0787722 A
- EP 0837063 A
- US 5747498 A
- WO 9810767 A
- WO 9730034 A
- WO 9749688 A
- WO 9738983 A
- WO 9630347 A
- WO 9633980 A
- WO 9503283 A
- WO 03013541 A

### Non-patent literature cited in the description

- **CANTLEY et al.** *Proc Nat. Acad Sci USA,* 1999, vol. 96, 4240-4245
- **VAZQUEZ et al.** *Biochimica et Biophysica Acta,* 2000, vol. 1470, M21-M35
- **SIMPSON et al.** *Exper Cell Res,* 2001, vol. 264, 29-41
- **LI et al.** *Science,* 1997, vol. 275, 1943-1947
- **STECK et al.** *Nat Genetics,* 1997, vol. 15, 356-362
- **NAGASE et al.** *Br J Cancer,* 1996, vol. 74, 1979-1983
- **PEIFFER et al.** *Cancer Res,* 1995, vol. 55, 1922-1926
- **GRAY et al.** *Cancer Res,* 1995, vol. 55 (21), 4800-4803
- **ITTMANN.** *Cancer Res,* 1996, vol. 56, 2143-2147
- **PERREN et al.** *Am J Pathol,* 1999, vol. 155 (4), 1253-1260
- **ROBERTSON et al.** *Cancer Res,* 1999, vol. 59 (15), 3596-3601
- **CAIRNS et al.** *Oncogene,* 1998, vol. 16, 3215-3218
- **GRONBAEK et al.** *Blood,* 1998, vol. 91, 4388-4390
- **KIM et al.** *Oncogene,* 1998, vol. 16, 89-93
- **HAAS-KOGAN et al.** *Curr Biol,* 1998, vol. 8, 1195-1198
- **WHANG et al.** *Proc Natl Acad Sci USA,* 1998, vol. 95, 5246-5250
- **WU et al.** *Proc Nail Acad Sci USA,* 1998, vol. 95, 15587-15591
- **MAEHAMA ; DIXON.** *Trends Cell Biol,* 1990, vol. 9, 125-128
- **DUYSTER et al.** *Oncogene,* 2001, vol. 20, 5623-5637
- **FERRARI ; THOMAS.** *Methods Enzymol,* 1991, vol. 200, 159-169
- **WOOD et al.** *Cancer Res,* 2000, vol. 60 (8), 2178-2189
- *In Vitro,* 1978, vol. 14, 911-915
- *In Vitro,* 1976, vol. 12, 331
- *Cancer Res,* 1978, vol. 38, 1345-1355
- *Cancer Res,* 1978, vol. 37, 4049-4058
- *Cancer Res,* 1980, vol. 40, 524-534
- *Int J Cancer,* 1976, vol. 17, 62-70
- *Science,* 1989, vol. 246, 491-494
- **TOMIOKA et al.** *Cancer Res,* 2001, vol. 61, 7518-7524
- **SUZUKI.** *Tetrahedron Lett,* 1979, vol. 20, 3437
- *J Am Chem Soc,* 1985, vol. 107, 972

- Protective Groups in Organic Chemistry. Plenum Press, 1973
- **HOUBEN-WEYL.** Methoden derorganischen Chemie. Georg-Thieme-Verlag, 1974, vol. 15/1
- **THEODORA W. GREENE.** Protective Groups in Organic Synthesis. John Wiley & Sons, 1981
- *Eur J Med Chem,* 1996, vol. 31, 133